# EUROPEAN PATENT APPLICATION

(11) **EP 1 842 909 A1**
(43) Date of publication of application: **10.10.2007**
(21) Application number: 06702679.9
(22) Date of filing: 12.01.2006
(51) Int. Cl.: C12N 15/00, A61K 35/48, A61L 27/00, C12N 5/10, C12N 15/09

(54) **METHOD FOR REMOVING DESIRED CHROMOSOME AND TAILOR-MADE MEDICAL TREATMENT UTILIZING THE SAME**

(30) Priority: 13.01.2005 JP 2005006859
(71) Applicant: President, Kyoto University, Kyoto 606-8501 (JP); ReproCELL Inc., Tokyo 100-0011 (JP)
(72) Inventor: TADA, Takashi c/o Institute for Frontier Medical, Sakyo-ku,Kyoto, 6068507 (JP); NAKATSUJI, Norio c/o Institute for Frontier Medica, Shogo-in, Sakyo-ku, Kyoto, 6068 (JP); MATSUMURA, Hiroyuki c/o Institute for Frontier Med, Shogo-in, Sakyo-ku, Kyoto, 6 (JP); TADA, Masako, 1-1-1, Uchisaiwaicho, Chiyoda-ku, Tokyo, (JP)
(74) Representative: Irvine, Jonquil Claire
(86) International application number: PCT/JP2006/300311
(87) International publication number: WO 2006/075671

(57) **Abstract**

It is intended to provide a regenerable cell in which a desired chromosome has been deleted, a method for producing the cell, and a gene cassette and a kit to be used for the method. More particularly, it is intended to obtain an individual corresponding pluripotent stem cell easily and simply. More specifically, it is intended to efficiently establish a cell, tissue or organ that can be a donor for treating a disease without causing immune rejection response, without newly obtaining and establishing a stem cell such as ES cell from the individual, and without using an ovum as a material. It was achieved by a gene cassette in which not two recombinase recognition sites in a cis orientation are inserted, but one recombinase recognition site or two recombinase recognition sites in an opposite orientation is/are inserted and a marker gene is connected, and by application of the gene cassette to a cell fusion technique.

## Description

### BACKGROUND OF THE INVENTION

### Field of the Invention:

The present invention relates to the field of regenerative medicine generally. The present invention provides a regenerable cell in which a desired chromosome is deleted, the production method thereof, and the gene cassette and kit for using thereof.

### Description of the Related Art:

The disease medical treatment by regenerative medicine (regeneration medicine) is attracted attention recently. However, by the time it is not routinely applied to many patients who show symptoms of organ or tissue dysfunction. Until now, organ transplantation as well as use of the supplementary system in medical equipment is applied for only a few limited patients as the medical treatment of such a patient. However, the treatment method has problems such as shortage of donor, rejection, infection, and durable period. Especially shortage of donor is a serious problem, and although marrow or cord blood bank is enhanced gradually both Japan and abroad in the case of a bone marrow transplantation, it is difficult to provide many patients with the limited sample. Therefore, in order to overcome these problems, the expectation for the regenerative medicine consisting mainly of the stem cell medical treatment by using embryonic stem cells (ES cells) or the like and application thereof is increasing.

ES cell is an undifferentiated totipotency cell which is induced from an early embryo and is increased rapidly, and shows the similar property of an embryonic carcinoma cell. ES cell was established by culturing the inner cell mass (Inner cell mass; ICM) in mouse blastocyst on the feeder cell layer of mouse fibroblast cell at first. ES cell has an infinite lifetime under conditions which maintain the undifferentiated state under the existence of the feeder cell layer and / or a leukemia inhibitory factor (LIF) [nonpatent literature 1: R. Williams et al., Nature 336: 684-687 (1988)]. It is known that ES cell has high in vitro differentiation capacity and can be made to differentiate a large variety of cells by only culturing as an assembly mass. ES cell is a cell which has the pluripotency, which is established from the preimplantation stage embryo and differentiates to the various cellular types derived from 3 germ layer of ectoderm, mesoderm, and entoderm. [nonpatent literature 2: M. J. Evansand M. H. Kaufman. Nature 2292 :154-156 (1981); nonpatent literature 3: G. R. Martin and Proc. Natal. Acad. Sci. USA. 78: 7634-7638 (1981)]. That is, ES cell has the capability to differentiate to all the mature cells of an adult, for example, by inducing in a normal early embryo and by forming a chimera embryo, it can be made to differentiate to both somatic cell of a chimera animal and germ cell. [nonpatent literature 4: R. L. Brinster, J. Exp. Med. 140: 1949-1956 (1974); nonpatent literature 5: A. Bradley et al., Nature 309: 255-256 (1984)]. By crossing as parents the chimera animal which the cells derived from ES cells were induced into germ cells such as testis, ovary and the like, the progeny who comprises only the cells derived from ES cells can be obtained. That is, the animal which has the artificial character to regulate genetically sufficiently can be obtained. By such an animal, the research on the mechanism of embryogenesis and differentiation become possible only in in vitro but in an individual level. ES cell is a normal cell which has the normal diploid karyotype unlike the embryonal carcinoma cell, which is the rate of chimera formation is high and the probability of differentiating to the germline cell is also high [nonpatent literature 5], and the application area of ES cell is also expanding except the embryology field.

ES cell also has important roles for cell research and genetic research to determine the cell differentiation. For example, for function analysis of a gene, which the sequence was known, mouse ES cells induced genetic modification and has been used for production of the mouse strain which the gene was destroyed. Use of an undifferentiated ES cells is useful with extreme efficiency in function analysis operation after decoding human genome. In in vitro, since ES cells can be made to differentiate to the wide variety of various cellular types, it has been used in order to study the cell differentiation mechanism in embryogenesis. By adding a growth factor or by forming embryoid, ES cells have become possible to urge to differentiate to a useful cell clinically, such as hematopoietic cell, cardiac muscle, nerve cell, and the like. [nonpatent literature 6: M. Wiles et al., Development 111: 259-267 (1991); nonpatent literature 7: W Miller-Hance et al. and J. Biol. Chem. 268: 25244-25252; nonpatent literature 8: V. A. Maltsev et al. and Mech. Dev. 44: 41-50 (1993); nonpatent literature 9: G. Bain et al. and Dev. Biol. 168: 342-357 (1995)]. The attempt of induction on mouse ES cells to differentiate to the useful cell was successful in hematopoietic cell, cardiac muscle, specific neuron, and manufacture of the blood vessel. [nonpatent literature 10: T. Nakanoeta1., Science 265: 1098-1101 (1994); nonpatent literature 11: R. Pacacios et al., Proc. Natal. Acad. Sci. USA 92: 7530-7534 (1995); nonpatent literature 8: V. A. Maltsev et al., Mech. Dev. 44: 41-50 (1993); nonpatent literature 12: S. H. Lee et al., Nat. Biotechnol. 18: 675-679 (1999); nonpatent literature 13: H. Kawasaki et al., Neuron 28: 31-40 (2000); nonpatent literature 14: S.-1. Nishikawa, Development 125: 1747-1757 (1998); nonpatent literature 15: M. Hirashima et al., Blood 93: 1253-1263 (1999)].

Presently, ES cells were established in various animals such as hamster, mouse, and the like, and human ES cells were also established and showed the similar differentiation capability like mouse ES cells. [nonpatent literature 16: J. A. Thomson et al., Science 282: 1145-1147 (1998); nonpatent literature 17: J. A. Thomson et al. and Dev. Biol. 38: 133-165 (1998); nonpatent literature 18: B. E. Reubinoff et al., Nat. Biotechnol.18:399-404(2000)]. By applying the accumulated enormous information about the differentiation inducing regulation, which is obtained mainly in mouse ES cells, human ES cells are provided as an infinite material of various cells and tissues for transplantation medical treatment in a large number of disease including myocardial infarction, Parkinson's disease, diabetes, and leukemia, and the problem for shortage of donor in transplantation medical treatment is expected to be solved. Three research teams by Australia, the U.S.A., and Germany reported having succeeded in making nerve cell, muscle cell, and the like from human ES cells for the first time in the International Society for Stem Cell Research symposium on June 23, 2000. The method to be made to differentiate to the hematopoietic cell from human ES cells is also reported recently. However, also when using ES cells in transplantation medical treatment, as well as the recent organ transplantation, the problem to cause the immune rejection response is remaining.

The transplantation of the living tissue is performed for various reasons. By complementing a function, which is deficient by the organ transplantation, for example, the fatal disease in important organs such as the kidney can be also saved. When transplantation to other parts in the same individual is called autogenous transplantation, the piece of autogenous transplantation is not rejected. The transplantation between identical twins and between inbred line animals is called syngeneic transplantation, and the piece of transplantation also survives permanently in the host in the case. The transplantation between the same species is called allogeneic (outcross) transplantation, and when not performing the special treatment to avoid rejection, the piece of transplantation is rejected. The transplantation between different species is called xenotransplantation, and the piece of transplantation is immediately destroyed by the host.

The factor which causes the rejection of the transplantation piece is called transplantation antigen or histocompatibility antigen. All the somatic cells except red corpuscles have transplantation antigen. Red corpuscles have an original blood type (ABO) antigen. The main human transplantation antigens are called the major histocompatibility antigen or HLA (human white corpuscle group A), and are related to the gene in the 6th chromosome. A HLA antigen is classified into two groups of the class I antigen which is the target of rejection response and the class II antigen which has the role to start of rejection. Although class I antigen is observed in all the tissues, class II antigens is not the same manner and expressed in dendritic cell which is a cell like macrophage with the fingerlike projection. Although there is an example of experimental success about the attempt to remove such a cell from organ transplantation tissue so that rejection can not begin, it is not suitable for practical use and not clinically applied at present.

The rejection appeared after transplantation can be classified into hyperacute rejection response, promoted type acute rejection response, acute rejection response, and chronic rejection response. Hyperacute rejection response occurs when the existing antibody, which is reacted to a donor's HLA antigen, exists in a recipient's serum. After completing blood vessel combination and resuming the blood flow to organs, transplantation organs are immediately abolished by the intense rejection occurring within several hours. Presently, there is no treatment method and when a lymphocyte cross match test is performed before transplantation and an antibody which reacted to donor lymphocyte in recipient serum is recognized, giving up the transplantation is only prevention to avoid the rejection. Promoted type acute rejection response is expressed when reactant T lymphocyte to donor's HLA antigen exists in recipient's body before transplantation. The symptom develops within seven days after transplantation in many cases and shows an intense rejection as well as hyperacute rejection response, but it also become possible to cure by the progress of curative medicine in recent years. Acute rejection response mainly occurs as a result of induction of cell-mediated immune response by T lymphocyte, which is induced by the donor HLA antigen of the transplanted organs. The rejection response most often appears after transplantation, it usually recognizes by about 2 weeks to one month after transplantation. Chronic rejection response is clinically characterized by the decline of the organ function which resists medical treatment and progresses gradually, and it occurs by six months to one year after transplantation in many cases. Basically, it is considered that tissue denaturation progressing over the long period by the tissue disorder of the transplantation organ, which is caused by the immune response of the recipient activated by invasion of the donor HLA antigen and the response of the corresponding organ tissue. Unless the organ having the same MHC molecular structure of a recipient is transplanted, the rejection response is certainly occurred. Presently, how to control the rejection response is a major problem.

As the immune suppression method for not starting the above rejection responses, it roughly divides that use of immunosuppressant, surgical operation, radiation irradiation, or the like is mentioned. First, a main immunosuppressant is adrenal-cortex steroid medicine, cyclosporin, FK506, or the like. Adrenal cortex steroid medicine decreases the number of cyclicity T cells, inhibits the nucleic acid metabolism of lymphocyte and cytokine production to suppress the function, and suppress the immune response by inhibition of migration and metabolism of a macrophage. On the other hand, cyclosporin and FK506 have the similar effect, combines with the receptor on the surface of a helper T cell, migrates into a cell, and acts directly to DNA for inhibiting the production of interleukin-2. Finally, a killer T cell cannot operate and an immune suppression effect occurs. In use of these immunosuppressants, side effects become a problem. Especially steroid has many side effects and cyclosporin has the toxicity to liver and kidney. FK506 has the toxicity to kidney. Next, for example, although there are lymph node isolation, spleen isolation, and thymus excision as a surgical operation, the effect is not fully demonstrated. Although thoracic duct funnel in surgical operation leads the circulating lymphocyte to the outside of the body and confirms the effect, the efflux of a lot of serum protein and fat is caused and there is a fault that nutritional disorder occurs easily. Although radiation irradiation includes the total body irradiation and the piece transplantation irradiation, since there is an aspect that the effect is uncertain and the burden to a recipient is large, it is used by the combined use with the above-mentioned immunosuppressant. It is clear that neither of the above-mentioned methods is perfect for prevention of the rejection response.

Presently, it is shown in the mammal that a somatic cell nucleus is reprogrammed to totipotency by introduction of somatic cell nucleus to egg cell after nuclear expulsion, and cloned sheep, cloned cow, cloned mouse, cloned pig, or the like is created. [nonpatent literature 19: Wilmut I.et al. and Nature 385: 810-813 (1997); nonpatent literature 20: Kato Y. et al. Science 282:2095-2098 (1998); nonpatent literature 21: Wakayama T. et al., Nature 394: 369-374 (1998); nonpatent literature 22: Onishi A. et al., Science 289 1188-1190 (2000); nonpatent literature 23: Polejaeva I. A. et al., Nature 407: 86-90 (2000)]. Utilizing the technique, by being reprogrammed of somatic cell nucleus derived from the host receiving transplantation with an egg cell and by producing the totipotency cell, it is considered that the transplantation piece, which does not cause the immune rejection response, can be produced. According to the method of such a cell culture, shortage of donor can also seem to be solved.

However, cloning for medical treatment in human has encountered the social problem of biomedical ethics problem. [nonpatent literature 24: Weissman, I. L., N Engl J Med 346, 1576-1579. (2002)]. In the above mentioned method, the necessity to use an egg cell become a problem in the ethical viewpoint. In human, taking into consideration that an ES cell is derived from the undifferentiated cell of an early embryo and the fact that the early embryo of an adult does not exist, there is a fundamental problem that ES cells cannot be established from the host after an early embryo stage, especially an adult. Therefore, obtaining pluripotent stem cells like ES cells corresponding to an individual is not realized by now, and the subject matter is desired to solve in the field.

Under such circumstances, the present inventors developed the production method of pluripotent stem cells which are deleted a part or all of the transplantation antigen derived from embryonic stem cells (also called ES cell in the present specification) by fusion and the production method of a cell, a tissue, or an organ including differentiation of the cell, the tissue, or the organ, which expresses only the major histocompatibility antigen derived from somatic cell from the fused cell preliminarily. (patent documents 1: international publication no. WO03/027278). However, the use of conventional method was only way to remove of a desired gene or a chromosome (especially 6th chromosome containing HLA gene), and the removal efficiency was not so superior. Therefore, also in preparation of such pluripotent stem cells, the development of gene removal method, which is usable, efficient, and maintained the regeneration capacity and pluripotency, is desired. Patent document 1 discloses the method of being a cell into an undifferentiated state by using cell fusion. Although the removal of specific gene is described in this literature, the technique related to produce a fused cell which is removed the chromosome and therefore is removed desired chromosome, and a regenerable cell which become an undifferentiated state by the fused cell, are not described.

Nonpatent literature 25 (Mark Lewandosk & G. R. Martin, Nature Genetics Vol. 17, 1997 223-25) discloses the method of removing Y chromosome out ofXY chromosome. The technique related to produce a fused cell which is removed the chromosome and therefore is removed the desired chromosome, and a regenerable cell which become an undifferentiated state by the fused cell is not described. In the technique of this literature, since the produced cell can only generate abnormal, the cell, which is usable in the regenerative medical treatment, cannot be provided.

In nonpatent literature 26 and patent document 2, the chromosome of embryonic stem cells was attempt to delete by using the construct which is inserted the loxP sequence in the same direction. However, this method has any change to the conventional method, therefore, the deletion efficiency is bad. Therefore, in the technique of this literature, the cell which is usable in the regenerative medical treatment cannot be provided efficiently,
Patent document 1: International publication no. WO03/027278.
Patent document 2: Japanese Patent 2003-512053.
Nonpatent literature 1: R.Williams et al., Nature 336: 684-687 (1988).
Nonpatent literature 2: M. J. Evansand M. H. Kaufman. Nature 292: 154-156 (1981)
Nonpatent literature 3: G. R. Martin, Proc. Natal. Acad. Sci. USA. 78: 7634-7638 (1981).
Nonpatent-literature 4:R. L. Brinster, J. Exp. Med. 140: 1949-1956 (1974).
Nonpatent literature 5: A. Bradley et al., Nature 309: 255-256 (1984).
Nonpatent literature 6: M. Wiles et al., Development 111: 259-267 (1991).
Nonpatent literature 7: W Miller-Hance et al., and J. Biol. Chem. 268: 25244-25252 (1993).
Nonpatent literature 8: V. A. Maltsevetal. Mech. Dev. 44: 41-50 (1993).
Nonpatent literature 9: G. Bain et al. Dev. Biol. 168: 342-357 (1995)
Nonpatent literacture 10: T. Nakano et al., Science 265: 1098-1101 (1994).
Nonpatent literature 11: R. Pacacios et al., Proc. Natal. Acad. Sci. USA 92: 7530-7534 (1995).
Nonpatent literature 12: S. H. Lee et al. Nat. Biotechnol. 18: 675-679 (1999).
Nonpatent literature 13: H. Kawasaki et al., Neuron 28: 31-40 (2000).
Nonpatent literature 14: S. -I. Nishikawa Development 125: 1747-1757 (1998).
Nonpatent literature 15: M. Hirashima et al., Blood 93: 1253-1263 (1999).
Nonpatent literature 16: J. A. Thomson et al. Science 282:1145-1147 (1998).
Nonpatent literature 17: J. A. Thomson et al., Dev.Biol. 38: 133-165 (1998).
Nonpatent literature 18: B. E. Reubinoff et al. Nat.Biotechnol. 18: 399-404 (2000).
Nonpatent literature 19: Wilmut I.et al., Nature 385: 810-813 (1997).
Nonpatent literature 20: Kato Y. et al., Science 282: 2095-2098 (1998).
Nonpatent literature 21: Wakayama T. et al., Nature 394: 369-374 (1998).
Nonpatent literature 22: Onishi A. et al., Science 289: 1188-1190 (2000).
Nonpatent literature 23: Polejaeva I. A. et al., Nature 407: 86-90 (2000).
Nonpatent literature 24: Weissman, I. L., N Engl J Med 346, 1576-1579 (2002).
Nonpatent literature 25: Mark Lewandosk & G. R. Martin and Nature Genetics Vol. 17, 1997 223-25.
Nonpatent literature 26: Koike, H., Horie, K., Fukuyama, H., Kondoh, G., and Nagata, S. and Takeda, J. (2002) Efficient biallelic mutagenesis with Cre/loxP-mediated inter-chromosomal recombination. EMBO Rep 3, 433-7.

### DESCLOSURE OF THE INVENTION

### Problems to be solved by the invention

An object of the present invention is to provide a regenerable cell in which a desired chromosome has been deleted, a method for producing the cell, and a gene cassette and a kit to be used for the method.

More particularly, an object of the present invention is to obtain an individual corresponding pluripotent stem cells easily and simply. More specifically, an object of the present invention is to efficiently establish a cell, a tissue or an organ that can be a donor for treating diseases without causing immune rejection response, without newly obtaining and establishing a stem cell such as an ES cell from the individual, and without further using an ovum as a material.

### Means of solving the problems

The invention solved that the recombinase recognition site is inserted for one or two opposite direction but is inserted in the two cis-directions, and that the gene cassette is connected the marker gene and the cassette is applied to cytogamy technique.

As the result, a desired chromosome is removed and the cell, which is regenerable and preferably an undifferentiated state, is provided. By applying the technique, a tailor made stem cell (for example, ES cell) is provided.

In addition the present invention was succeeded in producing a tailor made pluripotent stem cell by providing the technique of deleting the unnecessary chromosome efficiently without losing pluripotency from the cell having a desired genome and being provided pluripotency. Preferably, in the present invention, the pluripotent stem cell corresponding to "perfect" individual without the rejection response and not having any genes other than the desired genome can be obtained.

In the present invention, when a cell, a tissue, and an organ which are differentiated freely from the pluripotent stem cell having the desired genome such as the fused cell, the reprogrammed somatic cell, or the like and being eliminated efficiently the unnecessary chromosome are introduced into a recipient, the desired tailor made medicine becomes possible, for example, the medical treatment including that the rejection response is decreased, not detected completely, or the like in the recipient is efficiently realized comparing to the stem cell origin which has all of the transplantation antigen derived from the stem cell (e.g. ES cell). That is, the pluripotent stem cell in the present invention can be a perfect material for establishing the cell, the tissue, and the organ being a donor for the treatment of the disease. These cells, tissues, and organs have many applications in tailor made type medical treatment, and the industrial usefulness is high.

Thus, the embryonic stem (ES) cell in human and mouse can be provided the pluripotency to the somatic nucleus of an adult in the ES hybrid cell (Tada, M., Takahama, Y., Abe, K. Nakatsuji, N. Tada, T. Nuclear reprogramming of somatic cells by in vitro hybridization with ES cells.Curr Biol 11, 1553-8, (2001); Cowan, C. A., Atienza, J., Melton, D. A. Eggan, K. Nuclear reprogramming of somatic cells after fusion with human embryonic stem cells. Science 309, 1369-73 (2005)). It is the powerful approach for producing the pluripotent stem cell reprogrammed without using an embryonic substance from a somatic cell, and when the target chromosome can be eliminated from a hybrid cell, it has an extremely great meaning to the progress in the human medical treatment. The present inventors developed the universal chromosome elimination cassette (CEC) for the realization of the purpose. In the present specification, the present inventors show the use of CEC for eliminating both copies of the 6th chromosome derived from ES cell as a target from the hybrid cell having some important pluripotency related genes (e.g. Nanog). Subsequently the continuation of the pluripotency of the hybrid cell is depending on the expression of Nanog in the reprogrammed somatic cell nucleus. The invention of the present inventors demonstrates that the production of the personalized pluripotency stem cell for the use for the stem cell treatment is substantially feasible by the elimination of the ES origin MHC chromosome from the hybrid cell or replacement the MHC chromosome derived from the ES cell to the MHC chromosome derived from the somatic cell. Finally, all the chromosomes derived from ES cell can be eliminated from the hybrid cell. The view to the production of the pluripotent stem cell with various mutations is important similarly by eliminating the normal ES cell chromosome derived from the patient as a target for investigating the cause and medical treatment of the human disease.

### SUMMARY OF THE INVENTION

The present invention specifically provides the following.
(1) A gene cassette including a recombinase recognition sequence for one, or two in the opposite direction, wherein a sequence coding a marker gene is included therein.
(2) The gene cassette according to claim 1, wherein said marker gene is a homologous recombination sequence including the sequence selected from the group consisting of the loxP sequence, the FRT site, the attB sequence, the attP sequence and the res site sequence.
(3) The gene cassette according to claim 1, wherein said marker gene includes in the gene selected from the group consisting of the green fluorescence protein (GFP) gene, the yellow fluorescence protein (YFP) gene, the cyanogen fluorescence protein (CFP) gene, and the red fluorescence protein (dsRED) gene.
(4) The gene cassette according to claim 1, wherein the gene cassette further includes a selection sequence.
(5) The gene cassette according to claim 4, wherein said selection sequence includes in the sequence coded the selected gene from the group consisting of the thymidine kinase (TK) gene, the puromycin resistance gene, the neomycin resistance gene, the hygromycin resistance gene, the blasticidin resistance gene, the zeosin resistance gene, the HAT resistance gene, the diphtheria toxin resistance gene, and the fluorouracil resistance gene.
(6) The gene cassette according to claim 1, wherein said recombinase recognition sequence includes the sequence of ATAACTTCGTATAATGTATGCTATACGAAGTTAT, which is described in sequence number 1.
(7) The gene cassette according to claim 1, wherein the gene cassette further includes the IRES sequence, which is described in sequence number 2.
(8) The gene cassette according to claim 1, wherein the gene cassette further includes the CAG sequence, which is described in sequence number 3.
(9) The gene cassette according to claim 1, wherein said recombinase recognition sequence includes two in the opposite direction.
(10) The gene cassette according to claim 1, wherein said recombinase recognition sequence is included two in the opposite direction and includes in the CAG sequence, the marker sequence, the IRES sequence, and the selection sequence in between the recombinase recognition sequences.
(11) The gene cassette including a recombinase recognition sequence for one, or two in the opposite direction, wherein a composition for the elimination of the desired chromosome includes the sequence coded a marker gene and the gene cassette.
(12) The composition according to claim 11, wherein said chromosome includes a pluripotency related gene.
(13) The composition according to claim 11, wherein said elimination includes the removal of both copies in said chromosomes.
(14) A kit for the elimination of the desired chromosome, wherein comprises
   A) a gene cassette including the recombinase recognition sequence for one, or two in the opposite direction, wherein the gene cassette includes the sequence coded the marker gene; and
   B) a nucleic acid molecule which is recombinase corresponding to the recombinase recognition sequence or the nucleic acid molecules including the nucleic acid sequences coded the recombinase recognition sequence.
(15) A kit for the elimination of the desired chromosome from the stem cell, wherein comprises
   A) a gene cassette including the recombinase recognition sequence for one, or two in the opposite direction, wherein the gene cassette includes the sequence coded a marker gene;
   B) a nucleic acid molecule which is recombinase corresponding to the recombinase recognition sequence or a nucleic acid molecules including the nucleic acid sequences coded the recombinase recognition sequence; and
   C) a stem cell.
(16) A kit for providing the cell with the pluripotency by the elimination of the desired chromosome from the cell having the desired genome, the chromosome, or the group of chromosomes, wherein comprises
   A) a gene cassette including the recombinase recognition sequence for one, or two in the opposite direction, wherein the gene cassette includes the sequence coded a marker gene;
   B) a nucleic acid molecule which is recombinase corresponding to the recombinase recognition sequence or a nucleic acid molecules including the nucleic acid sequences coded the recombinase recognition sequence; and
   C) a stem cell; and
   D) a cell having the desired genome, the chromosome, or the group of chromosomes.
(17) A method for providing the cell with the pluripotency by the elimination of a desired chromosome or a group of chromosomes from a cell having the desired genome, wherein comprises
   A) a step providing a gene cassette including the recombinase recognition sequence for one, or two in the opposite direction including the sequence coded a marker gene;
   B) a step of introducing the gene cassette to a stem cell;
   C) a step of fusing between the stem cell and the desired genome, the chromosome, or the group of chromosomes;
   D) a step of providing a recombinase recognizing the recombinase recognition sequence or a nucleic acid molecules including a nucleic acid sequences coded the recombinase recognition sequence;
   E) a step of exposing the fused cell in the condition that occurs recombination; and
   F) a step of observing a signal derived from the marker gene in the fused cell and selecting the cell eliminated the desired chromosome.
(18) The cell made by the method according to claim 17.
(19) A cell in which is eliminated the desired chromosome.
(20) A stem cell in which is eliminated the desired chromosome.
(21) A stem cell according to claim 20, wherein said stem cell is the ES cell.
(22) The cell according to claim 19, wherein said chromosome includes the sequence coding the MHC or the corresponding gene.
(23) A chromosome recombinant cell, wherein the desired chromosome or the group of chromosomes is modified.
(24) A chromosome recombinant stem cell, wherein the desired chromosome or the group of chromosomes is modified.
(25) The stem cell according to claim 24, wherein said stem cell is the ES cell.
(26) A choromosome recombinant cell of a fused cell between the ES cell and the micronucleus having a desired genome, a chromosome or a group of chromosomes, having a pluripotency and eliminating of the desired chromosome derived from the ES cell from the fused cell.
(27) A fused cell between an ES cell and a cell having a desired genome, a chromosome or a group of chromosomes, wherein the fused cell provides a pluripotency and is eliminated of the desired chromosome from the fused cell.
(28) A fused cell between an ES cell and a cell having a desired genome, a chromosome or a group of chromosomes, wherein the fused cell provides a pluripotency and is eliminated the desired chromosome of the ES cell from the fused cell.
(29) A fused cell, wherein said desired chromosome is a chromosome including a sequence coded a MHC of an ES cell, the rejection response related gene, or the corresponding gene.
(30) A fused cell, wherein said desired chromosome is the chromosome being selected from a group of the 6^{th} chromosome, the 11^{th} chromosome, the 12^{th} chromosome, and the 17^{th} chromosome of mouse and the 6^{th} chromosome of human or the chromosome including one copy or both copies of the chromosome(s) corresponding to the chromosome(s).
(31) A fused cell, wherein said desired chromosome is the chromosome including one or more than two chromosome(s) of an ES cell.
(32) A fused cell, wherein said desired chromosomes are all the chromosomes of the ES cell.
(33) A fused cell, wherein a cell having said desired genome, a chromosome, or a group of chromosome is the cell having the genome, chromosome, or a group of chromosome derived from diseased individual.
(34) A fused cell, wherein a cell having said desired genome, a chromosome, or a group of chromosome is the cell having the genome, a chromosome, or a group of chromosome derived from diseased individual and said desired chromosomes are all the chromosomes in the ES cell.
(35) A cell library, wherein the cell library includes a plurality kind of cells being eliminated a desired chromosome.
(36) A use of gene cassette eliminating of a desired chromosome including the recombinase recognition sequence for one, or two in the opposite direction, wherein the gene cassette includes the sequence coded the marker gene.
(37) A use of gene cassette producing of eliminating of a desired chromosome including recombinase recognition sequence for one, or in the two opposite direction, wherein the gene cassette includes the sequence coded the marker gene.
(38) A method for the elimination of a desired chromosome from the cell including a desired genome, a chromosome, or a group of chromosomes, wherein comprises
   A) a step of providing a gene cassette including the recombinase recognition sequence for one, or two in the opposite direction, wherein the gene cassette includes the sequence coded a marker gene;
   B) a step of introducing the gene cassette to the stem cell;
   C) a step of fusing between the cell and the cell having the desired genome, the chromosome, or the group of chromosomes;
   D) a step of providing the fused cell with the recombinase recognizing the recombinase recognition sequence or a nucleic acid molecules including the nucleic acid sequences coded the recombinase recognition sequence;
   E) a step of exposing the fused cell in the condition that occurs the reconstitution; and
   F) a step of observing the signal derived from the marker gene of the fused cell and selecting the cell being eliminated the desired chromosome.
(39) A method for production a chromosome recombinant cell, comprising:
   A) a step of providing a gene cassette including the recombinase recognition sequence for one, or two in the opposite direction, wherein the gene cassette includes the sequence coded a marker gene;
   B) a step of introducing the gene cassette to the stem cell;
   C) a step of fusing between the cytoplasm of the second cell having the desired chromosome and the first cell;
   D) a step of providing a recombinase recognizing the recombinase recognition sequence or the nucleic acid molecules including the nucleic acid sequences coded the recombinase recognition sequence so that the first cell can be eliminated the chromosome in the chromosome of first cell corresponding to the desired chromosome of second cell;
   E) a step of exposing the fused cell in the condition that occurs the reconstitution; and
   F) a step of observing the signal derived from the marker gene of the fused cell and selecting the cell being eliminated the desired chromosome.
(40) The cell in which is produced by the method according to claim 38 or 39.

Therefore, these and the other advantages of the present invention become clear by referring the attached figures, reading the following detailed explanation, and understanding them for the person skilled in the art.

### Effect of the invention

The present invention provides the art in which the target chromosome can be eliminated efficiently and freely. Therefore, by eliminating a chromosome freely, the regeneration medicine and the tailor made medicine, which the immunological rejection is reduced or inhibited, became easy, for example.

In a preferred embodiment, the transcription product derived from the specific chromosome is only the somatic cell origin by elimination of the chromosome derived from the ES cell in the present invention. By applying this, the application to the innovative drug development field can be considered by the calibration of drug effect or the like corresponding to an individual.

### BRIEF EXPLANATION OF THE DRAWINGS

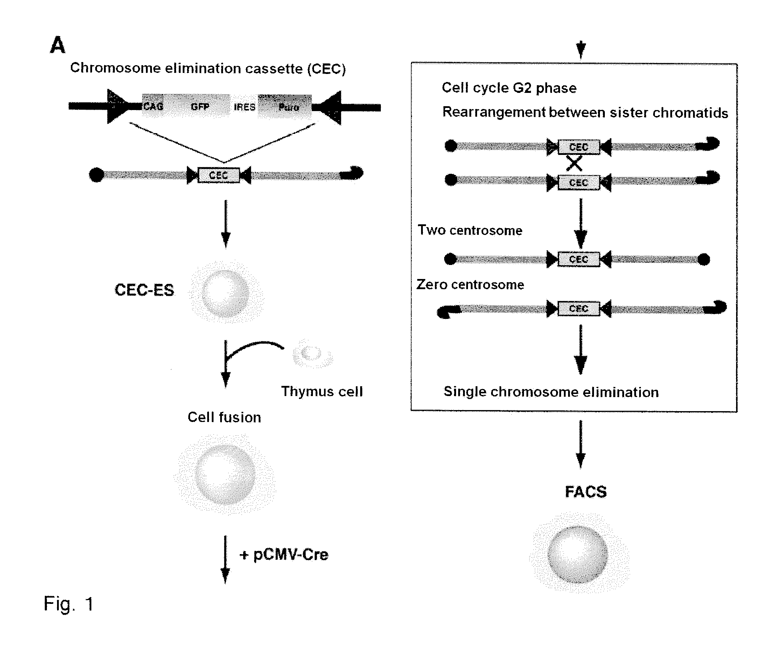

[Fig. 1A] Fig. 1A shows the implementation scheme of an embodiment in the present invention.

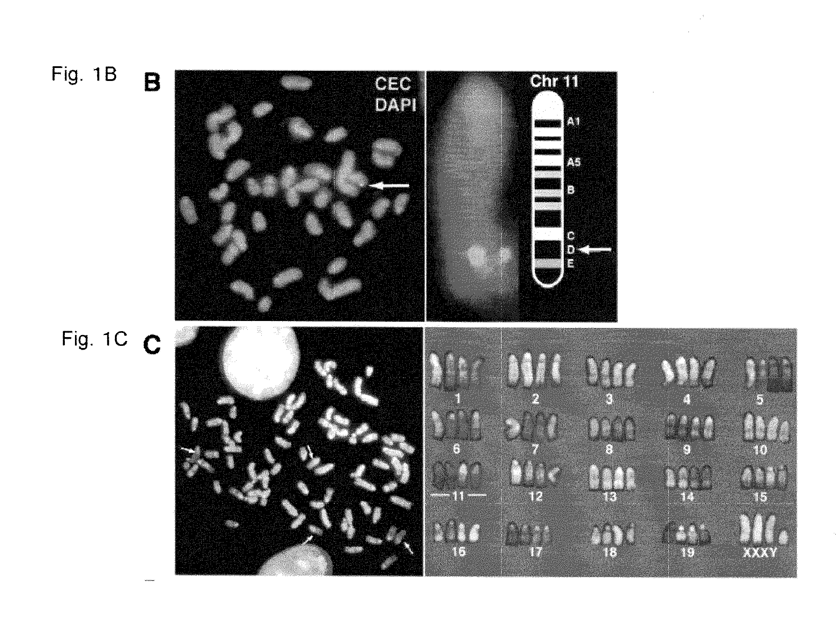

[Figs. 1BC] Fig. 1B is a drawing which is confirmed that the chromosome elimination cassette was introduced into the end of mouse 11th chromosome by FISH analysis. Fig. 1C shows the chromosome analysis of the fused cell between ES cell introduced the chromosome elimination cassette and the somatic cell. The white arrow indicates mouse 11th chromosome.

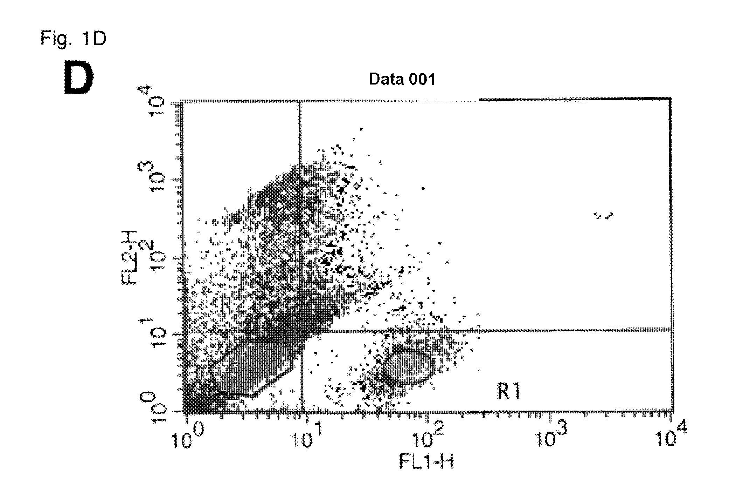

[Fig. 1D] Fig. 1D is the Facs analysis of the fused cell after treatment with Cre enzyme. The circle indicates a GFP positive cell population and the hexagon indicates the GFP negative cell population.

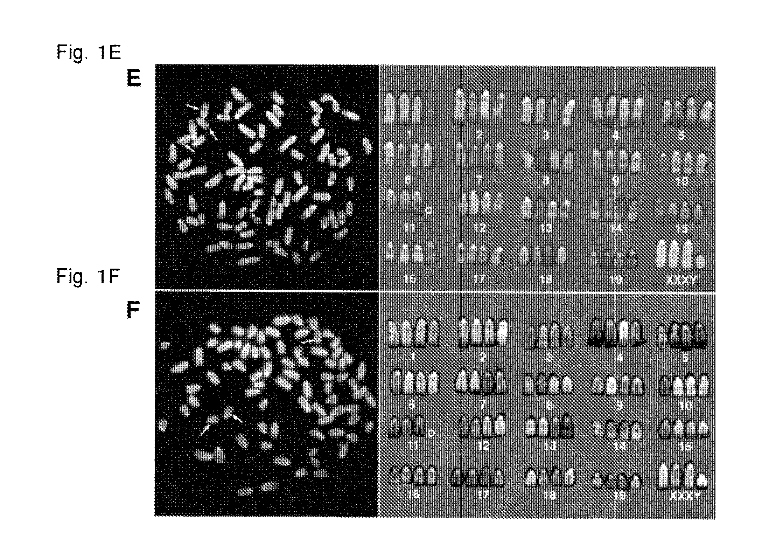

[Figs. 1EF] Fig. 1E is the chromosome analysis of the cell clone eliminating mouse 11th chromosome introduced the chromosome elimination cassette. The white circle indicates that the chromosome was eliminated. Fig. 1F shows the chromosome analysis of the cell clone, which is independent in Fig. 1E. Mouse 11th chromosome is eliminated as well as Fig. 1E.

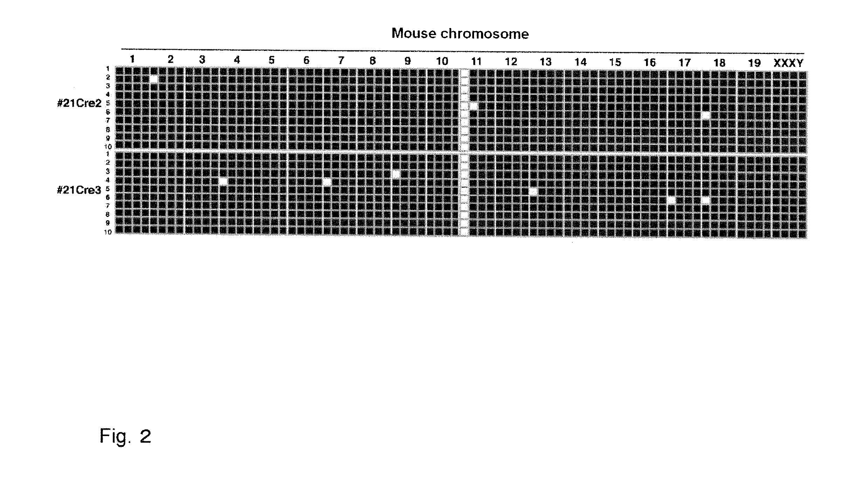

[Fig. 2] Fig. 2 shows the experimental result of the deleted chromosome distribution by the karyotype analysis of cell in an embodiment of mouse. Here, it is recognized that 11th chromosome is eliminated completely.

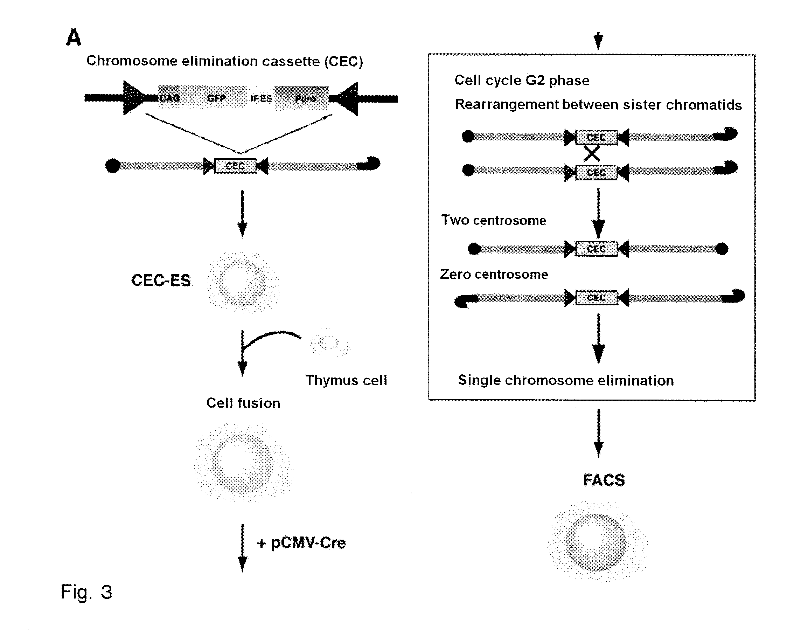

[Fig. 3] Fig. 3 is a pattern diagram explaining the mechanism of chromosome elimination in the present invention.

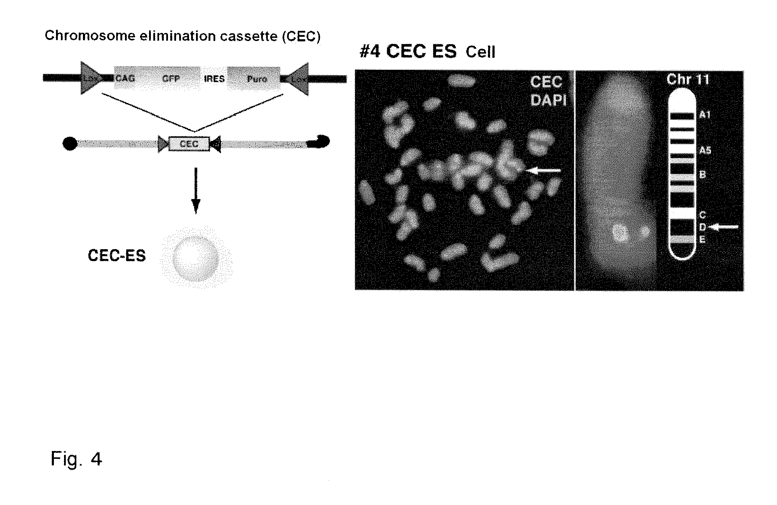

[Fig. 4] Fig. 4 is the introduction a chromosome elimination cassette to the ES cell and the FISH analysis to the introduced cassette.

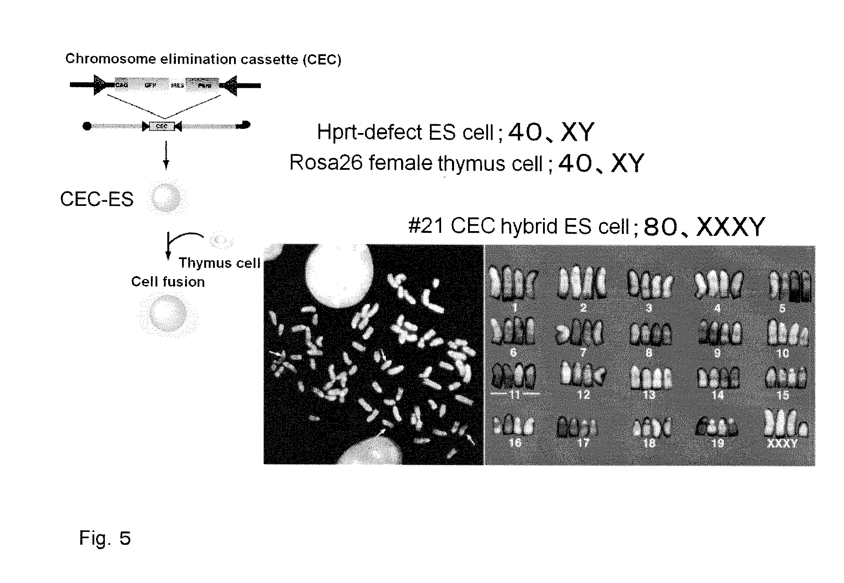

[Fig. 5] Fig. 5 is a fusion experiment between the ES cell which was introduced the chromosome elimination cassette (CEC-ES) and the somatic cell. The fused cell, which the number of chromosomes is 80, holds all of the chromosomes derived from the ES cell and the somatic cell. The number of mouse 11th chromosomes is four.

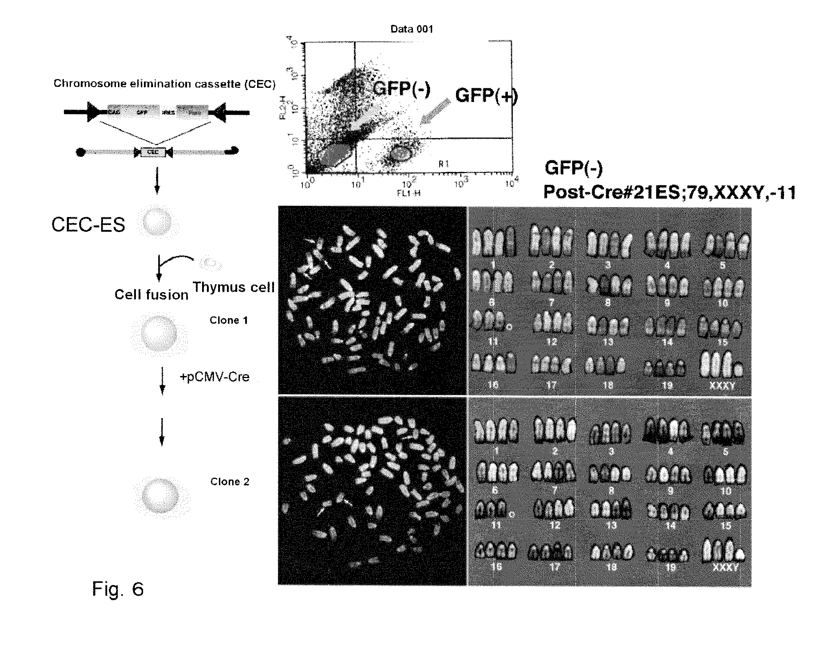

[Fig. 6] Fig. 6 shows an example which the fusion cell between the ES cell which was introduced the chromosome elimination cassette (CEC-ES) and the somatic cell is treated with Cre enzyme and eliminated 11th chromosome selectively. The cell eliminated 11th chromosome desappear the GFP fluorescence and can be easily isolated by Facs sorting. The isolated typical karyotype of 2 cell clones is shown. The 11th chromosome inserted the chromosome elimination cassette is both eliminated.

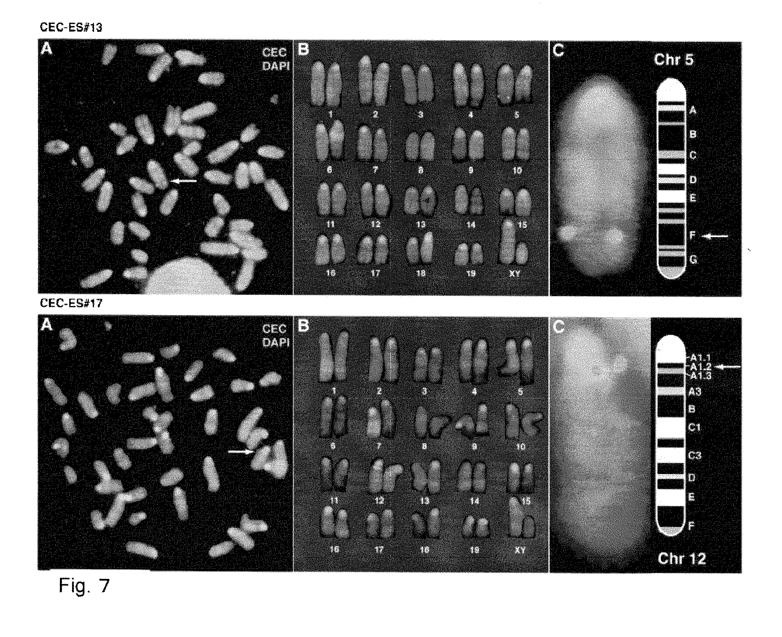

[Fig. 7] An example shows that the chromosome elimination cassette is introduced the 5th chromosome or the 12th chromosome of the mouse ES cell. The elimination cassette insertion site on the chromosome was analyzed by the FISH method.

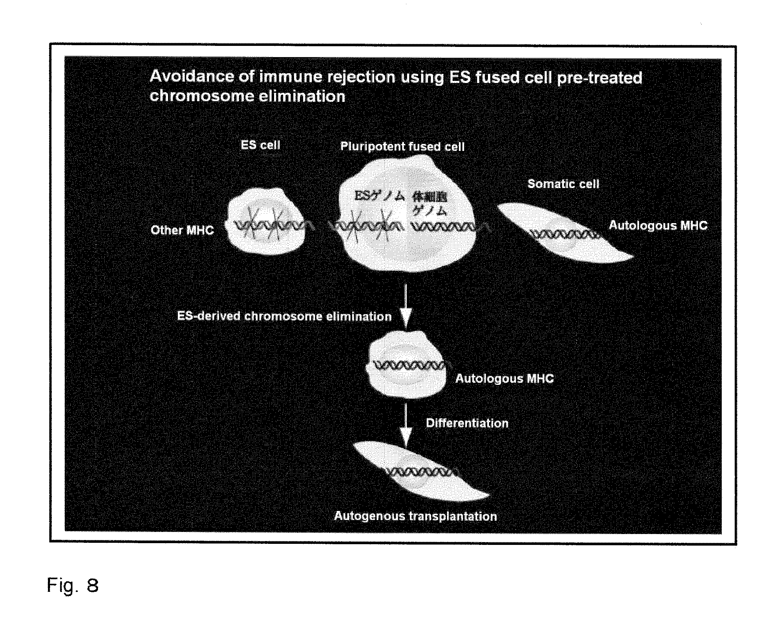

[Fig. 8] Fig. 8 shows the production pattern diagrams of the fused cell for tailor made medicine, which is a preferable embodiment of the present invention.

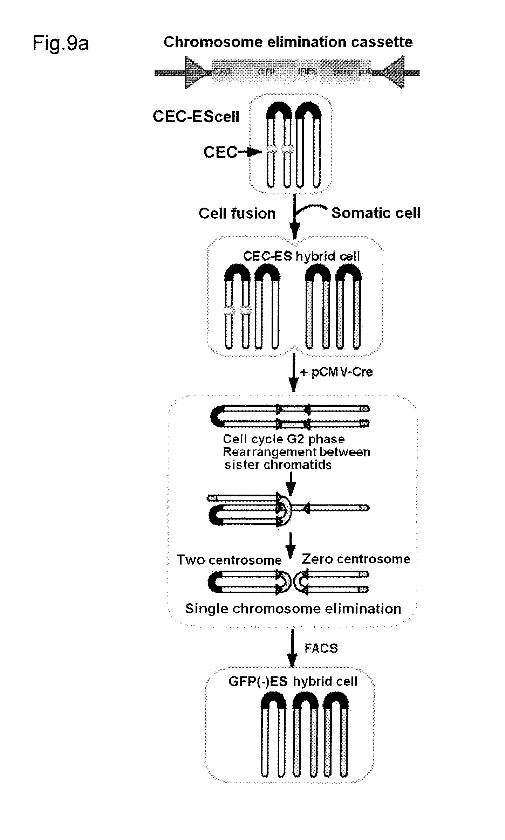

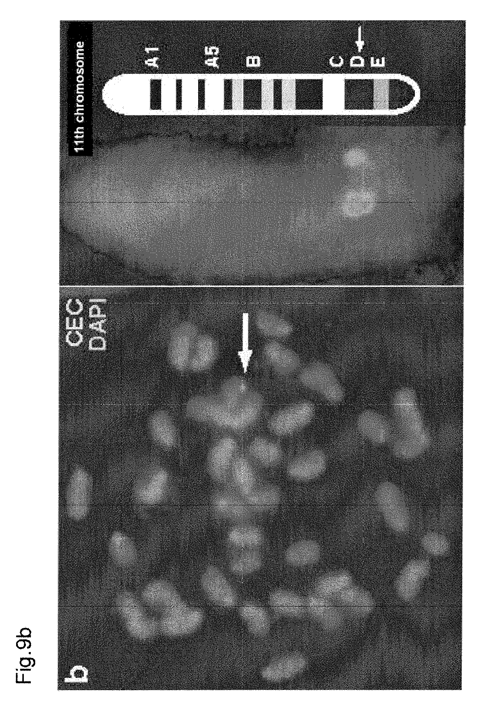

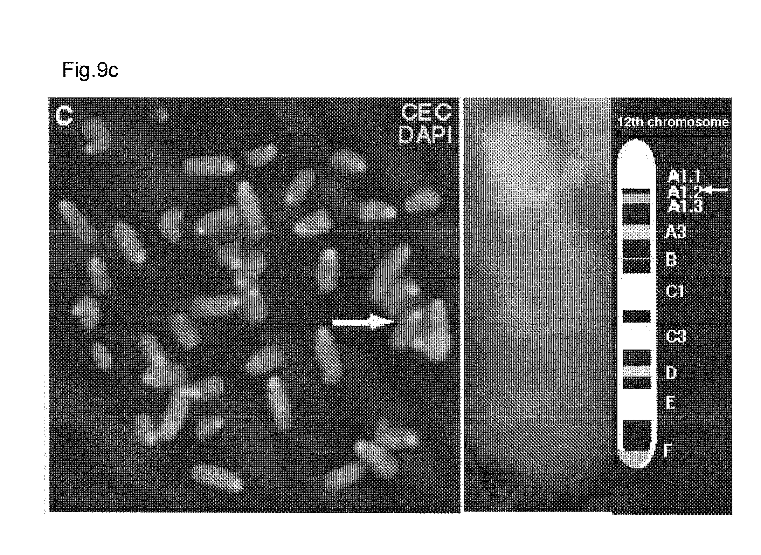

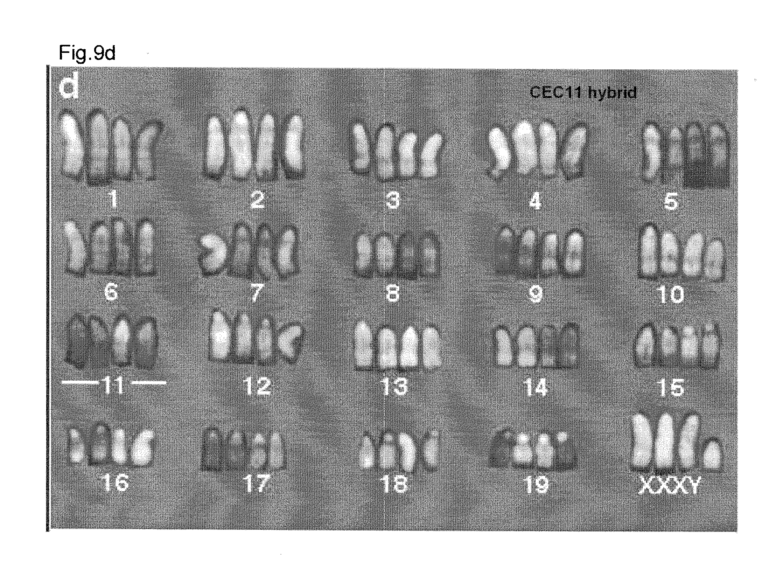

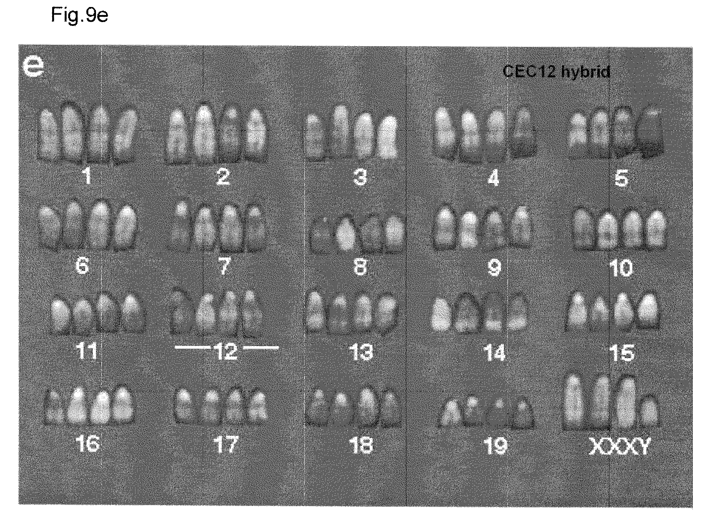

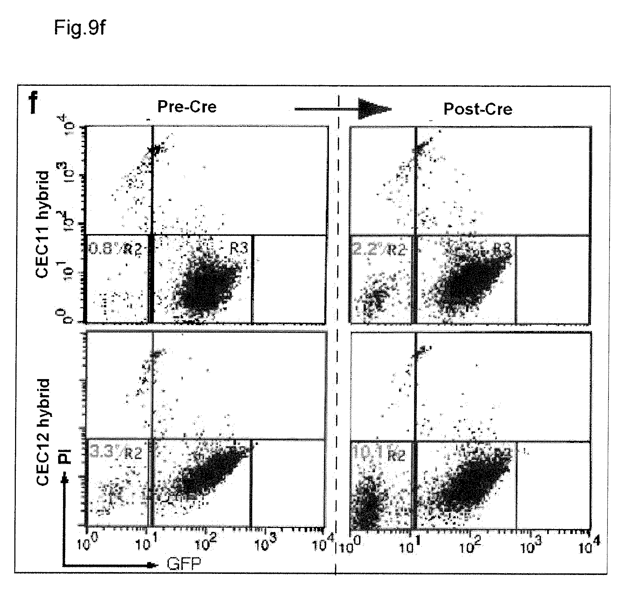

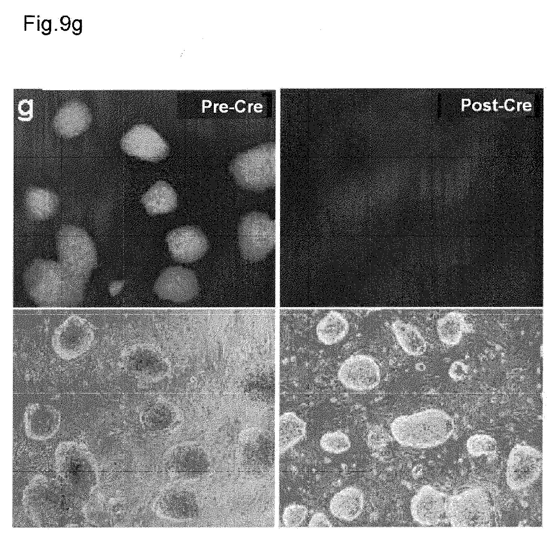

[Fig. 9a] Fig. 9 shows cell fusion between the embryonic stem (ES) cell having the chromosome elimination cassette (CEC) and the somatic cell. a. The pattern diagram of the cell fusion between the ES cell and the somatic cell, and the elimination of the target which is the ES chromosome having CEC-tag. The white arrow shows the CEC insertion site detected with a green signal.
[Fig. 9b] Fig. 9 shows the cell fusion between the embryonic stem (ES) cell having the chromosome elimination cassette (CEC) and the somatic cell. b. The map of CEC on the 11th chromosome of ES cell.
[Fig. 9c] Fig. 9 shows the cell fusion between the embryonic stem (ES) cell having the chromosome elimination cassette (CEC) and the somatic cell. c. The map of CEC on the 12th chromosome of ES cell.
[Fig. 9d] Fig. 9 shows the cell fusion between the embryonic stem (ES) cell having the chromosome elimination cassette (CEC) and the somatic cell. d. The G band formation karyotype of the hybrid cell having the 11th chromosome with the single CEC-tag (CEC11 hybrid) before the treatment of Cre (pre-Cre).
[Fig. 9e] Fig. 9 shows the cell fusion between the embryonic stem (ES) cell having the chromosome elimination cassette (CEC) and the somatic cell. e. The G band formation karyotype of the hybrid cell having the 12th chromosome with the single CEC-tag (CEC 12 hybrid) pre-Cre.
[Fig. 9f] Fig. 9 shows the cell fusion between the embryonic stem (ES) cell having the chromosome elimination cassette (CEC) and the somatic cell. f. The FACS analysis of the CEC 11 hybrid cell and the CEC12 hybrid cell pre-Cre and after the treatment with Cre (post-Cre). The GFP negative hybrid cell is in the boxed R2, and, on the other hand, the GFP positive hybrid cell is in the boxed R3.
[Fig. 9g] Fig. 9 shows the cell fusion between the embryonic stem (ES) cell having the chromosome elimination cassette (CEC) and the somatic cell. g. The GFP expression of the CEC11 hybrid clone pre-Cre and post-Cre.

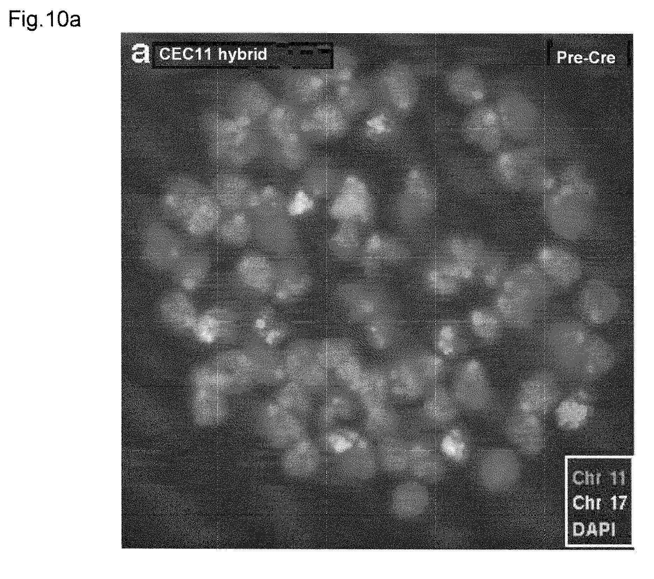

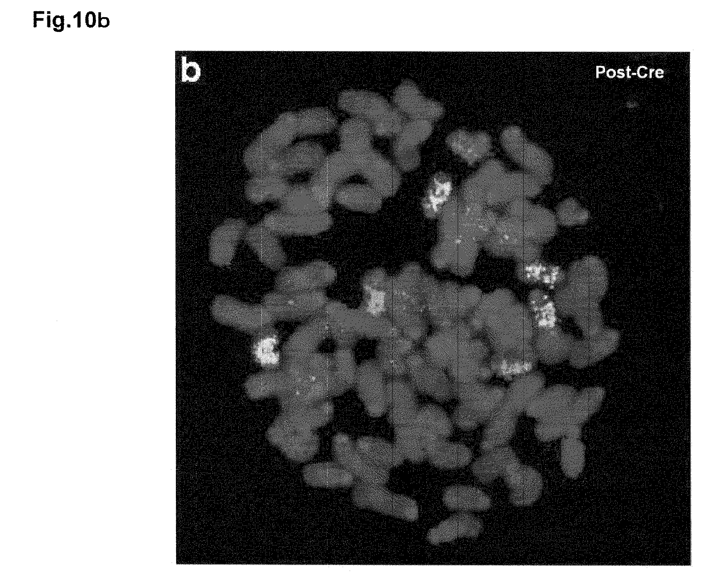

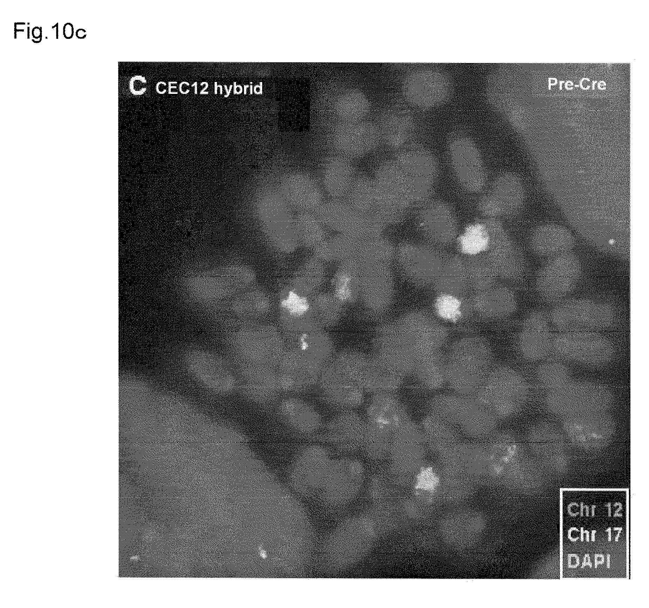

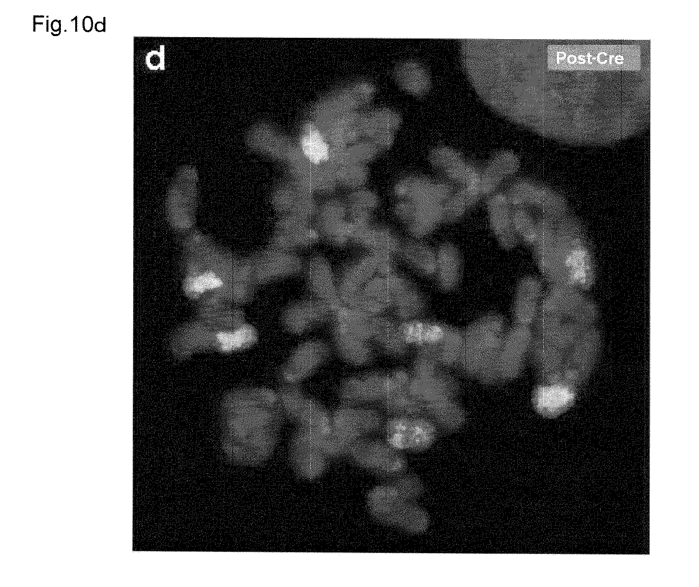

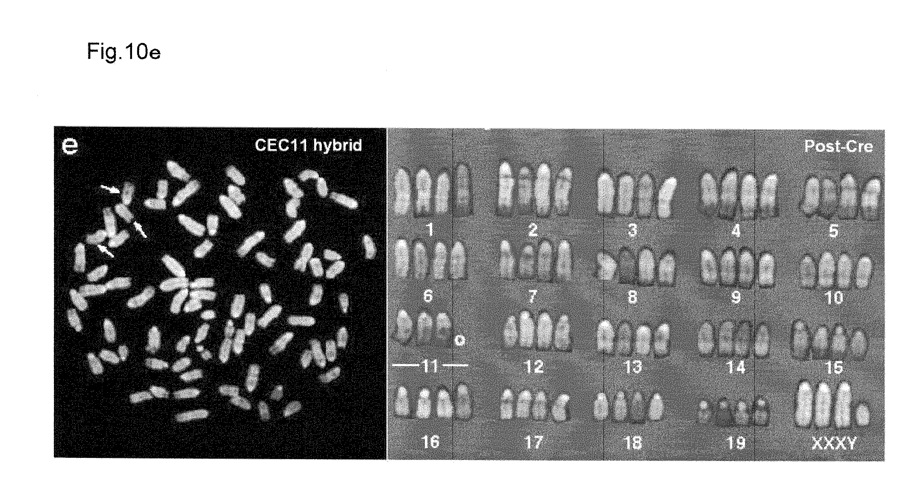

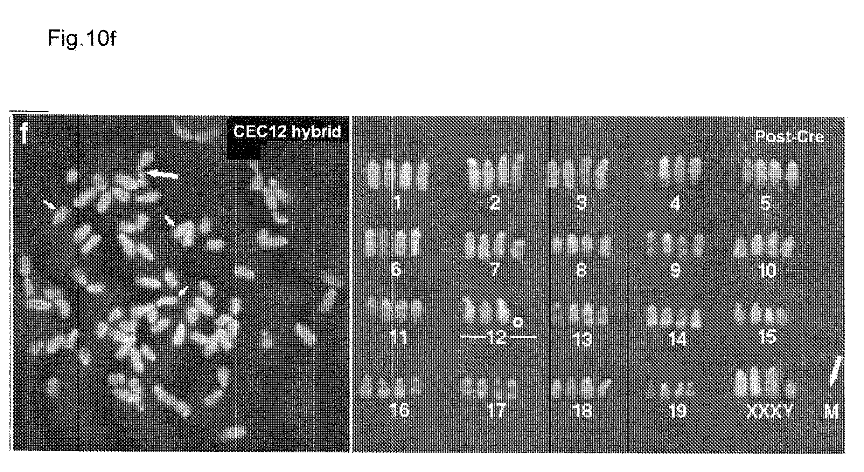

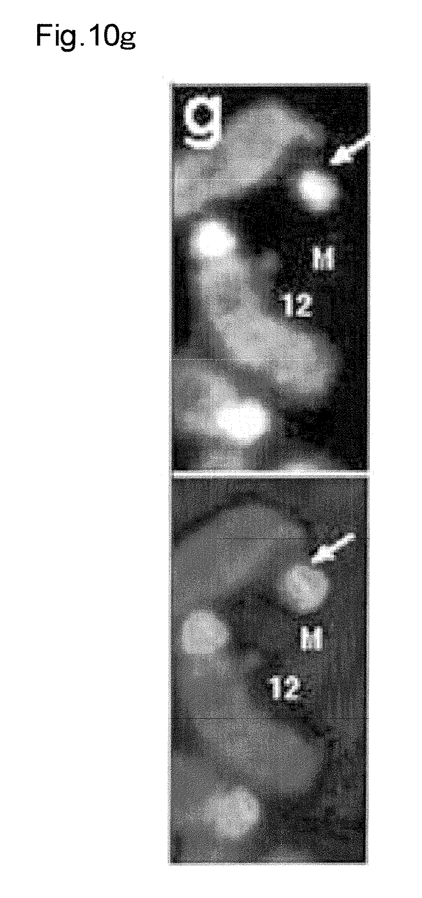

[Fig. 10a] Fig. 10 shows the karyotype of the chromosome painting and the metaphase after elimination of the 11th chromosome and the 12th chromosome. a. The chromosome painting of the CEC11 before the treatment with Cre (pre-Cre). The four 11th chromosomes in the nucleus are recognized as the red painting signal.
[Fig. 10b] Fig. 10 shows the karyotype of the chromosome painting and the metaphase after elimination of the 11th chromosome and the 12th chromosome. b. The chromosome painting of the CEC11 hybrid cell after the treatment with Cre (post-Cre). The three 11th chromosome in each nucleus are arosed by the elimination of the chromosome so that it can be recognized as the red painting signal.
[Fig. 10c] Fig. 10 shows the karyotype of the chromosome painting and the metaphase after elimination of the 11th chromosome and the 12th chromosome. c. The chromosome painting of the CEC12 hybrid cell pre-Cre. The four 12th chromosomes in the nucleus are recognized as the red painting signal.
[Fig. 10d] Fig. 10 shows the karyotype of the chromosome painting and the metaphase after elimination of the 11th chromosome and the 12th chromosome. d. The chromosome painting of the CEC12 hybrid cell post-Cre. The three 12th chromosome in each nucleus are arosed by the elimination of the chromosome so that it can be recognized as the red painting signal.
[Fig. 10e] Fig. 10 shows the karyotype of the chromosome painting and the metaphase after elimination of the 11th chromosome and the 12th chromosome. e. The G band formation karyotype of the CEC11 hybrid cell post-Cre. The white arrow indicates the three 11th chromosomes in the nucleus. The white circle indicates the selective elimination of one 11th chromosome.
[Fig. 10f] Fig. 10 shows the karyotype of the chromosome painting and the metaphase after elimination of the 11th chromosome and the 12th chromosome. f. The G band formation karyotype of the CEC12 hybrid cell post-Cre. The white arrow indicates the three 12th chromosomes in the nucleus. The white circle indicates the selective elimination of one 12th chromosome.
[Fig. 10g] Fig. 10 shows the karyotype of the chromosome painting and the metaphase after elimination of the 11th chromosome and the 12th chromosome. g. The minute chromosome (M) in the CEC12 hybrid cell post-Cre. Most small chromosomes (white arrow) is consisting of the centromere heterochromatin so that it can be shown by FISH.

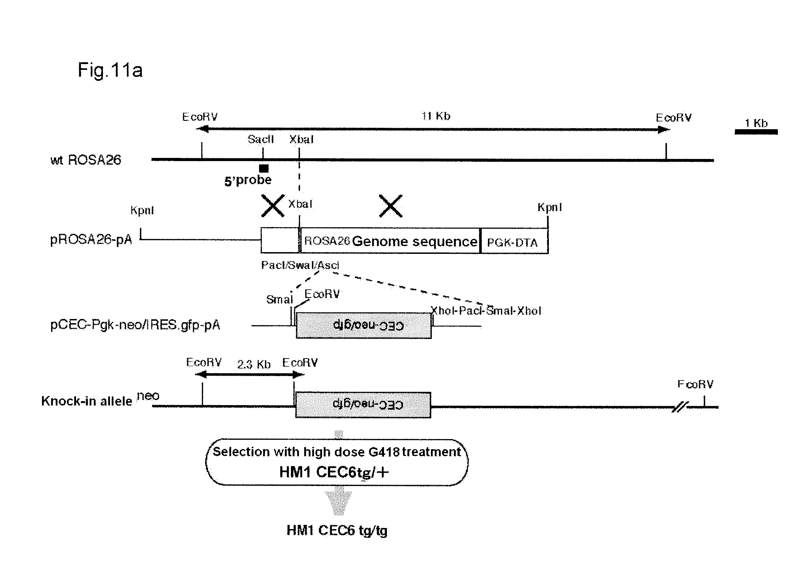

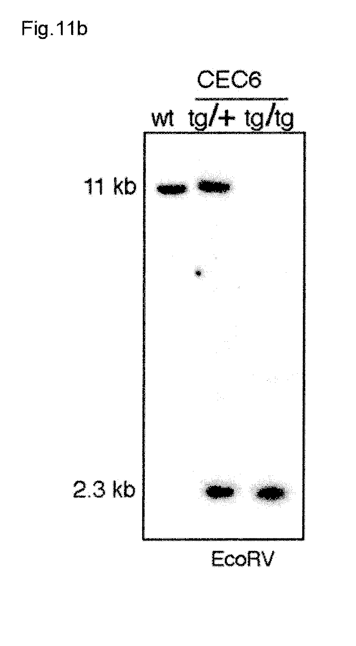

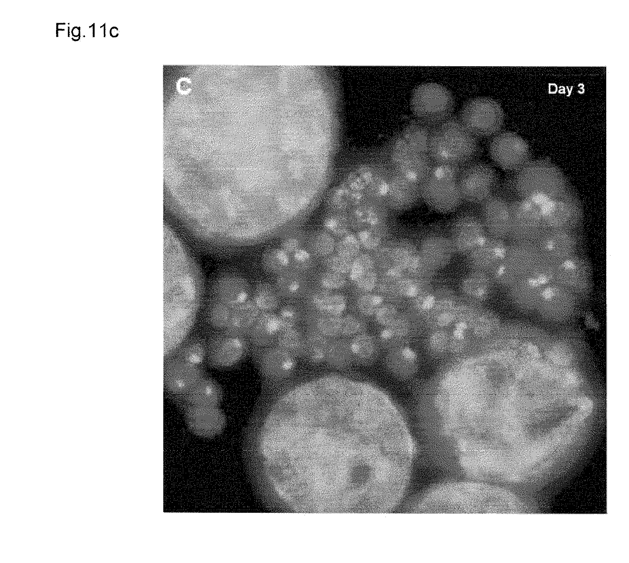

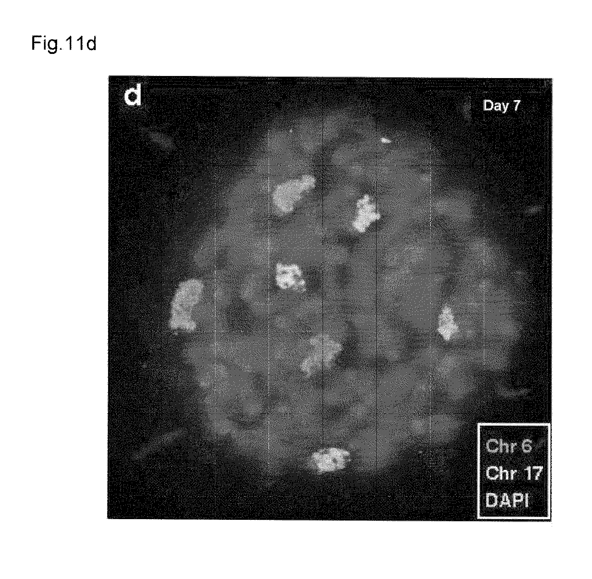

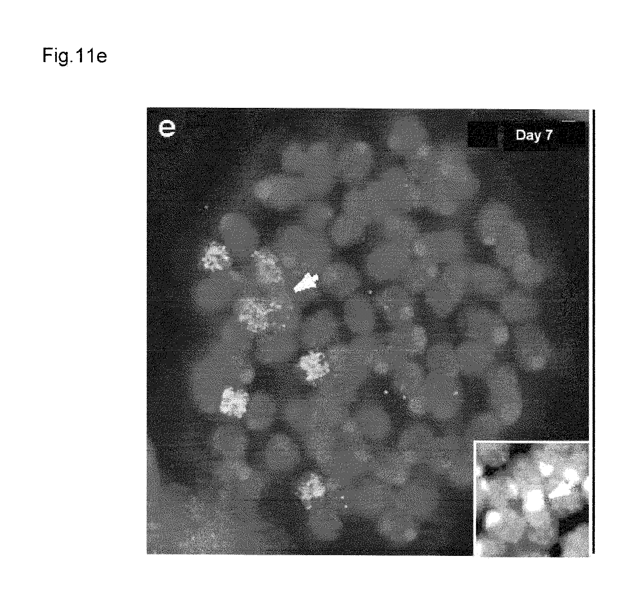

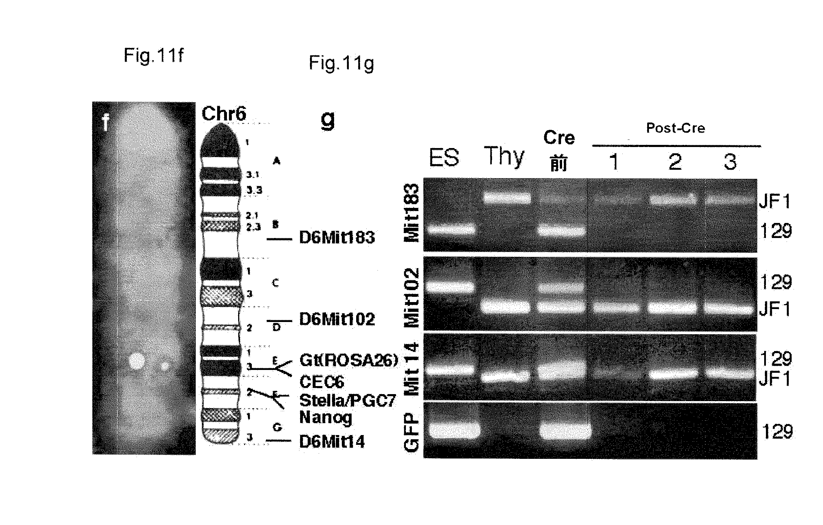

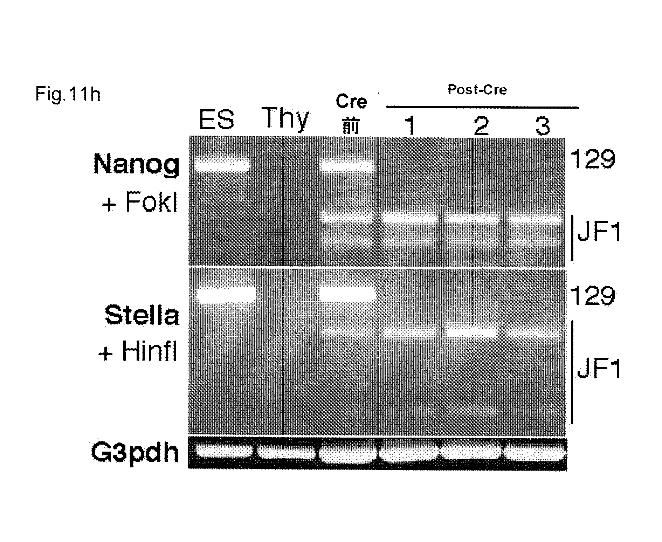

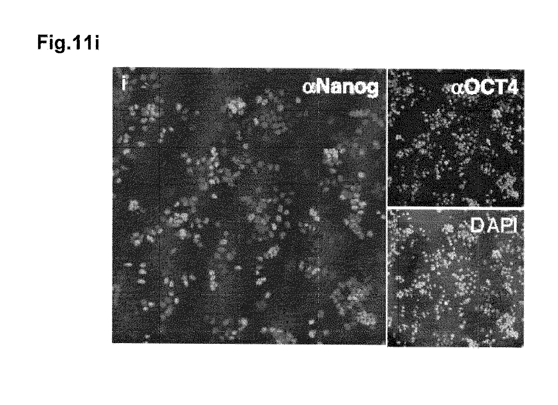

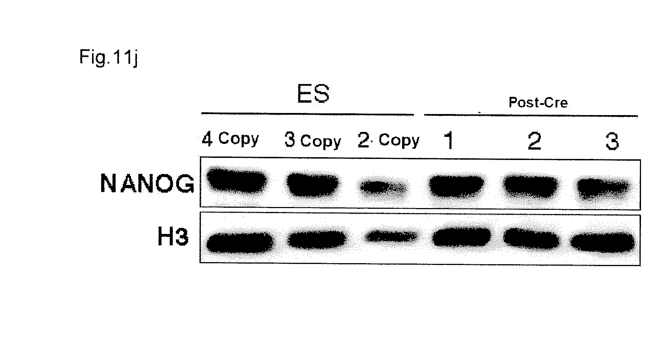

[Fig. 11a] Fig. 11 shows the elimination of the target 6th chromosome pair derived from the ES cell in the hybrid cell nucleus. a. The pattern diagram of the CEC-neo / gfp cassette insertion to the Gt(ROSA)26Sor locus by the homologous recombination (CEC6 ^{tg/+}) The ES cell homozygote (CEC6 ^{tg/tg}) to CEC was selected by the high dose 0418 treatment.
[Fig. 11b] Fig. 11 shows the elimination of the target 6th chromosome pair derived from the ES cell in the hybrid cell nucleus. b. The southern plot hybridization analysis of the homozygote recombination in the CEC6 ^{tg/+} ES cell and the CEC6 ^{tg/tg} ES cell. The 11kb band is specific to the wild type allele, and, on the other hand, the 2.3kb band is specific to the knock-in allele.
[Fig. 11c] Fig. 11 shows the elimination of the target 6th chromosome pair derived from the ES cell in the hybrid cell nucleus. c. The chromosome painting of the CEC6 (tg / tg) hybrid cell having two 6th chromosomes in the nucleus three days after the treatment with Cre (red).
[Fig. 11d] Fig. 11 shows the elimination of the target 6th chromosome pair derived from the ES cell in the hybrid cell nucleus. d. The chromosome painting of the GFP negative CEC6 (tg / tg) hybrid cell haivng three 6th chromosomes (red) and four 17th chromosome (green) in the nucleus seven days after the treatment with Cre.
[Fig. 11e] Fig. 11 shows the elimination of the target 6th chromosome pair derived from the ES cell in the hybrid cell nucleus. e. The chromosome painting of the GFP negative CEC (tg / tg) hybrid cell having 2 arm chromosomes formed by the Robertsonian translocation between two 6th chromosomes in the nucleus seven days after the treatment with Cre and the normal 6th chromosome.
[Fig. 11fg] Fig. 11 shows the elimination of the target 6th chromosome pair derived from the ES cell in the hybrid cell nucleus. f. The position of CEC6 (green) and the marker gene locus on the 6th chromosome. g. The determination of the 6th chromosome origin by the genome PCR analysis by using three marker gene loci on the 6th chromosome.
[Fig. 11h] Fig. 11 shows the elimination of the target 6th chromosome pair derived from the ES cell in the hybrid cell nucleus. h. The determination of the Nanog and STELLA /PGC7 transcript origin by the RT-PCR analysis of the polymorphism product.
[Fig. 11i] Fig. 11 shows the elimination of the target 6th chromosome pair derived from the ES cell in the hybrid cell nucleus. i. The expression of NANOG and OCT4 in the hybrid cell eliminated the 6th chromosome derived from the ES cell (immunohistochemistry staining).
[Fig. 11j] Fig. 11 shows the elimination of the target 6th chromosome pair derived from the ES cell in the hybrid cell nucleus. j. The expression level of NANOG in the hybrid cell post-Cre by the western blot hybridization.

### Explanation of the sequence table

Sequence number 1 is the loxP sequence.
Sequence number 2 is the IRES sequence.
Sequence number 3 is the CAG sequence.
Sequence number 4 is the FRT part.
Sequence number 5 is the attB sequence.
Sequence number 6 is the attp sequence.
Sequence number 7 is the sequence of the green fluorescence protein.
Sequence number 8 is the amino acid sequence of the green fluorescence protein.
Sequence number 9 is the coding sequence of the puromycin resistance gene.
Sequence number 10 is the amino acid sequence of the puromycin resistance gene.
Sequence numbers 11 are all the CEC sequences.
Sequence number 12 is D6Mit183 primer 1: 5'-TTCTCAATGAACACTAGAACATTCG-3'.
Sequence number 13 is D6Mit183 primer 2: 5'-AAAACACAGGTAGAAAACATACATACA-3'.
Sequence numbers 14 are D6Mit102 primer 1: 5'-CCATGTGGATATCTTCCC TTG-3'.
Sequence numbers 15 are D6Mit102 primer 2: 5'-GTATACCCAGTTGTAAATCTTGTGTG-3'.
Sequence numbers 16 are D6Mit14 primer 1: 5'-ATGCAGAAACATGAGTGG GG-3'.
Sequence numbers 17 are D6Mit14 primer 2: 5'-CACAAGGCCTGATGACCTC T-3'.
Sequence number 18 is GFPF primer: 5'-CGTAAACGGCCACAAGTTCA-3'.
Sequence number 19 is GFPR primer: 5'-CGCTTTACTTGTACAGCTCGT-3'.
Sequence number 20 is NanogF1 primer: 5'-GCGCA mm AGCACCCCAC A-3'.
Sequence number 21 is NanogR1 primer: 5'-GTTCTAAGTCCTAGGTTTGC-3'.
Sequence number 22 is STELLA / PGC7F primer: 5'-ACAGACTGACTGCTA ATTGG-3'.
Sequence number 23 is STELLA / PGC7R primer: 5'-GGAAATTAGAACGTACATACTCC-3'.
Sequence number 24 is G3pdhF primer: 5'-TGAAGGTCGGTGTGAACGGATTTGGC-3'.
Sequence number 25 is G3pdhR primer: 5'-CATGTAGGCCATGAGGTCCAC-3'.

### DESCRIPTION OF THE PREFERRED EMBODIMENTS

The most preferred embodiment to implement the present invention is explained below. It should be understood throughout the present specification that articles for singular forms (e.g., "a", "an", "the", or the like in English and the corresponding articles, adjectives, or the like in other languages) include plural referents unless the context clearly dictates otherwise. It should be also understood that the terms as used the present specification have definitions typically used in the art unless otherwise mentioned.

### (General art)

The molecular biological approach and the biochemical approach, the microbiological approach which are used in the present specification are widely known in the field and commonly used, for example it is described in Maniatis, T. et al. (1989). Molecular Cloning: A Laboratory Manual, Cold Spring Harbor and the 3^{rd} Ed. (2001); Ausubel, F. M. (1987). Current Protocols in Molecular Biology, Greene Pub. Associates and Wiley-Interscience; Ausubel, F. M. (1989). Short Protocols in Molecular Biology; A Compendium of Methods from Current Protocols in Molecular Biology, Greene Pub. Associates and Wiley-Interscience; Sambrook, J. et al. (1989). Molecular Cloning: A Laboratory Manual, Cold Spring Harbor; Innis, M. A. (1990). PCR Protocols: A Guide to Methods and Applications, Academic Press; Ausubel, F. M. (1992). Short Protocols in Molecular Biology: A Compendium of Methods from Current Protocols in Molecular Biology, Greene Pub. Associates; Ausubel, F. M. (1995). Short Protocols in Molecular Biology: A Compedium of Methods from Current Protocols in Molecular Biology, Greene Pub. Associates; Innis, M. A. et al. (1995). PCR Strategies, Academic Press; Ausubel, F. M. (1999). Short Protocols in Molecular Biology: A Compedium of Methods from Current Protocols in Molecular Biology, Wiley, and annual updates; Sninsky, J. J. et al. (1999). PCR Applications: Protocols for Functional Genomics, Academic Press; Bessatsu Jikken Igaku "Idenshidounyu & Hatsugenkaisekijikkennhou" [Experimental Method for Gene introduction & Expression Analysis], Yodo-sha, 1997; or the like. And these related section (everything might be included) is applied as reference in the present specification.

### (Explanation of a term)

The term used as follows in the present specification is explained.

### (Cell Biology)

As used in the present specification, the term "cell" is in its broadest sense in the art and refers to a living body which is a structural unit of tissue of a multicellular organism, is surrounded by a membrane structure which isolates it from the outside, has the capability of self replicating, and has genetic information and a mechanism for expressing it. Cells used in the present specification may be naturally occurring cells or artificially modified cells (e.g., fusion cells, genetically modified cells, or the like).

As used in the present specification, the term "stem cell" refers to a cell capable of self replication and pluripotency. Typically, stem cells can regenerate an injured tissue. Stem cells used in the present specification may be, but are not limited to, embryonic stem (ES) cells or tissue stem cells (also called tissue-specific stem cell, or somatic stem cell). Any artificially produced cell which can have the above described abilities (e.g., fusion cells, reprogrammed cells, or the like used in the present specification) may be a stem cell. ES cells are pluripotent stem cells derived from early embryos. ES cell was first established in 1981, which has also been applied to production of knockout mice since 1989. In 1998, human ES cell was established, which is currently becoming available for regenerative medicine. Tissue stem cells have a limited level of differentiation unlike ES cells, and tissue stem cells are present at particular locations in tissues and have an undifferentiated intracellular structure. Tissue stem cells have a higher nucleus /cytoplasm ratio and have few intracellular organelles. Most tissue stem cells have pluripotency, a long cell cycle, and proliferative ability beyond the life of the individual. As used in the present specification, stem cells may be preferably ES cells, though tissue stem cells may also be employed depending on the circumstance.

Tissue stem cells are separated into categories of sites from which the cells are derived, such as dermal system, digestive system, bone marrow system, nervous system, and the like. Tissue stem cells in dermal system include epidermal stem cells, hair follicle stem cells, and the like. Tissue stem cells in digestive system include pancreatic (common) stem cells, liver stem cells, and the like. Tissue stem cells in bone marrow system include hematopoietic stem cells, mesenchymal stem cells, and the like. Tissue stem cells in nervous system include neural stem cells, retinal stem cells, and the like.

As used in the present specification, the term "somatic cell" refers to any cell other than germ cells, such as egg, sperm, or the like, which does not directly transfer its DNA to the next generation. Typically, somatic cells have limited or no pluripotency. Somatic cells used in the present specification may be naturally occurring or genetically modified.

The origin of a cell is categorized into a stem cell derived from the ectoderm, endoderm, or mesoderm. Stem cells derived from ectodermal are mostly present in brain, including neural stem cells. Stem cells derived from endodermal are mostly present in bone marrow, including blood vessel stem cells, hematopoietic stem cells, mesenchymal stem cells, and the like. Stem cells derived from mesoderm are mostly present in organs, including liver stem cells, pancreatic stem cells, and the like. Somatic cells may be in the present specification derived from any germ layer. Preferably, somatic cells, such as lymphocytes, spleen cells or testis-derived cells, and bone marrow cells may be used.

As used in the present specification, the term "isolated" means that materials naturally accompanying in normal circumstances are at least reduced, or preferably substantially completely eliminated. Therefore, the term "isolated cell" refers to a cell substantially free from other accompanying in natural circumstances substances (e.g., other cells, proteins, nucleic acids, or the like). The term "isolated" in relation to nucleic acids or polypeptides means that, for example, nucleic acids or polypeptides are substantially free from cellular substances or culture media when they are produced by recombinant DNA techniques; or precursory chemical substances or other chemical substances when they are chemically synthesized. Isolated nucleic acids are preferably free from sequences naturally flanking nucleic acids within an organism from which the nucleic acids are derived (i.e., sequences positioned at the 5' terminus and the 3' terminus of the nucleic acid).

As used in the present specification, the term "established" in relation to cells refers to a state of cell in which a particular property (e.g., pluripotency) of cell is maintained and the cell undergoes stable proliferation under culture conditions. Therefore, established stem cells maintain pluripotency. In the present invention, the use of established stem cells is preferable since the step of collecting stem cells from a host can be avoided.

As used in the present specification, the term "non-embryonic" refers to not being directly derived from early embryos. Therefore, the term "non-embryonic" refers to cells derived from parts of the body other than early embryos. Also, modified ES cells (e.g., genetically modified or fusion ES cells, or the like) are encompassed by non-embryonic cells.

As used in the present specification, the term "differentiated cell" refers to a cell having a specialized function and form (e.g., myocytes, nerve cells, or the like), and differentiated cells have no or little pluripotency unlike stem cells. Examples of differentiated cells include epidermic cells, pancreatic parenchymal cells, pancreatic duct cells, hepatic cells, blood cells, cardiac myocytes, skeletal myocytes, osteoblasts, skeletal myoblasts, nerve cells, vascular endothelial cells, pigment cells, smooth myocytes, fat cells, bone cells, chondrocytes, and the like. Therefore, in the case when a certain differentiated cells are treated with the reprogramming factor of the present invention (for example, refere to WOO3/027277 WOO3/027278, or the like) and acquire pluripotency, such a differentiated cell may be used as a somatic cell in the present invention or the substitutes.

As used in the present specification, the term "differentiation" or "cell differentiation" refers to a phenomenon that two or more types of cells having qualitative differences in form and/or function occur in a daughter cell population derived from the division of a single cell. Therefore, "differentiation" includes a process during which a population (family tree) of cells, which do not originally have a specific detectable feature, acquire a feature such as production of specific proteins, or the like. Presently, cell differentiation is generally considered to be a state of cell in which a specific group of genes in the genome are expressed and the cell differentiation can be identified by searching for intracellular or extracellular agents or conditions elicited the above described state of gene expression. Differentiated cells are stable in principle, and particularly, animal cells which have been once differentiated are rarely differentiated into other types of cells. Therefore, it is considerably useful that the inheritable genetic modification of multipotential cell can be easily performed by the acquired pluripotent cells in the present invention.

As used in the present specification, the term "pluripotency" refers to a nature of a cell, i.e., an ability to differentiate into one or more, preferably two or more, tissues or organs. Therefore, the terms "pluripotent" and "undifferentiated" are in the present specification used interchangeably unless otherwise mentioned. Typically, the pluripotency of a cell is limited as the cell is developed, and in an adult, cells constituting a tissue or organ rarely alter to different cells Therefore, the pluripotency is usually lost. Particularly, epithelial cells resist altering to other types of epithelial cells. Such alteration typically occurs in pathological conditions, and is called metaplasia. However, since mesenchymal cells tend to easily undergo metaplasia, i.e., alter to other mesenchymal cells, with relatively simple stimuli, mesenchymal cells have a high level of pluripotency. ES cells have pluripotency. Tissue stem cells have pluripotency. As used in the specification, the term "totipotency" refers to the pluripotency of a cell, such as a fertilized egg, to differentiate into all cells constituting an organism and thus, the term "pluripotency" may include the concept of totipotency. An example of an in vitro assay for determining whether or not a cell has pluripotency, includes, but is not limited to, culture under conditions for inducing the formation and differentiation of embryoid bodies. Examples of an in vivo assay for determining the presence or absence of pluripotency, include, but are not limited to, implantation of a cell into an immunodeficient mouse so as to form teratoma, injection of a cell into a blastocyst so as to form a chimeric embryo, implantation of a cell into a tissue of an organism (e.g., injection of a cell into ascites) so as to undergo proliferation, and the like.

Cells used in the present invention include cells derived from any organisms (e.g., any multicellular organisms (e.g., animals (e.g., vertebrates, invertebrate), plants (monocotyledons, dicotyledons, or the like))). Preferably, the animal is a vertebrate (e.g., Myxiniformes, Petronyzoniformes, Chondrichthyes, Osteichthyes, amphibian, reptilian, avian, mammalian, or the like), more preferably mammalian (e.g., monotremata, marsupialia, edentate, dermoptera, chiroptera, carnivore, insectivore, proboscidea, perissodactyla, artiodactyla, tubulidentata, pholidota, sirenia, cetacean, primates, rodentia, lagomorpha, or the like). More preferably, primates (e.g., chimpanzee, Japanese macaque, human, or the like) are used. Most preferably, a cell derived from human is used.

Any organ may be targeted by the present invention and a tissue or cell targeted by the present invention may be derived from any organ. As used in the present specification, the term "organ" refers to a morphologically independent structure localized at a particular portion of an individual organism in which a certain function is performed. In multicellular organisms (e.g., animals, plants), an organ consists of several tissues spatially arranged in a particular manner, a tissue being composed of a number of cells. An example of such an organ includes an organ relating to the vascular system. In one embodiment, organs targeted by the present invention include, but are not limited to, skin, blood vessel, cornea, kidney, heart, liver, umbilical cord, intestine, nerve, lung, placenta, pancreas, brain, peripheral limbs, retina, and the like. Examples of cells differentiated from pluripotent cells in the present specification include epidermic cells, pancreatic parenchymal cells, pancreatic duct cells, hepatic cells, blood cells, cardiac myocytes, skeletal myocytes, osteoblasts, skeletal myoblasts, nerve cells, vascular endothelial cells, pigment cells, smooth myocytes, fatcells, bone cells, chondrocytes, and the like.

As used in the specification, the term "tissue" refers to an aggregate of cells having substantially the same function and/or form in a multicellular organism. "Tissue" is typically an aggregate of cells of the same origin, but may be an aggregate of cells of different origins as long as the cells have the same function and/or form. Therefore, when stem cells of the present invention are used to regenerate tissue, the tissue may be composed of an aggregate of cells of two or more different origins. Typically, a tissue constitutes a part of an organ. Animal tissues are separated into epithelial tissue, connective tissue, muscular tissue, nervous tissue, and the like, on a morphological, functional, or developmental basis. Plant tissues are roughly separated into meristematic tissue and permanent tissue according to the developmental stage of the cells constituting the tissue, alternatively, tissues may be separated into single tissues and composite tissues according to the type of cells constituting the tissue, and thus, tissues are separated into various categories.

### (Molecular biology, Gene manipulation)

A gene construct or a vector is introduced into a cell in the method of the present invention.

In the present specification, a "gene construct", a "nucleic acid construct", or a "gene cassette" is used exchangeably and refers to nucleic acid molecules (for example, DNA, RNA) which encodes a gene, a nucleic acid sequence which includes a control sequence (for example, promotor) connected to the nucleic acid molecule to be able to operate (that is, the expression of the nucleic acid can be regulated), and if needed a nucleic acid molecule which includes a heterologous gene connected to the control sequence (for examle, promotor)to be able to operate (that is, in frame). The gene cassette or the gene construct can be used with combining other regulation elements if needed, which is also included in the range of the present invention. A preferable expression cassette is the gene cassette or nucleic acid contruct, which is cut by the specific restriction enzyme and can be collected easily.

Any method for introducing DNA into cells can be used as an vector introduction method, including, for example, transfection, transduction, transformation, and the like (e.g., electroporation method, particle gun (gene gun) method, and the like). Such an introducting nucleic acid molecules art described above is common knowledge in the field and used commonly, for example, it is described in Ausubel F. A. et al., (1988), Current Protocols in Molecular Biology, Wiley, New York, NY; Sambrook J. et al. (1987) Molecular Cloning: A Laboratory Manual, 2nd Ed. and the third edition, Cold Spring Harbor Laboratory Press, Cold Spring Harbor, NY, Bessatsu Jikken Igaku "Idenshidonyu & Hatsugenkaisekijikkenhou" [Experimental Method for Gene introduction & Expression Analysis], Yodo-sha, 1997, or the like. The gene introduction can be confirmed by using the methods which are described in the present specification such as Northern blot, Western blot analysis, or the like, or other commonly used art.

When a gene is mentioned in the present specification, the term "vector" or "recombinant vector" refers to a vector capable of transferring a polynucleotide sequence of interest to a target cell. Such a vector is capable of self-replication or incorporation into a chromosome in a host cell (e.g., a prokaryotic cell, yeast, an animal cell, a plant cell, an insect cell, an individual animal, and an individual plant, or the like), and contains a promoter at a site suitable for transcription of a polynucleotide of the present invention.

Any method for introduction of DNA can be used in the present specification as a method for introduction of a vector, including, for example, transfection, transduction, transformation, and the like (e.g., a calcium phosphate method, the ribosome method, the DEAE dextran method, electroporation method [Methods. Enzymol., 194, 182 (1990)], a particle gun (gene gun) method, and the like), a lipofection method, a spheroplast method (Proc.Natl.Acad.Sci.USA.84,1929(1978)), a lithium acetate method (J.Bacteriol.,153,163(1983)), a method described in Proc. Natl. Acad. Sci. USA, 75, 1929 (1978), and the like.

As used in the present specification, a "gene introducing reagent" refers to a reagent used for accelerating the introductory efficiency in the introducing method of the nucleic acid (which is usually encoded a gene, but is not limited to). Such a transgenics reagent, for example, cationic polymer, cationic lipid, polyamine system reagent, polyimine system reagent, calcium phosphate, or the like. is included, but is not limited to. An example of the reagent used in the case of transfection is included from various sources which is commercially available, for example, Effectene Transfection Reagent (cat. no. 301425, Qiagen, CA), TransFastTM Transfection Reagent (E2431, Promega, WI), Tfx^{™}-20 Reagent (E2391, Promega, WI), SuperFect Transfection Reagent (301305, Qiagen, CA), PolyFect Transfection Reagent (301105, Qiagen, CA), LipofectAMINE 2000 Reagent (11668-019, Invitrogen corporation, CA), JetPEI(x4) conc. (101-30, Polyplus-transfection, France), and ExGen 500 (RO511, Fermentas Inc., MD), or the like is included, but is not limited to. In the present invention, when introducing the nucleic acid molecule of the present invention into a cell, such a transgenics reagent can be used.

Examples of "recombinant vector" for prokaryotic cells include pBTrp2, pBTac1, pBTac2 (all commercially available from Roche Molecular Biochemicals), pKK233-2 (Pharmacia), pSE280 (Invitrogen), pGEMEX-1 [Promega], pQE-8 (QIAGEN), pKYP10 (Japanese Laid-Open Publication No. 58-110600), pKYP200 [Agric. Biol. Chem., 48, 669(1984)], pLSA1 [Agric. Biol. Chem., 53, 277(1989)], pGEL1 [Proc. Natl. Acad. Sci. USA, 82, 4306(1985)], pBluescript II SK+ (Stratagene), pBluescript II SK(-) (Stratagene), pTrs30 (FERM BP-5407), pTrs32 (PERM BP-5408), pGHA2 (FERM BP-400), pGKA2 (FERM B-6798), pTerm2 (Japanese Laid-Open Publication No. 3-22979, US4686191, US4939094, US5160735), pEG400 [J. Bacteriol., 172, 2392(1990)], pGEX (Pharmacia), pETsystem (Novagen), pSupex, pUB110, pTP5, pC194, pTrxFus (Invitrogen), pMAL-c2 (New England Biolabs), pUC19 [Gene, 33, 103(1985)], pSTV28 (Takara), pUC118 (Takara), pPA1(Japanese Laid-Open Publication No. 63-233798), and the like. In the present specification, a procaryote cell is mainly used for gene manipulation.

Examples of "recombinant vector" for animal cells include pcDNAI / Amp, pcDNAI, pCDM8 (all commercially available from Funakoshi), pAGE107 [Japanese Laid-Open Publication No. 3-229 (Invitrogen), pAGE103 [J. Biochem., 101, 1307(1987)], pAMo, pAMoA [J. Biol. Chem., 268, 22782-22787(1993)], retroviral expression vectors based on murine stem cell viruses (MSCV), and the like.

As used in the present invention, the term "retrovirus vector" refers to, for example, without limitation, retroviral expression vectors based on Moloney Murine Leukemia Virus (MMLV) or Murine Stem Cell Virus (MSCV), and the like.

As used in the present specificion, the term "transformant" refers to the whole or a part of an organism, such as a cell, which is produced by transformation. Examples of a transformant include prokaryotic cell, yeast, animal cell, plant cell, insect cell, and the like. Transformants may be referred to as transformed cells, transformed tissue, transformed hosts, or the like, depending on the subject. A cell used in the present invention may be a transformant.

When a prokaryotic cell is used in the present specification for genetic manipulations or the like, the prokaryotic cell may be of, for example, genus Escherichia, genus Serratia, genus Bacillus, genus Brevibacterium, genus Corynebacterium, genus Microbacterium, genus Pseudomonas, or the like, including the prokaryotic cell, for example, Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No.49, Escherichia coli W3110, Escherichia coli NY49, Escherichia coli BL21(DE3), Escherichia coli BL21(DE3)pLysS, Escherichia coli HMS174(DE3), Escherichia coli HMS174(DE3)pLysS, Serratia ficaria, Serratia fonticola, Serratia liquefaciens, Serratia marcescens, Bacillus subtilis, Bacillus amyloliquefaciens, Brevibacterium ammmoniagenes, Brevibacterium immariophilum ATCC 14068, Brevibacterium saccharolyticum ATCC 14066, Corynebacterium glutamicum ATCC13032, Corynebacterium glutamicum ATCC14067, Corynebacterium glutamicum ATCC13869, Corynebacterium acetoacidophilum ATCC13870, Microbacterium ammoniaphilum ATCC 15354, Pseudomonas sp. D-0110, and the like.

Examples of animal cell as used in the present specificaion include mouse myeloma cell, rat myeloma cell, mouse hybridoma cell, Chinese hamster overy (CHO) cell, BHK cell, African green monkey kidney cell, human leukemic cell, HBT5637 (Japanese Laid-Open Publication No. 63-299), human colon cancer cell line, and the like. The mouse myeloma cell includes ps20, NSO, and the like, the rat myeloma cell includes YB2/0 and the like, the human embryo kidney cell includes HEK293 (ATCC: CRL-1573) and the like, the human leukemic cell includes BALL-1 and the like, the African green monkey kidney cell includes COS-1, COS-7, and the like, and the human colon cancer cell line includes HCT-15, and the like.

A retrovirus infection method as used in the present specification is well known in the art as described in, for example, above mentioned Current Protocols in Molecular Biology (particularly, Units 9.9-9.14), and the like, and specifically, for example, ES cells are trypsinized into a single-cell suspension, followed by co-culture with the culture supernatant of virus-producing cells (packaging cell lines) for 1-2 hours, thereby obtaining a sufficient amount of infected cells.

Gene expression (e.g., mRNA expression, polypeptide expression) may be "detected" or "quantified" by an appropriate method, including mRNA measurement and immunological measurement method. Examples of the molecular biological measurement method include Northern blotting method, dot blotting method, PCR method, and the like. Examples of the immunological measurement method include ELISA method, RIA method, fluorescent antibody method, Western blotting method, immunohistological staining method, and the like, where a microtiter plate may be used. Examples of a quantification method include ELISA method, RIA method, and the like. It can be performed by the gene analysis method using an array (for example, a DNA array, a protein array). The DNA array is widely outlined (Saiboukougaku Bessatsu edited by Shujunsha "DNA microarray and latest PCR method"). The protein array is explained in detail (Nat Genet. 2002 Dec; 32 Suppl: 526-32. An analysis method of gene expression, in addition to the above mentioned methods, RT-PCR, RACE method, SSCP method, immunoprecipitation method, two-hybrid system, in vitro translation, or the like is included, but is not limited to. Such a further analytical method is described in the genome analysis laboratory procedure of Yusuke Nakamura lab manual edited by Yusuke Nakamura Yodo-sha (2002), or the like., for example, and all of those descriptions are used as reference in the present specification.

As used in the present specification, the term "expression" of gene, polynucleotide, polypeptide, or the like, indicates that the gene or the like is affected by a predetermined action in vivo to be changed into another form. Preferably, genes, polynucleotides, or the like are transcribed and translated into polypeptides, and also in one form of the expression, genes may be transcribed into mRNA. More preferably, these polypeptides may have post-translational processing modifications.

As used in the present specification, the term "expression level" refers to the amount of a polypeptide or mRNA expressed in a subject cell. The expression level includes the expression level at the protein level of a polypeptide in the present invention evaluated by any appropriate method using an antibody of the present invention, including immunological measurement methods (e.g., ELISA method, RIA method, fluorescent antibody method, Western blotting method, immunohistological staining method, and the like, or the expression level at the mRNA level of a polypeptide in the present invention evaluated by any appropriate method, including molecular biological measurement methods (e.g., Northern blotting method, dot blotting method, PCR method, and the like). The term "change in expression level" indicates that an increase or decrease in the expression level at the protein or mRNA level of a polypeptide of the present invention evaluated by an appropriate method including the above described immunological measurement method or molecular biological measurement method.

Therefore, as used in the present specification, the term "reduction" of "expression" of gene, polynucleotide, polypeptide, or the like indicates that the level of expression is significantly reduced in the presence of the action of the agent of the present invention as compared to when the action of the agent is absent. Preferably, the reduction of expression includes a reduction in the amount of expression of a polypeptide. As used in the present specification, the term "increase" of "expression" of gene, polynucleotide, polypeptide, or the like indicates that the level of expression is significantly increased in the presence of the action of the agent of the present invention as compared to when the action of the agent is absent. Preferably, the increase of expression includes an increase in the amount of expression of a polypeptide. As used in the present specification, the term "induction" of "expression" of a gene indicates that the amount of expression of the gene is increased by applying a given agent to a given cell. Therefore, the induction of expression includes allowing a gene to be expressed when expression of the gene is not observed, and increasing the amount of expression of the gene when expression of the gene is observed.

As used in the present specification, the term "specifically expressed" in relation to a gene indicates that the gene is expressed in a specific site or for a specific period of time at a level different from (preferably higher than) that in other sites or periods of time. The term "specifically expressed" indicates that a gene may be expressed only in a certain site (specific site) or may be expressed in other sites. Preferably, the term "specifically expressed" indicates that a gene is expressed only in a given site. The gene introduced into a organism or a cell in the present invention may be modified in order to express specifically.

As used in the present specificaion, the term "biological activity" refers to activity possessed by an agent (e.g., a polynucleotide, a protein, or the like.) within an organism, including activities exhibiting various functions. For example, when a certain agent is an enzyme, the biological activity thereof includes its enzyme activity. When a certain agent is a reprogramming agent, the biological activity thereof includes its reprogramming activity. For example, when the collagen interacts to its ligand, the biological activity includes the formation of the aggregate or the other biological changes. In another preferable embodiment, such biological activity may be the gene rearrangement activity or the like. The gene rearrangement activity may be judged by any methods to confirm the movement of the sequence encoding the target gene. For example, when a certain factor is an enzyme, the biological activity includes the enzyme activity. In another example, when a certain factor is a ligand, the association to the receptor corresponding to the ligand is included. Such biological activity can be measured by the known art in the field (see Molecula rCloning, Current Protocols (quated in the present specification), or the like.).

### (biochemistry and molecular biology)

As used in the present specification, the term "gene" refers to an element defining a genetic trait. A gene is typically arranged in a given sequence on a chromosome. A gene which defines the primary structure of a protein is called a structural gene and a gene which regulates the expression of a structural gene is called a regulatory gene (e.g., promoter). Genes herein include structural genes and regulatory genes unless otherwise specified. Therefore, for example, in case of a recombinase gene, both the structural gene of the recombinase and the promotor of recombinase are included, and also only the structural gene or its variant is included as long as the purpose of the present invention can be realized. In the present specification, the control region, the coding region, the exon, and the intron are usually included in the gene.

As used in the present specification, "gene" may refer to "polynucleotide", "oligonucleotide", "nucleic acid", and/or "protein", "polypeptide", "oligopeptide", and "peptide". As used in the present specifcation, "gene product" includes "polynucleotide", "oligonucleotide", "nucleic acid", and/or "protein", "polypeptide", "oligopeptide", and "peptide", which are expressed by a gene. Those skilled in the art understand what a gene product is, according to the context. Therefore, in the present specification, a gene includes not only the double strand DNA but each single strand DNA which composes the double strand such as the sense strand and the antisense strand, and the length is not usually limited at all. Therefore, in the gene of the present invention unless reference is made, the double stranded DNA containing the human genome DNA, the single strand DNA (sense strand) containing cDNA, the single strand DNA (antisense strand) having the complementary sequence to the sense strand, and any fragments thereof is included.

As used in the present specification, the term "homology" in relation to a gene (e.g., nucleic acid sequence, amino acid sequence, or the like.) refers to the proportion of identity between two or more gene sequences. Therefore, the greater the homology between two given genes, the greater the identity or similarity between their sequences. Whether or not two genes have homology is determined by comparing their sequences directly or by a hybridization method under stringent conditions. When two gene sequences are directly compared with each other, these genes have homology if the DNA sequences of the genes have representatively at least 50% identity, preferably at least 70% identity, more preferably at least 80%, 90%, 95%, 96%, 97%, 98%, or 99% identity with each other. As used in the present specification, the term "similarity" in relation to a gene (e.g., nucleic acid sequence, amino acid sequence, or the like) refers to the proportion of identity between two or more sequences when conservative substitution is regarded as positive (identical) in the above-described homology. Therefore, the homology and similarity differ from each other in the presence of conservative substitutions. If no conservative substitution is present, the homology and similarity have the same value.

The similarity, identity and homology of amino acid sequences and base sequences are in the present specification compared by using BLAST (sequence analyzing tool) with the default parameters. The search for the identity can be performed by using BLAST 2. 2. 9 (2004. 5.12 issue) in NCBI. The value of the identity in the present specification is usually provided as the value aligned by the default condition using the above mentioned BLAST. However, when a higher value is provided by the change of a parameter, the highest value is the value of the identity. When the identity is evaluated in a plurality of domains, the highest value of them is the value of the identity.

As used in the present specification, the terms "protein", "polypeptide", "oligopeptide" and "peptide" are used as the same meaning and refer to an amino acid polymer having any length. This polymer may be a straight, branched or cyclic chain. An amino acid may be a naturally-occurring or nonnaturally-occurring amino acid, or a variant amino acid. The term may include those assembled into a composite of a plurality of polypeptide chains. The term also includes a naturally-occurring or artificially modified amino acid polymer. Such modification includes, for example, disulfide bond formation, glycosylation, lipidation, acetylation, phosphorylation, or any other manipulation or modification (e.g., conjugation with a labeling moiety). This definition encompasses a polypeptide containing one or more than two amino acid analog (e.g., nonnaturally-occurring amino acid, or the like.), a peptide-like compound (e.g., peptoid), and other variants known in the art, for example. Although the gene product of the protein usually takes a polypeptide form, it can be the variant of the polypeptide as long as it has the same function. The polypeptide having a specific amino acid sequence includes the fragment, the homologue, the inductor, and the variant.

As used in the present specification, the terms "polynucleotide", "oligonucleotide", "nucleic acid molecule", and "nucleic acid" are used as the same meaning and refer to a nucleotide polymer having any length. This term also includes "oligonucleotide derivative" or "polynucleotide derivative". The "oligonucleotide derivative" or the "polynucleotide derivative" includes nucleotide derivative, or refers to oligonucleotide or polynucleotide having different linkages between nucleotides from typical linkages, which are interchangeably used. Examples of such an oligonucleotide specifically include 2'-O-methyl-ribonucleotide, the oligonucleotide derivative in which phosphodiester bond in the oligonucleotide is converted to phosphorothioate bond, the oligonucleotide derivative in which phosphodiester bond in the oligonucleotide is converted to N3'-P5' phosphoroamidate bond, the oligonucleotide derivative in which ribose and phosphodiester bond in oligonucleotide are converted to peptide-nucleic acid bond, the oligonucleotide derivative in which uracil in the oligonucleotide is substituted with C-5 propynyl uracil, the oligonucleotide derivative in which uracil in the oligonucleotide is substituted with C-5 thiazole uracil, the oligonucleotide derivative in which cytosine in the oligonucleotide is substituted with C-5 propynyl cytosine, the oligonucleotide derivative in which cytosine in the oligonucleotide is substituted with phenoxazine-modified cytosine, the oligonucleotide derivative in which ribose in DNA is substituted with 2'-O-propyl ribose, and the oligonucleotide derivative in which ribose in the oligonucleotide is substituted with 2'-methoxyethoxy ribose. Unless otherwise indicated, a particular nucleic acid sequence also implicitly encompasses conservatively-modified variants thereof (e.g. degenerate codon substitutions) and complementary sequences as well as the sequence explicitly indicated. Specifically, degenerate codon substitutions may be produced by generating sequences in which the third position of one or more selected (or all) codons is substituted with mixed-base and/or deoxyinosine residues (Batzer et al., Nucleic Acid Res. 19: 5081 (1991); Ohtsuka et al., J. Biol. Chem. 260: 2605-2608 (1985); Rossolini et al., Mol. Cell. Probes 8:91-98(1994)). The gene encoding protein or the like usually takes this polynucleotide form.

As used in the present specification, the term "nucleotide" refers to a nucleotide, which the sugar portion is phosphate ester including DNA, RNA, or the like, and may be either naturally-occurring or nonnaturally-occurring. Here, the nucleotide is the compound having the glycosyl linkage between the base and sugar. The term "nucleotide derivative" or "nucleotide analog" refers to a nucleotide, which is different from naturally occurring nucleotides, and has a function similar to that of the original nucleotide. Such nucleotide derivatives and nucleotide analogs are commonly known in the art. Examples of such nucleotide derivatives and nucleotide analogs include, but are not limited to, phosphorothioate, phosphoramidate, methylphosphonate, chiral-methylphosphonate, 2-O-methyl ribonucleotide, and peptide-nucleic acid (PNA). DNA includes cDNA, genome DNA, and synthesized DNA.

As used in the present specification, the term "variant" refers to a substance, such as a polypeptide, polynucleotide, or the like, which differs partially from the original substance. Examples of such a variant include a substitution variant, an addition variant, a deletion variant, a truncated variant, an allelic variant, and the like. The allele refers to a genetic variant located at a locus identical to a corresponding gene, where the two genes are distinguished from each other. Therefore, the term "allelic variant" refers to a variant which has an allelic relationship with a given gene. The term species "homolog" refers to one that has an amino acid or nucleotide homology with a given gene in a given species (preferably at least 60% homology, more preferably at least 80%, at least 85%, at least 90%, and at least 95% homology). A method for obtaining such a species homolog is clearly understood from the description of the present specification. Thus, the cell used in the present invention may contain the modified nucleic acid or polypeptide.

In one embodiment, the variant means the allelic variant which exists naturally, the variant which does not exist naturally, and the variant which has deletion, substitution, addition, and/or insertion; the polynucleotide sequence which does not change substantially the function of the coded polypeptide.

In one embodiment, although the changes (mutation or the like.) of these amino acid sequences can be naturally produced by the mutation, the modification after translation, or the like, it can be also artificially produced by using the gene of natural origin (for example, the gene in the present invention).

In one embodiment, the above mentioned polypeptide contains the allelic variant, the homologue, and the natural variant having the homology at least 70%, preferably 80%, more preferably 95%, and further more preferably 97%.

As used in the present specification, the term "corresponding" amino acid or nucleic acid refers to an amino acid or nucleotide in a given polypeptide or polynucleotide molecule, which has, or is anticipated to have, a function similar to that of a predetermined amino acid or nucleotide in a polypeptide or polynucleotide as a reference for comparison, and particularly, in the case of enzyme molecules, the term refers to an amino acid which is present at a similar position in an active site and similarly contributes to catalytic activity. For example, in the case of transposon sequence, it can be a similar portion in an ortholog corresponding to a particular portion of the transposon sequence.

As used in the present specification, the term "corresponding" gene (e.g., a nuleotide molecule, polypeptide, or the like) refers to a gene in a given species, which has, or is anticipated to have, a function similar to that of a predetermined gene in a species as a reference for comparison and when there are a plurality of genes having such a function, the term refers to a gene having the same evolutionary origin. Therefore, a gene corresponding to a gene may be an ortholog of the gene or a homolog. Therefore, genes corresponding to mouse reombinase or the like genes can be found in other animals. Such a corresponding gene can be identified by techniques well known in the art. Therefore, for example, a corresponding gene in an animal can be found by searching a sequence database of the animal (e.g., human, rat, dog, cat) using the sequence of a reference gene (e.g., mouse recombinase gene, or the like.) as a query sequence. Such a corresponding gene can be easily obtained by using the genome database for the skilled person in the art. The method for obtaining such a genome sequence is common knowledge in the field, and is described in the present specification at other section. In the present invention, the sequence obtained by such a search is also available.

As used in the present specification, the term "fragment" with respect to a polypeptide or polynucleotide refers to a polypeptide or polynucleotide having a sequence length ranging from 1 to n-1 with respect to the full length of the reference polypeptide or polynucleotide (of length n). The length of the fragment can be appropriately changed depending on the purpose, for example, in the case of polypeptides, the lower limit of the length of the fragment includes 3, 4, 5, 6, 7, 8, 9, 10, 15, 20, 25, 30, 40, 50 or more nucleotides, and lengths represented by integers which are not herein specified (e.g., 11 and the like) may be appropriate as a lower limit. For example, in the case of polynucleotides, the lower limit of the length of the fragment includes 5, 6, 7, 8, 9, 10, 15, 20, 25, 30, 40, 50, 75, 100 or more nucleotides, and lengths represented by integers which are not herein specified (e.g., 11 and the like) may be appropriate as a lower limit. As used in the present specification, the length of polypeptides or polynucleotides can be represented by the number of amino acids or nucleic acids, respectively, however, the above-described numbers are not absolute and the above-described numbers as the upper or lower limit are intended to include some greater or smaller numbers (e.g., ±10%), as long as the same function (e.g. the function of mouse recombinase, or the like) is maintained. For this purpose, in the present specification, "about" may be put ahead of the numbers. However, it should be understood that the interpretation of numbers is not affected by the presence or absence of "about" in the present specification.

The term "biological molecule" used in the present specification means the molecule related to organism.

In the present specification, the "organism" refers to a biological organism, and animal, plant, fungi, virus, or the like is included, but is not limited to. Therefore, in the present specification, although a biological molecule includes the molecule extracted from an organism, it is not limited to, but when the molecule can influence the organism, the definition of the biological molecule encompasses the molecule. Therefore, the molecule compounded by combinatorial chemistry and the low molecular which may be used as medical supplies (for example, the low molecular ligand or the like) is encompassed the definition of the biological molecule as long as the effect is may be intended to a organism. In such a biological molecule, protein, polypeptide, oligopeptide, peptide, polynucleotide, oligonucleotide, nucleotide, nucleic acid (for example, cDNA, DNA like genomic DNA, and RNA like mRNA are included), polysaccharide, oligosaccharide, lipid, low molecular (for example, hormone, ligand, intracelluar messenger, organic low molecular, or the like), these complex molecules (glycolipid, glycoprotein, lipoprotein, or the like) is included, but is not limited to. As long as the introduction to a cell is planned, a cell itself and a part of the tissue may be also included by the biological molecule. Normally, the biological molecules may be nucleic acid, protein, lipid, sugar, proteolipid, lipoprotein, glycoprotein, proteoglycan, or the like. Preferably, a biological molecule includes nucleic acid (DNA or RNA) or protein. In another preferable embodiment, a biological molecule is nucleic acid (for example, genomic DNA, cDNA, or synthesized DNA by PCR or the like). In other preferable embodiments, a biological molecule may be protein. Preferably, such a biological molecule may be hormone or cytokine.

As used in the present specification, the term "search" indicates that a certain nucleic acid sequence is utilized to find other nucleic acid base sequences having a specific function and/or property either electronically or biologically, or using other methods. Examples of an electronic search include, but are not limited to, BLAST (Altschul et al., J. Mol. Biol. 215:403-410 (1990)), FASTA (Pearson & Lipman, Proc. Natl. Acad. Sci., USA 85:2444-2448 (1988)), Smith and Waterman method (Smith and Waterman, J. Mol. Biol. 147:195-197 (1981)), and Needleman and Wunsch method (Needleman and Wunsch, J. Mol. Biol. 48:443-453 (1970)), and the like. Examples of a biological search include, but are not limited to, stringent hybridization, a macroarray in which genomic DNA is attached to a nylon membrane or the like or a microarray (microassay) in which genomic DNA is attached to a glass plate, PCR and in situ hybridization, and the like. In the present invention, the recombinase sequence or the like identified by such a search may be used.

In the present specification, "highly stringent condition" makes possible the hybridization with the DNA strand which has highly complementations in the nucleic acid sequence, and is the conditions designed except the hybridization of DNA which has a mismatch significantly. The stringency of hybridization is mainly determined by the conditions of temperature, ionic strength, and denaturing agent such as formamide. Examples of "highly stringent condition" about such hybridization and washing is 0.0015M sodium chloride, 0.0015M sodium citrate, 65-68 °C or 0.015M sodium chloride, 0.0015M sodium citrate and 50% formamide, 42 °C. Such "highly stringent condition" is referred in Sambrook et al., Molecular Cloning: A Laboratory Manual the 2nd edition, Cold Spring Harbor Laboratory (Cold Spring Harbor, N, Y. 1989); and Anderson et al., Nucleic Acid Hybridization: a Practical approach, IV, IRL Press Limited (Oxford, Engl and), Limited, Oxford, England. If needed, more stringent condition (for example, a higher temperature, a lower ionic strength, a higher formamide, or other denaturing agents) may be used. Other drug may be included in hybridization buffer solution and washing buffer solution in order to decrease the nonspecific hybridization and/or the background of the hybridization. Examples of such other drugs are 0.1 % bovine serum albumin, 0.1 % polyvinylpyrrolidone, 0.1 % sodium pyrophosphate, 0.1% sodium dodecyl sulfate (NaDodSO or SDS), Ficoll, Denhardt solution, sonicated salmon sperm DNA (or another non-complementary DNA), and dextran sulfate and also other suitable drugs may be used. The concentration and the type of these additives may be changed without giving the stringency of hybridization conditions substantially. Although the hybridization experiment is usually conducted by pH 6.8-7.4; on typical ionic strength conditions, most speed of the hybridization is pH independence. Refer to Anderson et al., Nucleic Acid Hybridization: a Practical Approach, Chapter 4, IRL Press Limited (Oxford, England).

The factor which influences the stability of DNA double strand is the degree of the base composition, the length, and the base pairing discrepancy. The hybridization conditions can be adjusted by the person skilled in the art, and applying these variables and allowing the DNA relating to the different sequence to form a hybrid. The melting temperature of the DNA double strand which is matched completely can be estimated by the following formulas. Tm (°C) = 81.5 + 16.6 (log[Na⁺]) + 0.41(%G+C) - 600/N-0.72 (% formamid). Here, N is the length of formed double strand length, [Na⁺] is the molarity of the sodium ion in the hybridization solution or the washing solution, and %G + C is the percentage of the base (guanine + cytosine) in the hybrid. The melting temperature decreases about 1 °C corresponding to each 1 % discrepancy (mismatch) regarding the hybrid which is matched imperfectly.

As used in the present specification, the "middle stringent condition" refers to the condition that can be formed the DNA double strand having the higher degree of the base pairing discrepancy comparing to the "high stringent condition". Examples of typical "middle stringent condition" are 0.015M sodium chloride and 0.0015M sodium citrate at 50-65 °C or 0.015M sodium chloride, 0.0015M sodium citrate, and 20% formamide at 37-50 °C. As an example, 50 °C the condition of "middle stringent condition" allow about 21 % of discrepancy in 0.015M sodium ion.

In the present specification, the complete differences between "high" stringent condition and "middle" stringent condition it can not exist, which is understood by the person skilled in the art. For example, in 0.015M sodium ion (without formamide), the melting temperature of long DNA which matched completely is about 71 °C. In washing at 65 °C (the same ionic strength), the discrepancy of about 6% is allowed. In order to capture the related sequence which further separated, a person skilled in the art can only reduce the temperature or can increase the ionic strength.

About the oligonucleotide probe up to about 20 nucleotides, the suitable estimate of the melting temperature in 1M NaCl is provided by Tm = (2 °C per A-T base)+ (4 °C per G-C pair). The sodium ion concentration in 6x sodium citrate salt (SSC) is 1M (refer to Suggs et al., Developmental Biology Using Purified Genes, 683 pages, Brown, and Fox (edit) (1981)).

The recombinase, the recombinase recognition sequence, or the variant or the natural nucleic acid encoding the protein such as the fragment and the promotor sequence used in the present invention are separated easily from the cDNA library which has the PCR primer and the hybridization probe including all or a part of the nucleic acid sequences (for example, the sequence number 1 or the like) or the variant used and illustrated in an embodiment for example. The recombinase, the recombinase recognition sequence, or the variant or the natural nucleic acid encoding the protein such as the fragment, preferably under the low stringent condition defined with the hybridization buffer solution substantially containing 7% SDS and 1% bovine serum albumin (BSA); 500mM sodium phosphate (NaPO₄); 1mM EDTA in the temperature at 42 °C, and the washing buffer solution substantially containing 0.1 % SDS and 2 x SSC (600mM NaCl; 60mM sodium citrate) in the temperature at 50 °C, the more preferably under the low stringent condition defined with the hybridization buffer solution substantially containing 7% SDS and 1% bovine serum albumin (BSA); 500mM sodium phosphate (NaPO₄); 15% formamide; 1mM EDTA in the temperature at 50 °C, and the washing buffer solution substantially containing 1% SDS 1 x SSC (300mM NaCl; 30mM sodium citrate) in the temperature at 50 °C, the most preferably under the low stringent condition defined with the hybridization buffer solution substantially containing 7% SDS and 1% bovine serum albumin (BSA); 200mM sodium phosphate (NaPO₄); 15% formamide; 1mM EDTA in the temperature of 50 °C, and the washing buffer solution substantially containing 0.1% SDS 0.5 x SSC (150mM NaCl; 15mM sodium citrate) in the temperature at 65 °C, for example, can be hybridized with one or the part of nucleic acid sequence shown in the sequence number 1 or the like as a target.

As used in the present specification, the term "probe" refers to a substance for use in searching, which is used in a biological experiment, such as in vitro and/or in vivo screening or the like, including, but not being limited to, for example, a nucleic acid molecule having a specific base sequence or a peptide containing a specific amino acid sequence.

Examples of a nucleic acid molecule as a common probe include one having a nucleic acid sequence having a length of at least 8 contiguous nucleotides, which is homologous or complementary to the nucleic acid sequence of a gene of interest. Such a nucleic acid sequence may be preferably a nucleic acid sequence having a length of at least 9 contiguous nucleotides, more preferably a length of at least 10 contiguous nucleotides, and even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 20 contiguous nucleotides, a length of at least 25 contiguous nucleotides, a length of at least 30 contiguous nucleotides, a length of at least 40 contiguous nucleotides, or a length of at least 50 contiguous nucleotides. A nucleic acid sequence used as a probe includes a nucleic acid sequence having at least 70% homology to the above-described sequence, more preferably at least 80%, and even more preferably at least 90% or at least 95%. By using such a probe, the transposon which may be used in the present invention can be obtained.

As used in the present specification, the term "primers" refers to a substance required for initiation of a reaction of a macromolecule compound to be synthesized, in a macromolecule synthesis enzymatic reaction. In a reaction for synthesizing a nucleic acid molecule, a nucleic acid molecule (e.g., DNA, RNA, or the like) which is complementary to part of a macromolecule compound to be synthesized may be used.

A nucleic acid molecule which is ordinarily used as a primer includes one that has a nucleic acid sequence having a length of at least 8 contiguous nucleotides, which is complementary to the nucleic acid sequence of a gene of interest. Such a nucleic acid sequence preferably has a length of at least 9 contiguous nucleotides, more preferably a length of at least 10 contiguous nucleotides, even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 16 contiguous nucleotides, a length of at least 17 contiguous nucleotides, a length of at least 18 contiguous nucleotides, a length of at least 19 contiguous nucleotides, a length of at least 20 contiguous nucleotides, a length of at least 25 contiguous nucleotides, a length of at least 30 contiguous nucleotides, a length of at least 40 contiguous nucleotides, and a length of at least 50 contiguous nucleotides. A nucleic acid sequence used as a primer includes a nucleic acid sequence having at least 70% homology to the above-described sequence, more preferably at least 80%, even more preferably at least 90%, and most preferably at least 95%. An appropriate sequence as a primer may vary depending on the property of the sequence to be synthesized (amplified). Those skilled in the art can design an appropriate primer depending on the sequence of interest. Such primer design is well known in the art and may be performed manually or using a computer program (e.g., LASERGENE, Primer Select, DNAStar). By using such a primer, the transposon which may be used in the present invention can be produced.

As used in the present specification, the term "agent capable of binding specifically to" a certain nucleic acid molecule or polypeptide refers to an agent which has a level of binding to the nucleic acid molecule or polypeptide equal to or higher than a level of binding to other nucleic acid molecules or polypeptides. Examples of such an agent include, but are not limited to, when a target is a nucleic acid molecule, a nucleic acid molecule having a complementary sequence of a nucleic acid molecule of interest, a polypeptide capable of binding to a nucleic acid sequence of interest (e.g., a transcription agent; or the like), and the like, and when a target is a polypeptide, an antibody, a single chain antibody, either of a pair of a receptor and a ligand, either of a pair of an enzyme and a substrate, and the like. In the present specification, such factors (for example, the factor specifically associated with calcium and the antibody against a specific gene product, or the like) associated specifically can be used when measuring the signal transduction.

As used in the present specification, the term "agent" refers to any substances or other elements (for example, the energy such as light, radioactivity, heat, electricity, or the like can be used) as long as the intended purpose can be realized. Examples of such substances is protein polypeptide, oligopeptide, peptide, polynucleotide, oligonucleotide, nucleotide, nucleic acid (for example, DNA such as cDNA or genomic DNA and RNA such as mRNA are included), polysaccharide, oligo saccharides, lipid, organic low molecular (for example, hormone, ligand, signal transduction substance, organic low molecule, the molecule synthesized by combinatorial chemistry, and low molecular which can be used as medical supplies (for example, low molecular ligand or the like), the complex molecules thereof is included, but is not limited to. As the specific factor to polynucleotide, typically a polynucleotide which is complementary with constant homology sequence (for example, more than 70% of sequence identity) to the polynucleotide sequence, a polypeptide such a transcription factor which is associated on the promoter region, or the like is included, but is not limited to. As the specific factor to a polypeptide, typically the antibody or the inductor specifically designed to the polypeptide or the analogue (for example, a single chain antibody), the specific ligand or receptor when the polypeptide are a receptor or a ligand, or the substrate when the polypeptide are enzyme is included, but is not limited to.

As used in the present specification, the term "contact (contacted)" refers that a compound being either directly or indirectly comes close physically to the polypeptide or the polynucleotide in the present invention. The polypeptide or the polynucleotide may present in many buffer solution, salt, solution, or the like. As the contact, the compound including the polypeptide which encodes nucleic acid molecules or the fragment set, for example, on beaker, microtiter plate, cell culture flask, or microarray (for example, gene chip) is included.

In a certain protein molecule, a certain amino acid contained in a sequence may be substituted with another amino acid in a protein structure, such as a cationic region or a substrate molecule binding site, without a clear reduction or loss of interactive binding ability. A certain biological function of a protein is defined by the interactive ability or other property of the protein. Therefore, a particular amino acid substitution may be performed in an amino acid sequence, or at the DNA code sequence level, to produce a protein which maintains the original property after the substitution. Therefore, various modifications of peptides as disclosed in the present specification and DNA encoding such peptides may be performed without clear losses of biological usefulness.

When the above described modifications are designed, the hydrophobicity indices of amino acids may be taken into consideration. The hydrophobic amino acid indices play an important role in providing a protein with an interactive biological function, which is generally recognized in the art (Kyte, J. and Doolittle, R. F., J. Mol. Biol. 157(1): 105-132, 1982). The hydrophobic property of an amino acid contributes to the secondary structure of a protein and then regulates interactions between the protein and other molecules (e.g., enzymes, substrates, receptors, DNA, antibodies, antigens, or the like). Each amino acid is given a hydrophobicity index based on the hydrophobicity and charge properties thereof as follows: isoleucine (+4.5); valine (+4.2); leucine (+3.8); phenylalanine (+2.8); cysteine / cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamic acid (-3.5); glutamine (-3.5); aspartic acid (-3.5); asparagine (-3.5); lysine (-3.9); and arginine (-4.5).

It is well known that if a given amino acid is substituted with another amino acid having a similar hydrophobicity index, the resultant protein may still have a biological function similar to that of the original protein (e.g., a protein having an equivalent enzymatic activity). For such an amino acid substitution, the hydrophobicity index is preferably within ±2, more preferably within ±1, and even more preferably within about ±0.5. It is understood in the art that such an amino acid substitution based on hydrophobicity is efficient.

A hydrophilicity index is also useful for modification of an amino acid sequence of the present invention. As described in U.S. Pat. No. 4,554,101, amino acid residues are given the following hydrophilicity indices: arginine (+3.0); lysine (+3.0); aspartic acid (+3.0±1); glutamic acid (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4). It is understood that an amino acid may be substituted with another amino acid which has a similar hydrophilicity index and can still provide a biological equivalent. For such an amino acid substitution, the hydrophilicity index is preferably within ±2, more preferably ±1, and even more preferably ±0.5.

### (Kit)

As used in the present specification, the term "kit" refers to the unit provided with the parts which should be provided (for example, a reagent, particles, or the like) by normally separating into more than two sections. In the present invention, the gene cassette or the like is provided as a product with a kit form, or the cell or the like is provided as a library, for example. When the cell is provided as a kit, it can be provided with the preserved state by a preserving method such as freezing or the like. The embodiment of the kit is preferable when it is intended to provide a composition which should not be provided after being mixed and which is preferably mixed for using just before use. Preferably, such a kit is favorable to provide the manual which describes how the provided sections (for example, a reagent, particles, or the like) should be treated. Such a manual can be provided through any medium, for example, the medium such as a paper medium, a transmission medium, a recording medium, or the like is included, but is not limited to. As a transmission medium, the Internet, intranet, extranet, LAN, or the like is included, but is not limited to, for example. As a recording medium, CD-ROM, CD-R, flexible disk, DVD-ROM, MD, mini disc, MO, memory stick, or the like is included, but is not limited to.

### (Transgenic organism)

The general art for producing a transgenic mouse is described in the international publication WO01/13150 (LudwigInst.CancerRes.), for example. U.S. Pat. No. 4,873,191 (Wagner et al.) is instructing the mammalian having the exogenous DNA obtained by the DNA microinjection to a mammalian zygote. In these arts, the present invention by using a recombinase can be applied.

In addition, the various methods for making a transgenic organism, for example, M. Markkula et al., Rev. Reprod., 1, 97-106 (1996); R. T. Wall et al. Dairy Sci., 80, 2213-2224 (1997); J. C. Dalton et al. Adv. Exp. Med. Biol., 411, 419-428 (1997); and H. Lubon et al., Transfus. Med. Rev., 10, 131-143 (1996), or the like is included, but is not limited to. Each of these references is incorporated as references in the present specification.

Thus, the analysis of the transgenic (including knockout and knockin) animal used the homologous recombination of the embryonic stem (ES) cell for the purpose of the gene function analysis is presently becoming an important means.

In the higher organism, the efficient screening of the recombinant is performed by the positive selection using the neomycin resistance gene and the negative selection by using the thymidine kinase gene of HSV or the diphtheria toxin gene, for example. The object of homologous recombination is selected by the PCR or the Southern blot method. The object of homologous recombination is selected by the PCR or Southern blot method. That is, a part of the target gene is substituted to the neomycin resistance gene or the like for positive selection, the targeting vector ligating the HSVTK gene or the like for negative selection is produced with the end, introduced into the ES cell by electroporation, and selected under the existence of G418 and ganciclovir, and then the produced colony is isolated, and also the object of homologous recombination is selected by PCR or Southern blot.

Thus, the method to produce the transgenic (the target gene recombination) mouse which has the function of losing or having the changed mutation by substituting or destroying the intrinsic target gene is useful to the analysis of the genetic function since the mutation is introduced only into the targeted gene.

After selecting a desired homologous recombinant, the chimera mouse with the ES cell and the host embryo is produced by mixing the obtained recombinant ES cell with normal embryo by using the blastodisk injection method or the assembly chimera method. In the blastodisk injection method, the ES cell is injected into the blastocyst with a glass pipette. In the assembly chimera method, the ES cell mass and the 8 cell embryo eliminated the zona pellucida is adhered. The blastocyst introduced the ES cell is transplanted to the uterus of the surrogate mother which is the false pregnancy, and the chimeric mouse is obtained. Since the ES cell has the totipotency and can differentiate to any kinds of cell including the germ cell in vivo. When the chimeric mouse which have a germ cell derived from ES cell and the normal mouse are mated, the mouse which has the hetero-chromosome of the ES cell will be obtained, and when these mice are mated, the transgenic mouse which has a modified homo-chromosome of the ES cell will be obtained. In order to obtain the transgenic mouse which has a modified homo-chromosome from the obtained chimeric mouse, the male chimeric mouse and the female wild type mouse are mated and the F1 generation heterozygote mouse is made to produce, then the male and female produced heterozygote mouse are mated and the F2 generation homozygote mouse is selected. Whether a desired gene mutation is introduced in each F1 and F2 generation can be analyzed by using the common method used in the fields, such as Southern blotting, PCR, and the base sequence decoding as well as the assay of the recombination ES cell.

The transient expression of Cre enzyme, DNA mapping on a chromosome, and the like, which are used in the present specification in a method for removing a genome, a gene locus, or the like, are well known in the art, as described in Kenichi Matsubara and Hiroshi Yoshikawa, editors, Saibo-Kogaku [Cell Engineering], special issue, "Experiment Protocol Series "FISH Experiment Protocol From Human Genome Analysis to Chrmosome / Gene diagnosis", Shujun-sha (Tokyo), and the like.

Therefore, the art which uses together the cell strain specific expression of the Cre recombinase and the site specific recombination of Cre-loxP attracts attention as the next generation art of overcoming the problem that various genetic functions cannot be analyzed selectively. The transgenic mouse by using Cre-loxP is introduced the neomycin resistance gene into the position which does not inhibit the expression of a target gene, the targeting vector which inserted the loxP sequence is introduced into the ES cell in between the exon which will be deleted afterward, and then the homologous recombinant is isolated. The chimeric mouse is obtained from the isolated clone and the genetically modified mouse is produced. Next, when the transgenic mouse which expresses the site specific recombination enzyme Cre defived from the P1 phage of escherichia coli in a tissue specific manner and the mouse mentioned above are made to mate, the gene is destroyed only in the tissue which expresses Cre (here, Cre recognizes a loxP sequence (about 34 bps) specifically and the recombination is caused in the sequence in between two loxP sequences, and it is destroyed). Cre can be expressed in the adult by mating with the transgenic mouse which has the Cre gene connected with the organ specific promotor or using the viral vector which has the Cre gene.

As used in the present specification, the term "recombinase", also calls recombinant enzyme, refers to the enzyme which recognizes the specific sequence on DNA (for example, loxP sequence in case of Cre recombinase) and promotes the recombination of the position. As a recombinase, the Cre recombinase or the like can be included, for example. In addition, as a recombinase which may be used, ΦC31 (ACCESSIONNC_001978; GI: 40807285) can be included, but is not limited to, for example.

As used in the present specification, the term "recombinase recognition sequence" refer to the sequence recognized by at least one recombinase, and such a sequence is usually specific to the recombinase. Such a recognition sequence can be determined as follows: the Southern hybridization analysis, the DNA sequence of PCR product, or the like is included. As such a recognition sequence, loxP sequence, FRT site, attB sequence, attP sequence, and res site sequence can be included, but is not limited to, for example. The concrete sequence of such recognition sequence is as follows:
LoxP sequence
   ATAACTTCGTATAATGTATGCTATACGAAGTTAT (sequence number 1)
FRT site
   GAAGTTCCTATTCTCTAGAAAGTATAGGAACTTC (sequence number 4)
attB sequence 
TCGAGTGAGGTGGAGTACGCGCCCGGGGAGCCCAAGGGCACGCCCTGGCAC
CCGCA (the sequence number 5)
attp sequence 
CTAGACCCTACGCCCCCAACTGAGAGAACTCAAAGGTTACCCCAGTTGGGGC
ACG (the sequence number 6).
Such a recombinase recognition sequence can include all the above mentioned sequences and can include one or a plurality of substitution, addition, and/or deletion to these sequences as long as recognized by the recombinase. Or other recombinase recognition sequence can be produced by screening using the recombinase and the library of any sequences under constant condition.

As used in the present specification, the term "opposite direction" refers that the recombinase recognition sequence is arranged at the chain of another side when used about the recombinase recognition sequence and the recombinase recognition sequence is arranged at one side of double stranded DNA. Preferably, the substantially homology sequence is arranged from 5' to 3' at one chain, and is arranged from 5' to 3' at the chain of another side. More preferably, the identical sequence is arranged from 5' to 3' at one chain, and is arranged from 5' to 3' at the chain of another side.

Conventionally, although it has been considered that the recombinase recognition sequence is required for more than two in the identical directions in order to demonstrate the recombinase activity and to occur the recombination as a result is required for more than two in the identical directions, it was discovered that the deletion of a desired chromosome can be occurred by using a plurality of or only one recombinase recognition sequence in the opposite direction in the present invention. Especially it was unexpected discovery that the cell has the capacity to regenerate even after occurring the deletion of a chromosome.

The gene trap method attracts attention as a method for analyzing the specific gene. In the gene trap method, the reporter gene which does not have a promotor is introduced into a cell, and when the gene is accidentally inserted on a genome, a new gene will be isolated (trapped) by using the expression of the reporter gene. A gene trap method is a method for efficient insertion mutation and strange gene identification, which is based on the early mouse embryo operating method, embryonic stem cells culture method, and the gene targeting method by homologous recombination (Stanford W L., et al., Nature Genetics 2: 756-768 (2001)). In the gene trap method, the introduction or the gene, the selection of the insertion mutation, and the phenotype analysis are comparatively easy.

In the gene trap method, for example, the gene trap vector which connected β-geo, which is a fusion gene of lacZ and neo between the splicing / acceptor sequence and the poly A addition signal is introduced into the ES cell and selected by G418, and only the clone will be selected which incidentally traps the gene expressed in the ES cell.

Thus, when the chimera embryo is produced from the obtained clone, the gene expression pattern which is trapped shows the staining pattern of the various X-gal. Thus, in the gene trap method, a unknown gene is isolated, the genetic expression pattern is analyzed, and the gene is destroyed.

In the present invention, the desired transgenic organism can be prescreened. As the prescreening method, for example, the gene trap method can be used. (Zambrowicz et al. Nature, 392: 608-611 (1998); Gossler et al. Science, 244: 463-465 (1989); Skallnes, W. C. et al. Genes Dev. 6: 903-918 (1992); Friedrich, G. et al. Genes Dev. 5: 1513-1523 (1991)). Thus, by performing prescreening, the transgenic organism promising for the elucidation of the gene function can be selected preliminarily, and the transgenic organism mutated both the genes of one pair of chromosomes can be obtained by mating two or more generations or the other proper means.

According to the present invention, it is possible to eliminate or introduce in a chromosomal level. According to the present invention, the new organism of a present invention or a part of that provides the useful model system for the elucidation of the gene function in a chromosomal level. In the living animal model, the embodiment of the present invention can provide the disease model system for the research of the hereditary disease. In the system, the target disease gene in the animal model is a human disease causative gene or the homologous gene of those in an organism, which a full length gene of cDNA, a gene fragment of cDNA, a full length gene of genomic DNA, or a gene fragment of genomic DNA is included. Preferably the disease causative gene can be provided the research as a human disease model animal, which is the transgenic organism produced by introducing into an organism, but is not limited to, preferably the human disease causative gene.

As used in the present specification, the term "marker gene" refers to any genes encoding the gene product which can be checked by the explicit method such as the visual recognition whether or not transformation has been performed or the like. As such a marker gene, the green fluorescence protein (GFP), the yellow fluorescence protein (YFP), the cyanogen fluorescence protein (CFP), the red fluorescence protein (dsRED), or the like can be included, but is not limited to. The sequence of the green fluorescence protein is included as shown in the sequence number 7, for example.

As used in the present specification, the term "selection sequence" refers to that the sequence which enables screening the cell which is not introduced and is introduced when the nucleic acid including the sequence introduces into a cell, for example, an antibiotic resistance gene or the like can be included. As an example of such a selection sequence, for example, thymidine kinase (TK) gene, puromycin resistance gene, neomycin resistance gene, hygromycin resistance gene, blasticidin resistance gene, zeosin resistance gene, HAT resistance gene, diphtheria toxin resistance gene, and fluorouracil resistance gene, but is not limited to. The gene encoding the puromycin resistance gene can be included as shown the sequence number 9, for example.

As used in the present specification, the term "IRES sequence" or "internal ribosomal entry site sequence" is used exchangably, also called polycistron, and refers to the sequence intervening the CAP structure independent translation mechanism. Such IRES sequence is known in variety and compiled a database. For example, refer to the following:
http://www.rangueil.inserm.fr/IRESdatabase/

As used in the present specification, the term "CAG sequence" refers to the sequence, which is repeated CAG, and is usually observed 10-30 times repeat. Preferably, the encoding sequence has the glutamine repeat. The protein which recognizes mRNA with the CAG repeat sequence is present, the more the protein has many CAG repeat, the more the protein combines with mRNA strongly, and it is considered to regulate the expression of other proteins.

In the present invention, the transgenic organism in the present invention can be used as the donor for organ donation. For example, nerve cell, heart, lung, liver, pancreas, kidney, cornea, skin, or the like is concretely included as an organ considered as a donor of the xenotransplantation to human. In the case, the introduced gene is preferably the gene having a function which reduces the rejection response in the organ transplant between different species, or the gene having a function which can expect to increase the rate of graft survival, for example.

About the production of the transgenic organism is included in U.S. Pat. No. 5,464,764; U.S. Pat. No. 5,487,992; U.S. Pat. No. 5,627,059; Japanese patent Publication No. 2001-54337;Gossler, A. et al. (1989), Science 244, 463-465; Wurst, W et al. (1995), Genetics 139, 889-899; Zambrowicz, B. P. et al. (1998), Nature 392, 608-611 Proc. Natl. Acad. Sci. USA, Vol. 86, 8932-8935, 1989; Nature, Vol.342, 435-438, 1989; Masami Muramatsu, Miyabi Yamamoto edit, under "Jikkenigaku-bessatsu shintei idenshikogaku handbook 3rd edition" (1999, Yodo-sha issue, the 3rd edition) especially 239-256 page; Shinichi Aizawa (1995) experimental medicine separate volume "Gene targetting-ES saibou wo mochiita heni mouse no sakusei" [Production of the mutant mouse by using gene targeting-ES cell] or the like, but is not limited to.

As used in the present specification, when the term "knockout" is mentioned about a gene, it indiccates to make the gene into destruction (defect) or malfunction. Therefore, the concept of knockout is included in the transgenic.

As used in the present specification, the term "knockout organism" refers to the organism (for example, mouse) in which a certain gene was knocked out. Therefore, the concept of the knockout organism is included in the transgenic organism.

The "organism" of the transgenic object in the present specification is treated the transposon and any organisms which can function such a system may function are included. Animal, plant, bacteria, or the like is included in such an organism, but is not limited to. As used in the present specification, the term "animal" may be any animals as long as the object can be the introduction of a nucleic acid sequence (foreign sequence encoding a gene preferably). Therefore, a vertebrate and an invertebrate are included in the animal. As an animal, a mammal (for example, mouse, dog, cat, rat, ape, pig, cow, sheep, rabbit, dolphin, whale, goat, horse, or the like), a bird (for example, chicken, quail, or the like), an amphibia (for example, frog or the like), a reptile, an insect (for example, drosophila or the like), or the like is included. Preferably, the animal may be mammalian and more preferably the animal may be the animal which is produced the knockout easily (for example, mouse). In another preferable example, the animal may be the animal which is suitable to the human model animal (for example, monkey). In a certain embodiment, the animal may be non-human animal or non-human mammal, but is not limited to. For example, pig, monkey, cow, horse, goat, sheep, cat, dog, rabbit, mouse, rat, or hamster, or the like, more preferably, mouse or rat is included. Unless reference is made especially, not only a mammalian individual but a part of individual and a body part or an organ are included in the organism as the present invention herein. These are useful as a human disease model and a donor for organ transplant.

In the above mentioned organism, the introduction art of the gene is microinjection, combinations with the nucleic acid fragment, the cation lipid microsome, or DNA condensation reagent; and the nucleic acid fragments are introduced into a viral vector and the viral vector is contacted to the cell, and the method selected from the group which comprises the particle bombardment and electroporation are included.

The viral vector which may be used in the present specification, which is selected from the group consisting lentivirus vector, retroviral vector, adenovirus vector, herpesvirus, or adenovirus related viral vector is included, but is not limited to.

As used in the present specification, the term "retrovirus" refer to the virus which have a genetic code in the form of RNA and synthesizes DNA from the information of RNA by the reverse transcriptase. Therefore, the "retroviral vector" means the form which uses the retrovirus as a porter (vector) of the gene. As the "retroviral vector" used in a present invention, for example, the retrovirus type expression vector based on Moloney Murine Leukemia Virus (MMLV) and Murine Stem Cell Virus (MSCV) or the like, but is not limited to.

Preferably, as the retroviral vector, pGen-, pMSCV, or the like is included, but is not limited to.

As used in the present specification, the term "gene trap (method)" refers to the identification method of the gene, which utilizes that the reporter activity can be detected only when inserted in the downstream of the promotor activated on the chromosome by the reporter gene lacking the promotor is introduced into the target cell. Such the gene trap is realized by introducing the "gene trap vector" into the host chromosome of the eukaryotic organism and destroying the host gene. Since the gene in which the reporter gene was inserted expresses the complex protein with the reporter, it is possible to identify the gene by monitoring the protein. Therefore, since the reporter gene is incorporated in an original locus as well as the homologous recombination, the reporter system with the perfect transcriptional regulation can be made. By using the approach, the gene which was not obtained by an approach of isolating the mutant by using the gene disruption can be identified. Therefore, such the gene trap procedure can also be used in the present invention.

As used in the present specification, the term "gene trap vector" refers to a vector for selecting the vector inserted into the gene by using the phenomenon of receiving splicing in the process which mRNA in the eukaryotic organism gene turns into mature mRNA. As the gene trap vector, (1) a vector including a DNA sequence including the coding region and splice acceptor site of the reporter gene which does not have a promotor, (2) a vector including a DNA sequence including the coding region and splice donor site of the reporter gene which has a promotor, and (3) a vector including the DNA sequence both (1) and (2) is included, but is not limited to.

The gene trap vector including the above splice acceptor sequences may also include a poly A addition signal if needed. The gene trap vector including a splice donor sequence may also include an enhancer region and/or a mRNA instability region if needed. As the poly A addition signal, "AATAAA" is included, but is not limited to.

As a promotor used in the present invention, MC1 promotor, RNA pol II promotor, or the like is included, but is not limited to.

As an enhancer used in the present invention, polyoma vinus enhancer (PYF441) or the like is included, but is not limited to.

As a splice donor sequence used in the present invention, mouse hprt gene exon 8 splice donor is included, but is not limited to.

As a splice acceptor sequence used in the present invention, human bcl-2 gene exon 3 splice acceptor is included, but is not limited to.

As used in the present specification, the term "reporter" molecule or "reporter" gene refers to the molecule which can be used as an index of the gene expression in a cell (for example, polypeptide) or the gene. As such a molecule, commonly known reporter protein can be used, for example, chloramphenicol acetyltransferase (CAT), β-glucuronidase (GUS), β-D-galactosidase, luciferase, green fluorescence protein (GFP), aequorin, or the like is included. Here, the introducing method of the gene itself can be performed by using a desired material with commonly known art in the field. In such a case, for example, the reporter gene which lacked the promotor (for example, luciferase gene, green fluorescence gene, β-galactosidase gene (lacZ), alkaline phosphatase gene, Cre recombinase gene, or the like) is introduced into the target embryonic stem cells, and the reporter activity is detected only when inserted the downstream of the promotor activated on the chromosome. The vector used besides the reporter gene is selective marker gene, (for example, neomycin resistance gene, hygromycin resistance gene, puromycin resistance gene, rescue marker gene (for example, Ampicillin resistance gene + colicin E1 duplicate beginning point)), or the like may be included. Here, selective marker gene is used in order to select the host containing the vector. A rescue marker gene is used in order to rescue the vector (refer to Joyner, A. L. ed. "Gene Targeting, 2nd edition" (Oxford University Press, 2000)). Embryonic stem cells are produced by using the above mentioned art. The modified embryonic stem cells is trapped the gene. Here, trapped means the state which the internality gene is destroyed by the insertion of the trap vector to a genome and simultaneously is marking the destroyed gene by the vector.

The preparation of an oligonucleotide which has a specific sequence can be performed by using commonly known art in the field, for example, the method described in Joyner, A. L. ed. "Gene Targeting, 2nd edition" (Oxford University Press, 2000). The oligonucleotide can be labeled by fluorescence, radioactivity, or the like. if needed. Such a labeling method is common knowledge in the field and is described in the literature quoted in the present specification.

### (Screening)

As used in the present specification, the term "screening" refers to select a target such as an organism, a substance, or the like having a certain specific property of interest from a population containing a number of elements by using a specific operation / evaluation method. For screening, the method or the organism in the present invention can be used. In the present invention, any nucleic acid molecule and the function regulator can be screened by producing various transgenic organisms.

In the present invention, any nucleic acid molecules can be screened by using the nucleic acid molecule, method, or system of the present invention. The present invention intends to include the chemical agent identified by such screening or the combination thereof.

The system in the present invention is applicable to various fields. For example, identification, isolation and characteristic of growth, maintenance, regulation, or development of a living organism can be determined (for example, Kaiser et al., 1995 "Eukeryotic transposable, elements as tools to study gene structure and function" Mobile Genetic Elements, IRL Press, pp. 69-100); Identification, isolation and characteristic of the transcription regulatory sequence regulated growth, maintenance, regulation, or development of a living organism can be determined (for example, Anderson et al., 1996, Mol. Mar. Biol. Biotech., 5, 105-113).

### (Description of Preferred Embodiments)

Hereinafter, although explanation of a preferred embodiment is described, the embodiment is the illustration of the present invention and it should be understood that the scope of the present invention is not limited to such a preferred embodiment should be understood.

### (gene cassette)

In one aspect, the present invention provide a gene cassette including a recombinase recognition sequence in one or two opposite direction, wherein the gene cassette including the sequence coded a marker gene. It became clear that the gene cassette can be used in the recombination system which is used recombinase. Especially, since elimination of the desired chromosome from a regenerable (that is, the next generation is producible) cell (especially stem cell) by using recombinase was producible, which was not expected, the present invention provided the superior effect unexpectedly.

Unlike the conventional method, in the gene cassette of the present invention, the recombinase recognition sequence is not set two in the same direction, but set two in the opposite direction or only one, it was discovered that elimination of the desired chromosome is producible unexpectedly by using recombinase from a regenerable (that is, the next generation is producible) cell (especially stem cell). In the conventional method, since the cell which is not regenerable is produced or is not effectively to eliminate the desired chromosome, the present invention has a prominent effect. The conventional chromosome elimination is introducing Hprt gene or the like into the chromosome of Hprt(-) cell and making drug selection by 6TG (6-thioguanine), and the method of screening the defect cell by the mutation of Hprt introduced chromosome was taken. In this method, the elimination of chromosome is observed only less than 10⁻⁶ probability. On the other hand in the method, introducing the chromosome elimination cassette can be performed the elimination of chromosome at the high probability of mutation more than 100 times. The method in the present invention is superior in the point which can be eliminated a plurality of chromosomes easily.

Any sequences can be used as long as the recombinase recognition sequence used in the present invention can recognize the recombinase used and the person skilled in the art of recombinase art can design such recombinase recognition sequences easily. In the present specification, whether such a recombinase recognition sequence is the recombinase recognition sequence, it is easily possible to identify that the DNA recombination occurs between two recombinase sequences, the recombinase sequences are integrated into one as the result, and DNA between recombinase sequences are defected or combined together by treatment of recombinase enzyme. As such a sequence, loxP sequence, FRT site, attB sequence, attp sequence, res site sequence, or the like can be includeded, but is not limited to, for example. Such a recombinase recognition sequence includes the sequence (loxP sequence) described in sequence number 1.

The marker gene as used in the present invention can be used any genes, when the marker gene is introduced into a cell and the distinction of the cell is possible. As such a marker gene, for example, GFP gene, (including EGFP gene), CFP gene, YFP gene, dsRED gene, or the like can be includeded.

According to the preferable embodiment, the gene cassette in the present invention may have selection sequence further. As a selection sequence used in the present invention, for example, any sequence may be used as long as the sequence which can select the introduced cell under particular conditions after the gene introduction, for example, antibiotic resistance gene (for example, thymidine kinase (TK) gene), puromycin resistance gene, neomycin resistance gene, hygromycin resistance gene, blasticidin resistance gene, zeosin resistance gene, diphtheria toxin resistance gene, fluorouracil resistance gene, or the like), HAT resistance genes, or the like can be included, but is not limited to.

According to the preferable embodiment, the gene cassette in the present invention may include IRES sequence (sequence number 2). IRES sequence used in the present invention can be any sequence as long as a sequence which has IRES activity. Such IRES sequence includes the sequence described in sequence number 2 or the functional equivalent thereof (for example, including 1 or some substitution, addition, or deletion).

In the preferable embodiment in the present invention, the gene cassette of the present invention may further include CAG sequence (sequence number 3). CAG sequence used in the present invention may be used any sequence as long as CAG sequence which has the normal activity.

Therefore, in the preferable embodiment of the present invention, in the gene cassette of the present invention, recombinase recognition sequence is included in two opposite directions and includeed CAG, marker sequence, IRES sequence, and selection sequence in between the recombinase recognition sequences. According to another preferable embodiment, Pgk, selection sequence, (IRES sequence), and marker arrangement are included. The former is used for introducing at random and the latter is used for introducing into a target chromosome by homologous recombination. The latter may be used for selecting the CEC homologous ES cell by high concentration G418.

### (Constituent for eliminating a desired chromosome)

In another aspect, the present invention provides a gene cassette which includes The gene cassette including a recombinase recognition sequence in one or two opposite direction, wherein a composition for the elimination of the desired chromosome including the sequence coded a marker gene and including the gene cassette. It is understood that the gene cassette included herein can be used for any style of gene cassette described above (gene cassette).

Here, any cells are included as object cells to eliminate a desired chromosome. As such a cell, for example Stem cells, such as ES cell, EG cell, tissue stem cell, or the like, somatic cell, the fused cell between somatic cell and stem cell (refer to international publication WO03/027278), the reprogrammed stem cell (refer to international publication WO03/027277, international publication WO03/027278, or the like), or the like can be included.

A chromosome which may be eliminated can includes any chromosomes and preferably any autosomes except for X chromosome and Y chromosome as the object. As such an example of chromosome, for example, 1st chromosome to 22nd chromosome especially 5th chromosome, 9th chromosome, 11th chromosome, 13th chromosome, 18th chromosome, 21st chromosome, or the like can be included in human 46 chromosomes. In mouse, 1st chromosome to 19th chromosome especially 2nd, 7th, 11th chromosome, or the like can be included. It is preferred to eliminate the chromosome containing MHC or HLA (respectively mouse 17th chromosome and human 6th chromosome) especially. In the eliminated cell, it is because immunorejection can be removed.

A chromosome consists of two chromatids. The point which chromatids join is termed centromere. At the time of nuclear division, microtubules combine together in the point and pull toward the two poles. On both sides of centromere, a long side is termed long arm and a short side is termed short arm. The terminal part of chromosome has a characteristic structure called telomere. On the first stage of mitosis, the chromatin chain is condensed gradually. A chromosome changes to the compact form which can move from the form which functions as genetic material in this process. Then, two corresponding chromatids (condensed chromatin chain) turn into a chromosome associated the centromere. The long microtubule (spindle) extended from two poles in the cell associates the centromere. During nuclear division, spindle pulls apart each chromatid toward two poles of the cell, and finally each daughter cell inherits one set of chromatid. The number of chromosome is constant for each species. The cell of the species proliferated by asexual reproduction have only one set of chromosomes, and the same is mentioned all the cells in the organism. The species which perform sexual reproduction has a somatic cell of diploid [2x] or multiploid [Nx] and a gamete of haploid [integral multiple of n=x]. Diploid is two set chromosomes and one set is derived from a father or the like and another set is derived from another individual origin such as a mother or the like. Multiploid has three sets or more chromosomes and haploid has only one set. In mammals, when the gametes of male and female are fused in fertilization, meiosis occurs in egg cell, which is still diploid at that time, and maturation of fertilized egg occurs. In process of meiosis, the chromosomes corresponding to mother and father occurs intersection and exchanges the part each other. The fused cell between stem cell and somatic cell produced by fusion is tetraploid. In this case, since sister chromatids after DNA replication occured homologous recombination between CEC(s) by using recombinase, centromere and telomere are disordered and chromosome comes to be eliminated. Monoploid is termed haploid phase and diploid is also termed diploid phase. The pattern diagram for eliminating chromosome in the present invention is illustrated in Fig. 3.

1. When chromosome is eliminated by differentiated cell, the cell from which the chromosome was eliminated is difficult to obtain for the gene dosage compensation mechanism. However, it is clear that the undifferentiated cell has tolerance about the gene dosage compensation from the invention of the present inventors related tp inactivation of X chromosome or genome imprinting. Originality is the point solved the problem of the gene dosage compensation by eliminating chromosome in the embryonic stem (ES) cell which is an undifferentiated cell based on these discovery.

2. Although elimination of Y chromosome, which is sex chromosome, is reported in the conventional chromosomal engineering experiment, there is no example of the diploid cell eliminated whole one autosome. Although recombination between homologous chromosomes and recombination between non-homologous chromosomes is reported in autosome, the frequency to obtain is remarkably low. Originality is the point of having brought about chromosome elimination relatively high frequency with forcibly inducing a chromosome aberration by applying the mechanism of homologous recombination between sister chromatids in the G2 phase cell after DNA replication.

Originality is the point of having built the chromosome elimination cassette which introduced homologous recombination sequences (recombinase sequence), such as LoxP or the like, into two as one pair in the opposite direction for the purpose of the improvement of the recombination efficiency between sister chromatids, i.e., the improvement of chromosome elimination efficiency.

According to one embodiment, in the cell targeted the constituent in the present invention, the chromosome which should be eliminated may include a pluripotency related gene. As a pluripotency related gene, Nanog, Oct3/4, other specific antigens, or the like can be included here, for example.

In another embodiment, in the cell targeted the constituent in the present invention, the chromosomes which should be eliminated may be both copies.

### (Kit)

The present invention provides the kit for the elimination of the desired chromosome, wherein A) the gene cassette including the recombinase recognition sequence in one or two opposite direction, wherein the gene cassette including the sequence coded the marker gene; andB) the recombinase corresponding to the recombinase recognition sequence or the nucleic acid molecules including the nucleic acid sequences coded the recombinase recognition sequence. Here, as a gene cassette, it is understood that any forms described above (gene cassette) can be used. The recombinase used here or nucleic acid molecules may be used any kinds of that as long as the recombinase or nucleic acid sequences encoding the recombinase is appropriate. According to the included recombinase recognition sequence, such a suitable recombinase can be selected by a person skilled in the art. In this case, the following correspondences are illustrated:
loxP sequence Cre recombinase
FRT (Flprecognitiontarget) site: Flp integrase
attB sequence: ΦC31 integrase
attP sequence: ΦC31 integrase
res (resolution) site sequence: IntI1 integrase.

In another aspect, the present invention provide the kit for the elimination of the desired chromosome from the stem cell, wherein A) the gene cassette including the recombinase recognition sequence in one or two opposite direction, wherein the gene cassette including the sequence coded a marker gene; B) the recombinase corresponding to the recombinase recognition sequence or a nucleic acid molecules including the nucleic acid sequences coded the recombinase recognition sequence; and C) the stem cell. Here, as a stem cell, any stem cells aiming at eliminating a desired chromosome can be provided. The present invention has a prominent effect which is the point providing the art which can be eliminated chromosome from the cell having pluripotency such as stem cell while maintaining the pluripotency. The stem cell used may be provided in a fresh state, and may be provided by a preservation state. Although such a preservation is frequently the cryopreservation of stem cells, such as ES cell or the like, the conventional slow cooling method (for example, Freshney R. I., Culture of Animal Cells: A Manual of Basic Technique, Wiley-Liss, Inc., pp.255-265, 1994), the later developed method of vitrification (Reubinoff BE, Para MF, Vajta G, Trounson AO., Hum Reprod. 2001 Oct; 16(10): 2187-94) (This method has higher efficiency and is simpler than the slow cooling method), or the like can be used.

In another aspect, the present invention provide the kit for providing the cell with the pluripotency by the elimination of the desired chromosome from the cell having the desired genome, the chromosome, or the group of chromosomes, wherein A) the gene cassette including the recombinase recognition sequence in one or two opposite direction, wherein the gene cassette including the sequence coded a marker gene; B) the recombinase corresponding to the recombinase recognition sequence or a nucleic acid molecules including the nucleic acid sequences coded the recombinase recognition sequence; C) the stem cell; and D) the cell having the desired genome, the chromosome, or the group of chromosomes. Here, a desired tailor made stem cell can be produced after providing the stem cell and the cell having a desired genome. The production method of the fused cell is described in WO03/027278 and the description is used entirely as reference in the present specification. For example, the method of fusing between a stem cell (for example, ES cell) and a somatic cell can be any methods of fusing by contact a stem cell with a somatic cell and forming a fused cell, but is not limited to. For example, as described in the embodiment, ES cell and somatic cell in making a fused cell with a constant ratio, for example, the case making the fused cell between ES cell and thymus cell at the ratio of 1:5, are mixed and washed. It can be produced by suspending the cells into a suitable buffer solution such as mannitol buffer solution or the like and performing electrofusion. The high voltage pulse cell fusion method by using structural change of cell membrane by such electrical stimulation (electroporation) [for example, EMBOJ.1: 841-845(1982)], and the cell fusion method used chemical cell fusion promoting agents such as Sendai Virus, lysolecithin, glycerol, oleate ester, polyethylene glycerol, or the like may be used. Any fusion methods which the formed cell by fusion between stem cell and somatic cell can be made to increase stably as a fused cell and the nucleus derived from somatic cell can change to undifferentiated cell like having pluripotency, are not especially limited.

As used in the present specification, the term "fusion" or "cell fusion" in relation to a cell are used interchangeably and refers to a phenomenon that a plurality of cells are fused together into a multinucleated cell. Fusion naturally occurs in, for example, fertilization of germ cells, and is used as a cell engineering means. To achieve fusion, 2 types of different cells are chemically or physically fused and cultured by using a selective medium in which only fusion cells can grow. For example, cell fusion can be induced by using a virus whose infectiosity is inactivated by ultraviolet (e.g., paramyxoviruses, such as HVJ (Sendai virus), parainfluenza virus, Newcastle disease virus, or the like). Also, by using chemical substances, cell fusion can be achieved by such chemical substances including lysolecithin, polyethyleneglycol (PEG) 6000, glycerol oleate ester, or the like. As a physical technique, for example, cell fusion (electric fusion) with electric stimuli is performed. Cell fusion with chemical substances is preferably independent and non-specific to viruses.

In another aspect, the present invention provide the method for providing the cell with the pluripotency by the elimination of the desired chromosome from the cell having the desired genome, wherein A) the gene cassette including the recombinase recognition sequence in one or two opposite direction, wherein a step of providing the gene cassette including the sequence coded a marker gene; B) a step of introducing the gene cassette to the stem cell; C) a step of fusing between the stem cell and the desired genome, the chromosome, or the group of chromosomes; D) a step of providing the recombinase recognizing the recombinase recognition sequence or the nucleic acid molecules including the nucleic acid sequences coded the recombinase recognition sequence; E) a step of exposing the fused cell in the condition that occurs reconstitution; and F) a step of observing the signal derived from the marker gene in the fused cell and selecting the cell eliminated the desired chromosome.

In another aspect, the present invention provide a method for the elimination of the desired chromosome from the cell including the desired genome, the chromosome, or the group of chromosomes, wherein A) the gene cassette including the recombinase recognition sequence in one or two opposite direction, wherein a step of providing the gene cassette including the sequence coded a marker gene; B) a step of introducing the gene cassette to the stem cell; C) a step of fusing between the cell and the cell having the desired genome, the chromosome, or the group of chromosomes; D) a step of providing the fused cell with the recombinase recognizing the recombinase recognition sequence or the nucleic acid molecules including the nucleic acid sequences coded the recombinase recognition sequence; E) a step of exposing the fused cell in the condition that occurs the reconstitution; and F) a step of observing the signal derived from the marker gene of the fused cell and selecting the cell being eliminated the desired chromosome.

In other aspect, the present invention provide a method for production the chromosome recombinant cell, comprising: A) the gene cassette including the recombinase recognition sequence in one or two opposite direction, wherein a step of providing the gene cassette including the sequence coded a marker gene; B) a step of introducing the gene cassette to the stem cell; C) a step of fusing between the cytoplasm of the second cell having the desired chromosome and the first cell; D) a step of providing a recombinase recognizing the recombinase recognition sequence or the nucleic acid molecules including the nucleic acid sequences coded the recombinase recognition sequence so that the first cell can be eliminated the chromosome in the chromosome of first cell corresponding to the desired chromosome of second cell; E) a step of exposing the fused cell in the condition that occurs the reconstitution; and F) a step of observing the signal derived from the marker gene of the fused cell and selecting the cell being eliminated the desired chromosome.

Here, it is understood that recombinase recognition sequence, gene cassette, marker gene, or the like can be used any forms explained in the present specification (gene cassette).

It is understood that stem cells and cells having a desired genome used in the method of the present invention can be used any cells as long as the fusion may be obtained.

In the present invention, it is understood that the fusion art used and recombinase or nucleic acid molecules encoding the recombinase are used any forms explained in (kit) of the present specification.

In the present invention, the condition of "enabling chromosome elimination" can be used any conditions explained in the present specification. Here, the condition that occurs the reconstitution may be used.

In the present invention, "the condition that occurs the reconstitution" can be any conditions in which recombination occurs, and such conditions can be easily selected by a person skilled in the art. The conditions which such reconstitution occurs can be included 1. cell is dividing 2. cell is maintaining the undifferentiated state, or the like, for example.

In the method of the present invention, the observation of signal derived from the marker gene can be performed by the means which the person skilled in the art selected suitably according to the signal generating from the marker gene. For example, in the case of green fluorescence protein, it can be performed by using the means to observe fluorescence, for example, by using fluorescence microscope or the like.

In other aspect, the present invention provide a method for the elimination of the desired chromosome from the cell including the desired genome, wherein A) the gene cassette including the recombinase recognition sequence in one or two opposite direction, wherein a step of providing the gene cassette including the sequence coded a marker gene; B) a step of introducing the gene cassette to the stem cell; C) a step of providing the fused cell with the recombinase recognizing the recombinase recognition sequence or the nucleic acid molecules including the nucleic acid sequences coded the recombinase recognition sequence; D) a step of exposing the fused cell in the condition that occurs the reconstitution; and E) a step of observing the signal derived from the marker gene and selecting the cell being eliminated the desired chromosome.

### (Cell or the like)

In other aspect, the present invention also provide the cell in which is eliminated the desired chromosome (for example, stem cells such as ES cell or the like). Such a cell can be produced by the method of the present invention, for example. As the preferable embodiment, the chromosome including MHC is eliminated in this cell. Conventionally, such a chromosome eliminated cell was not succeeded in obtaining efficiently.

In another aspect, the present invention provides the cell obtained by the method of the present invention.

In one embodiment, the present invention provides the fused cell between the ES cell and the cell having the desired genome, wherein the fused cell provided the pluripotency by the elimination of the desired chromosome from the fused cell.

In another preferred embodiment, the present invention provides the fused cell between the ES cell and the cell having the desired genome, wherein the fused cell provided the pluripotency by the elimination of the chromosome including the sequence encoding major histocompatibility (antigen gene) complex (MHC) of the ES cell from the fused cell. As such MHC or the corresponding gene, mouse H-2 antigen (17th chromosome) and human HLA antigen (6th chromosome) can be included.

In one embodiment, the present invention provides the fused cell between the ES cell and the cell having the desired genome, the fused cell mentioned above, wherein said desired chromosome is the chromosome being selected from a group of 6^{th} chromosome, 11^{th} chromosome, 12^{th} chromosome, and 17^{th} chromosome of mouse and 6^{th} chromosome of human (corresponding to 17^{th} chromosome of mouse) or the chromosome including one copy or both copies of the chromosome(s) corresponding to the chromosome(s) and wherein the fused cell which has pluripotency by the elimination of one or a plurality of chromosome. Here, the correspondence (synteny) between chromosomes can be determined by using known art, for example, Nature 409, 860-921 (15 February 2001), http://bioportal.ddbj.nig.ac.jp/synteny/index.html, http://www-btls.jst.go.jp/ComparativeGenomics/indexj.html, or the like. In the present specifcation, "Synteny" means that the gene is arranged in the same order or the region between the chromosomes in different species. Before decoding the genome sequence, synteny was confirmed by genetic map, chromosome mapping, or the like, but presently, as the genome is decoded in detail, the analysis was progressed in a sequence level, and now, chromosomes between human and puffer fish can be compared.

In one detailed embodiment, the above mentioned desired chromosome, which is the fused cell between ES cell and the cell having a desired genome, is a chromosome including Nanog gene of ES cell, and the cell being eliminated the one or both copy of chromosome and having pluripotency is provided.

In one embodiment, the present invention provides the fused cell between the ES cell and the cell having the desired genome, wherein the fused cell provided the pluripotency by the elimination of one or more than two chromosome (for example, all of chromosome) of ES cell. It is preferred that all are eliminated. It is further preferred that such cells are all eliminated the chromosome or chromosome group related to disease derived from ES cell substantially. It is because immune rejection can be prevented. The elimination of two or more chromosomes may repeat the art to eliminate one chromosome provided in the present invention a plurality of times. Such art can be applied by commonly known art in the field, and can be performed based on the sequence information of the detailed chromosome. When the number of chromosomes is repeated, all desired chromosomes can be eliminated. For example, all the chromosomes defived from ES cell in the fused cell between ES cell and a desired cell can also be eliminated.

In one embodiment, it is provided that the fused cell between the ES cell and the cell having the desired genome, wherein the cell having said desired genome can be the cell derived from diseased individual. Such a cell may be used in order to examine the cause of disease.

In another embodiment, it is provided that the fused cell between the ES cell and the cell having the desired genome, wherein the cell having said desired genome is the cell derived from diseased individual, eliminated all the chromosomes in the ES cell, and having pluripotency. Such a cell is preferable in order to examine the cause of disease.

In another aspect, the present invention provides the chromosome recombinant cell, wherein the desired chromosome or the group of chromosomes is modified. Here, in chromosomal recombination, cytoplasm with the chromosome (two copies or one copy) of somatic cell is fused with ES cell, and the same chromosome defived from ES cell as the inserted chromosome can be eliminated by the cassette, for example. Preferably, the cell is the chromosome recombinant stem cell, wherein the desired chromosome or the group of chromosomes is modified, but is not limited to. For example, such a stem cell may be ES cell.

In one detailed embodiment, the present invention provides the fused cell with ES cell and the micronucleus having the desired genome, the chromosome or the group of chromosomes, wherein the chromosome recombinant cell provided the pluripotency by the elimination of the desired chromosome derived from the ES cell from the fused cell.

The present invention provides any cells produced by the art in the present invention.

### (Use)

In another aspect, the present invention provides the gene cassette including the recombinase recognition sequence in one or two opposite direction, wherein the use for the elimination of the desired chromosome in the gene cassette including the sequence coded the marker gene.

In the other aspect, the present invention provides the gene cassette including the recombinase recognition sequence in one or two opposite direction, wherein the use of producing the composition for the elimination of the desired chromosome in the gene cassette including the sequence coded the marker gene.

Here, it is understood that the art about chromosome elimination or the like can be used any art described in the present specification at other points.

### (Cell library)

In another aspect, the present invention provides the cell library, wherein including a plurality kind of cells being eliminated the desired chromosome. Such a library can be provided by producing the gene cassette for eliminating the target chromosome by using the gene cassette in the present invention described above (gene cassette), and producing the cell from which various chromosomes were eliminated. The designing method of the gene cassette for eliminating a desired chromosome is as follows:
The unique DNA sequence on 1st chromosome, in many cases the genomic DNA sequence of particular gene on 1st chromosome, is identified.
The above mentioned gene cassette is inserted in the identified DNA sequence.
Under the present circumstances, it is preferred that the DNA sequence to be used is a length of 5 or more kbs and the both sides of insertion gene cassette become equal length (one side 2.5kb).

When the gene cassette in between the DNA sequence on 1st chromosome is performed the gene introduction into ES cell, the gene cassette is inserted on the target 1st chromosome because the recombination in the DNA homology region occurs. Insertion the gene cassette to 1st chromosome can be confirmed by the FISH method.

As compared with the conventional methods, Unexpected effect is accomplished in the point that the present invention provides the deletion for each chromosome which was impossible or inefficient conventionally could be performed easily with maintaining regenerative capacity or pluripotency preferably.

References quoted in the present specification, such as scientific literatures, patents, and patent applications, are applied as reference in the present specification to the same degree of each concrete description wholly.

In the above, the present invention has been explained with shown the preferable embodiment for easy understanding. Although the present invention was explained below based on the embodiment, the above mentioned explanation and the following embodiments are provided as only the purpose of illustration, and are not provided in order to limit the present invention. Therefore, the scope of the present invention is not limited to the embodiments and examples described concretely in the present specification, but is limited to the scope of claims.

### (Embodiment)

Although the present invention is explained in more detail with embodiments shown below, the presemt invention is not limited to the following examples. Reagents or the like used in the following example are commercially available from Sigma (St. Louis, USA), Wako Pure Chemical Industries, Ltd. (Osaka, Japan), with some exceptions. The handling of animals was performed by observing the guideline specified in Kyoto University. The present invention is explained below with concrete examples. It is easy for a person skilled in the art to implement by replacing the constituent factor such as start plasmid, promotor, or the like used in such an example to the equivalent.

### (Embodiment 1 Calibration of the validity of CEC; Chromosome Elimination Cassette)

### 1. Construction of CEC

It is confirmed whether elimination of a particular chromosome is possible by designed CEC and the function of CEC in the first step of experiment, and introduction of CEC was attempted to a particular chromosome by homologous recombination in the second step.

CEC was consisted of LoxP->CAG-GFP-IRES-Puro<-LoxP (sequence number 11), and recombination occurred between homology chromatids by LoxP sequence introduced into the opposite direction. The dicentric chromosome and the akinetic chromosome were formed by the combination of homologous recombination in a certain frequency. These abnormal chromosomes were eliminated from the cell through cell division. As a result, the cell which defects only a chromosome into which CEC was introduced was produced.

The result is shown in Fig. 1 (especially Figs. 1E and 1F) and Fig. 2. Distribution of the deleted chromosome in mouse is shown by the karyotype analysis of cell in one embodiment. Here, it is understood that 11th chromosome is eliminated completely.

### 2. Introduction of Chromosome Elimination Cassette to ES Cell

CEC is introduced to the male ES cell strain defected Hprt gene as described in HM-1 (Selfridge, J., Pow, A. M., McWhir, J., Magin, T. M. and Melton, D. W. (1992) Gene targeting by using mouse HPRT minigene / HPRT-deficient embryonic stem cell system: inactivation of the mouse ERCC-1 gene. Somat Cell Mol Genet 18, 325-36 and in the present example, use permission is obtained and received (approved distribution /use permission) from Dr. Martin J. Evans, in School of Biosciences, Cardiff University, Cardiff, Wales, UK). By using HM-1 for the experiment, it became possible to select the fused cell with all the normal somatic cells of Hprt (+) by HAT medium. The gene introduction to HM-1 is performing by the electroporation method, and the introduction of single copy of CEC was urged. pCECDNA of 50-100µg was introduced into 1x10 cells under conditions of 250V / 500µF. After performing drug selection by puromycin, GFP positive cell was cloned selectively under the microscope. DNA was extracted from the obtained clone, introduced gene was reconfirmed by PCR, and the introduced CEC copy number was confirmed by Southern hybridization method. The clone which is introduced the single copy and is expressed high GFP was used for the cell fusion experiment. The result is shown in Fig. 4. Fig. 4 shows the introduction of the chromosome elimination cassette to ES cell, and FISH analysis of the introduced cassette. As explained in the pattern diagram illustrating the mechanism of chromosome elimination of the present invention shown in Fig. 3, it can be considered that various mechanisms have occurred.

### 3. Cell Fusion between Change ES Cell and Somatic Cell

CECES cell and thymus cell derived from the 129Rosa26 were fused electrically. The cell mixed solution of ES cells and thymus cells (1:5) was treated and fused by using ECM2001 (BTX) device (existing) treated on conditions (10 V (AC) / 90 s -250 V (DC) / 10 µsecond). After treatment for the cells, only ES -thymusl fused cell can be survived by culturing in HAT selection medium. The expression of GFP in the fused cell and the normal karyotype were confirmed. The result is shown in Fig. 5. The pattern diagram is illustrated in Fig. 8.

### 4. Chromosome elimination induction from Fused Cell

pCMV-Cre was intrroduced by lipofection into the fused cell, Cre enzyme was transiently expressed, and recombination in CEC introduction chromosome was induced by culturing for 48 to 96 hours.

### 5. Screening of fused cell eliminated chromosome

By using cell sorter, the fused cell clone treated with Cre enzyme is separated into GFP positive cell and GFP negative cell, and GFP negative cell is isolated. G-band analysis of the chromosome karyotype of GFP negative cell was performed, and the commonly defected chromosome between cells was identified. The operation was repeated by the clone.

### 6. Check of chromosome elimination

The chromosome FISH (Fluorescence in situ hybiridization) analysis was performed by using pCEC as the probe to CEC-HM-1 ES cell before cell fusion, and the chromosome introduced CEC was identified. If CEC chromosome is the same chromosome as the chromosome identified by 5., it means that the chromosome was selectively eliminated by CEC. FISH analysis by chromosome specific repeat sequences other than G-band analysis for chromosome identification was used in parallel, and the identified chromosome was confirmed. The result is shown in Figs. 4-6.

As shown in Fig. 7, when the elimination cassette insertion site on the chromosome was analyzed by FISH method, the chromosome was eliminated efficiently.

### (Embodiment 2, production of chromosome elimination library)

### 1. Elimination of Any Chromosome

CEC was introduced on the other chromosome in ES cell, and it demonstrated that any chromosomes could be eliminated from ES-somatic fused cell by using different chromosomes, respectively. The method was to perform repeatedly the experiment of 1-6 in other chromosomes.

2. Construct pLox P->Pgk-Neo-IRES-GFP<-LoxP with neomycin instead of puromycin for selection of production CEC of CEC neomycin selection construct was produced and used for any chromosome elimination.

### 3. Production of CEC cassette homozygote

pLoxP->Pgk-Neo-IRES-GFP<-LoxP and CEC cassette were inserted on any chromosomes. By treatment with high concentration (8 mg/ml) neomycin, ES cell having CEC in homo junction was obtained.

### 4. Production of chromosome elimination library

ES library having CEC homozygously in a particular chromosome and somatic cell were fused, and the system which can produce freely the fused cell, which a particular chromosome set is derived from only somatic cell by eliminating chromosome derived from ES cell by using treatment Cre enzyme with the fused cell, was created.

By using the mouse which has disease, a particular characteristic genetic polymorphism, or the like in somatic cells, the application to tailor made innovative drug development ore the like became available. Introducing the similar system to human ES cell hereby, more effective analysis become available.

### (Embodiment 3)

### Elimination of the specific chromosome by adapting homologous recombination

### 1. Elimination of mouse 17th chromosome

As the first step of tailor made stem cell production, construction is performed for the purpose of eliminating mouse 17th chromosome. On mouse 17th chromosome, MHC (major histocompatibility antigen) gene cluster in respect of immunorejection is existed. By eliminating selectively 17th chromosome derived from ES cell, MHC expression from the fused cell is derived only somatic cell. As a result, a large decrease of rejection response to the differentiated cell derived from the fused cell is observed.

### 2. Homologous Recombination Sequence

Genomic DNA of H2-b gene on 17th chromosome is directly cloned by PCR from mouse genomic library or genome. The genome fragment required for homologous recombination (Right side arm (RA) and left side arm (LA)) were connected to CEC, and pRA-LoxP->CAG-GFP-IRES-Puro<-LoxP-LA plasmid and pRA-LoxP->CAG-dsRed-IRES-hygro<-LoxP-LA were constructed. The pattern diagram is shown in Fig. 3 upper left.

### 3. Homologous Recombination

pRA-LoxP->CAG-GFP-IRES-Puro<-LoxP-LA is inserted on 17th chromosome by homologous recombination using HM-1 ES cell. Then, it is inserted on the second locus by using pRA-LoxP->CAG-dsRed-IRES-hygro<-LoxP-LA. ES cell introduced CEC into both loci of H2-b gene was obtained.

### 4. FISH Analysis

ES cell having CEC in each chromosome was identified, and the library was produced.

ES cell introduced CEC homozygously into the H2-b gene locus of mouse 17th chromosome and somatic cell were fused. The fused cell was treated by pCMV-Cre and the clone eliminated 17th chromosome derived from ES cell was obtained. The genetic manipulating ES cell was induced differentiation to various tissues in in vivo and in vitro, and the presence and the degree of ejection response was examined by performing replanting experiments.

### (Embodiment 4, elimination of chromosome containing HLA related gene by applying homologous recombination)

The chromosome containing HLA related gene is eliminated by using the similar approach as Embodiment 3. Since HLA related gene is contained in human 6th chromosome, in the above mentioned procedure, the experiments are performed according to the above mentioned embodiment except by using the specific procedure for human 6th chromosome. Then, it is understood that human 6th chromosome is eliminated efficiently similarly.

### (Embodiment 5, the example of different recombinase)

Chromosome elimination is performed by using FRT sequence instead of LoxP sequence. Elimination experiment is performed based on the above mentioned embodiment, by using GAAGTTCCTATTCTCTAGAAAGTATAGGAACTTC (sequence number 4) as FRT site and by using Flp integrase as integrase. For example, it is understood that the target chromosome is efficiently eliminated in a similar manner when chromosome elimination experiment is performed in mouse 17th chromosome as a target.

### (Embodiment 6, the example of different cell)

By using EG (Embryonic Germ) cell instead of ES cell, the fused cell is produced and chromosome elimination can be performed. Here, EG cell can be produced based on Reprod Fertil Dev. 2001; 13(7-8): 661-4 or the like. In EG cell, it is understood similarly that chromosome elimination occurs efficiently.

### (Embodiment 7, the example of different animal species cell)

A similar experiment is performed by using monkey ES cell. The experimental procedure follows the above mentioned embodiment. As a result, it is understood similarly that chromosome elimination occurs efficiently in monkey ES cell.

### (Embodiment 8: the example of elimination ofNanog gene)

### (Method)

### (Chromosome elimination cassette)

The insertion of loxP² cassette vector (pBS246 (Gibco-BRL)) was amplified by PCR and subcloned into pGEM-T Easy (Promega). The loxP site was recloned in the opposite direction (pGEM-CEC). CAG-gfp / IRES (Internal Ribosome Entry Site). puro-pA fragment was inserted into pGEM-CEC, and pCEC-CAG-gfp / IRES. puro-pA was produced for random insertion (Fig. 9a). pGEM-CEC was subcloned into pBS-SK(+) (Stratagene) for the 6th chromosome elimination (pBS-CEC). pBS-CEC Pgk-neo / IRES.gfp-pA fragment was inserted into pBS-CEC, and pCEC-Pgk-neo /IRES.gfp pA was produced. For homologous recombination in a Gt(ROSA)26Sor locus, CEC-Pgk-neo / IRES.gfp-pA was inserted into pROSA26-pA (pROSA26-CEC) (Srinivas, S. et al., Cre reporter strains produced by targeted insertion of EYFP and ECFP into the ROSA26 locus, BMC Dev Biol 1, 4(2001)) (Fig. 11a).

In order to obtain stable transformants, electroporation of the straight chain pCEC-CAG-gfp / IRES.puro-pA DNA by ScaI was performed to HMIES cell by using Gene Pulser II (BioRad). In order to make Cre expression vector and pBS185 (pCMV-Cre) (Gibco-BRL) express transiently, they are transfected to the hybrid cell by using LIPOFECTAMINE 2000 (Invitrogen). For the homologous recombination to Gt(ROSA)26Sor locus, electroporation of the straight chain pROSA-CEC plasmid DNA by PvuI was performed to HMI ES cell.

### (Cell fusion)

HMIES cell with defect about Hprt gene (129 / O1a Mus musculus domesticus) was cultured on the first embryonic fibroblast feeder cells in ES medium (Tada, M. et al., Pluripotency of reprogrammed somatic genomes in embryonic stem hybrid cells. Dev Dyn 227, 504-10 (2003)). CEC-transgenic HMI ES cell was electrically fused with the thymus cells collected from female 129ROSA26 (M m. domesticus) or JF1 (M m. molossinus) mouse (Tada, M. et al. Pluripotency of reprogrammed somatic genomes in embryonic stem hybrid cells. Dev Dyn 227, 504-10 (2003).). The hybrid cell clone was selected by HAT medium.

### (Selection of G418 high concentration drug resistance clone (protocol for homozygous cell selection))

The transgenic ES cell introduced Pgk-neo cassette is selected by 250 µg/ml G418.
2. G418 resistance ES cells (5x10⁶ pieces) are unleashed to culture dish of 3cm in diameter (6-well plate), and are cultured for three days under 5-10 mg/ml high concentration G418. Selection media are changed every day during this period. They are subcultured to 3 pieces of 3cm culture dish after three day from starting selective culture, and are cultured for 4-7 days under 5-10 mg/ml high concentration G418. Selection media are changed every day during this period.
4. The colony of high concentration G418 resistance ES cells appears about 40.
5. DNA from each colony is extracted and the clone which has Pgk-neo cassette in homozygous is selected by genome PCR method or Southern hybridization method.

### (FACS classification)

Hybrid cells were suspended in PBS containing 2% fetal calf serum and 2 µg/ml propidium iodide (Sigma). About 10,000-500,000 cells were provided to the quantitative analysis, and 100-1,000 GFP negative hybrid cells were collected by FACS Vantage (Becton Dickinson).

### (FISH analysis)

Followining the standard G band formation procedure (standard G-banding procedure), the chromosome analysis was performed (Tada, M., Tada, T., Lefebvre, L., Baton, S. C. and Surani, M. A. Embryonic germ cells induce epigenetic reprogramming of somatic nucleus in hybrid cells. EMBO J. 16, 6510-20 (1997)). For mapping, chromosomal preparations were hybridized to the CEC vector specific probe or the centromere main satellite specific probe (centromere major satellite specific probe) (Lehnertz, B. et al., Suv39h-mediated histone H3 lysine 9 methylation directs DNA methylation to major satellite repeats at pericentric heterochromatin. Curr Biol 13, 1192-200 (2003)), and were detected (Refer to Lawrence, J.B., Singer, R. H. and Marselle L. M. Highly localized tracks of specific transcripts within interphase nuclei visualized by in situ hybridization. Cell 57, 493-502 (1989)). For chromosome painting, according to the chromosome painting protocol, chromosomal preparations were hybridized to the specific STAR FISH probe (Cambio) for 6th chromosome, 11th chromosome, 12th chromosome, and 17th chromosome, and were detected.

### (Genome PCR and RT-PCR)

Genomic PCR products were amplified for 35 cycles with an annealing temperature of 60 °C. D6Mit183, D6Mit102, and D6Mit14 were used as the mouse 6th chromosome specific marker for detecting the DNA sequence polymorphism between 129 and JF1: D6Mit183 (129; 94bp and JF1; 190bp), Primer 1; 5'-TTCTCAATGAACACTAGAACATTCG-3' (sequence number 12), and primer 2; 5'-AAAACACAGGTAGAAAACATACATACA-3' (sequence number 13); D6Mit102 (129; 177 bp, and JFI; 125bp), Primer 1; 5'-CCATGTGGATATCTTCCCTTG-3' (sequence number 14) and primer 2; 5'-GTATACCCAGTTGTAAATCTrGTGTG-3' (sequence number 15); D6Mit14 (129; 155 bp, and JFI; 147bp), Primer 1; 5'-ATGCAGAAACATGAGTGGGG-3' (sequence number 16), and Primer 2; 5'-CACAAGGCCTGATGACCTCT-3' (sequence number 17). GFP DNA was amplified by PCR using the following primer sets: GFPF; 5'-CGTAAACGGCCACAAGTTCA-3' (sequence number 18) and GFPR; 5'-CGCTTTACTTGTACAGCTCGT-3' (sequence number 19). cDNA was synthesized by using oligo dT primer from DNaseI treated RNA extracted from ES cell, Cre hybrid cell, and Cre treatment hybrid cell for RT-PCR of Nanog and Stella / PGC7. RT-PCR products were amplified for 35 cycles with an annealing temperature at 60 °C by using the following specific primers: Nanog F1; 5'-GCGCATTTTAGCACCCCACA-3' (sequence number 20) and R1; 5'-GTTCTAAGTCCTAGGTTTGC-3' (sequence number 21); Stella / PGC7 F; 5'-ACAGACTGACTGCTAATTGG-3' (sequence number 22) and R; 5'-GGAAATTAGAACGTACATACTCC-3' (sequence number 23). G3pdh was amplified by using the following primers; F; 5'-TGAAGGTCGGTGTGAACGGATTTGGC-3' (sequence number 24) and R; 5'-CATGTAGGCCATGAGGTCCAC-3' (sequence number 25).

### (Southern blot hybridization)

In order to detect homologous recombination phenomena, genomic DNA was extracted from GFP positive clone, digested by EcoRV, separated by 1.0% agarose gel, and transported to Hybond N+ membrane by alkali blotting. The membrane was preliminarily hybridized, hybridized with pROSA26-S' plasmid which is labeled by 32 P-dCTP using the Mega primeDNA label system (Amersham) (Srinivas, S. et al. Cre reporter strains produced by targeted insertion of EYFP and ECFP into the ROSA26 locus, BMC Dev Biol 1, 4 (2001) (Fig. 11a) at 42 °C overnight, and then washed by 2 x SSPE / 0.1% SDS and 0.1 x SSPE / 0.1% at 65 °C.

### (Western blot hybridization)

All the cell extracts were separated by the electrophoresis in 12%SDS polyacrylamide gel. These proteins were transported on the PVDF membrane (Millipore). The membrane was preliminarily hybridized in 3% skim milk (Difco) and in PBS at room temperature for 1 hour, and incubated with anti-NANOG antibody (1:1000 dilution) (Abeam) and anti-histone H3 antibody (1:3000 dilution) (Abeam) at 4 °C overnight. The bands were detected by using ECL Western blotting detection kit (Amersham).

### (Fluorescence immunohistochemistry)

Cre treatment hybrid cell was fixed with 4% formaldehyde for 10 minutes. Following the pretreatment by blocking solution (2% skim milk) for 1 hour, cells were incubated at 4 °C overnight with anti-NANOG polyclonal antibody (rabbit IgG) (1:500) (Abeam) and anti-OCT4 monoclonal antibody (mouse IgG) (1:100) (SantaCnlz). After rinsing samples, they were incubated for 1 hour with Alexa546-conjugated anti-rabbit IgG antibody (1:500) (Molecular Probes) and FITC-conjugated anti-mouse IgG (1:1000) (Zymed).

### (Results and verification)

In order to examine the hypothesis, electroporation of GFP reporter and CEC (pCEC-CAG-gfp / IRES.puro-pA) including puro resistance gene was performed to Hprt defect HM-1 ES cell. Two out of 57 puro resistance and GFP positive clones were selected at random, and analyzed by fluorescence in situ hybridization (FISH) by using CEC specific probe. Both clones maintained the normal karyotype (2N=40, XY). In the other clone, CEC was inserted in the distance region of 11th chromosome (CEC11) (Fig. 9b). In the second clone, CEC was located on the proximal region of 12th chromosome (CEC12) (Fig. 9c). Cell fusion was performed between the both clones and thymus cells derived from female ROSA26 mouse having neo / lacZ gene which was expressed ubiquitously, they were selected by HAT (hypoxanthine, aminopterin, thymidine). Both CEC 11 hybrid cells and CEC12 hybrid cell had complete set (4N=80, XXXY) of chromosome (Fig. 9d and e).

CEC11 hybrid cell and CEC12 hybrid cell was induced recombination in CEC-mediated sister chromatids by the transient Cre expression and following pCMV-Cre lipofection. After subculturing 3 to 4 times and culturing further for ten days without selection, about 5.0x10⁴ to 2.5x10⁵ cells were classified by FACS (Fig. 9f). Without Cre treatment, GFP negative population were 0.8% and 3.3% in CEC11 hybrid cell and CEC12 hybrid cell, respectively. However, after Cre treatment, the above mentioned population were increased to 2.2% and 10.1%, respectively. In accordance with this, without Cre treatment, all the hybrid cells are GFP positives, on the other hand, with Cre treatment, the GFP negative hybrid cell classified by FACS produced the GFP negative colony (Fig. 9g).

The selective elimination of 11th (12th) chromosome from CEC11 hybrid cell (or CEC12 hybrid cell) was confirmed by chromosome painting analysis using the particular probe. Without Cre treatment, each CEC11 hybrid cell which was analyzed the nucleus included four 11th chromosomes and four 17th chromosomes (Fig. 10a). In each case, one 11th chromosome might be eliminated after Cre treatment, on the other hand, four 17th chromosomes were maintained (Fig. 10b and e). The similar situation was found in 12th chromosome elimination in CEC hybrid cell (Fig. 10c, d, and f). The attempt which amplifies the gfp specific product derived from Cre treatment FACS classification hybrid cell DNA was negative, and showed disappearance of CEC clearly. In order to further investigate the specificity of elimination which narrowed down the target and the insertion of non-target chromosome, a karyotype of CEC11 hybrid cell and CEC12 hybrid cell with Cre treatment was determined. Two separate isolated CEC11 hybrid clones is a karyotype of 79, XXXY, and -11, and it hardly or never had harmful influence to the other chromosome (Fig. 10e). On the other hand, the analyzed CEC12 hybrid clone is a karyotype of 79, XXXY, -12, and +marker (Fig. 10f). The marker was chromosome including minute centromere repeat as shown by FISH analysis using main satellite specific probes (Fig. 10g). That is, analysis of present inventors shows clearly that individual autosome can be effectively eliminated from ES x somatic cell hybrid nucleus. Interestingly, chromosome elimination frequency greatly differed between two insertion sites, which was 26.1% in CEC11 hybrid cell which showed disappearance of 11th chromosome and 88.4% in CEC12 hybrid cell which showed disappearance of 12th chromosome (Table 1).

**[Table 1]**

| Table 1 Cre-mediated chromosome elimination in somatic hybrid cell having ES cells carrying the CEC | | | | | | | | | | | | |
|---|---|---|---|---|---|---|---|---|---|---|---|---|
| | Number metaphase | Number of 11th chromosome of in CEC 11 hybrid | | | | | Number of metaphase | Number of 12th chromosome in CEC 12 hybrid | | | | |
| | | 1 | 2 | 3 | 4 | 5 | | 1 | 2 | 3 | 4 | 5 |
| GFP(+) | 63 | 0 | 0 | 0 | 63 | 0 | 66 | 0 | 0 | 1 | 64 | 1 |
| Pre-Cre cell | | | | | (100%) | | | | | (1.5%) | (97.0%) | (1.5%) |
| GFP(-) | 69 | 0 | 0 | 18 | 51 | 0 | 95 | 0 | 1 | 84 | 10 | 0 |
| Post-Cre cell | | | | (26.1%) | (73.9%) | | | | (1.1%) | (88.4%) | (10.5%) | |

Probably it reflects the influence of insertion site on a possibility that CEC contacts to Cre molecule. Regardless of this, analysis of present inventors showed clearly that the extensive possibility of the method of elimination which the target is any chromosomes or a desired chromosome group and the method can be applied to all the ES genomes.

In order to begin the elucidation whether or not the reprogramming of somatic genome was sufficient to give the stabilized stem cell characteristic under non-existence of ES cell genome, then present inventors targeted both ES cell copies in 6th chromosome. The copy includes the population (cluster) of pluripotency specificity gene (including Nanog gene) and Gt(ROSA)26Sor locus (Fig. 11a and f). Present inventors were able to investigate the following through the approach: (i) the validity of CEC tagged chromosome by homologous recombination which narrowed down the target, and (ii) the influence of eliminating one or more chromosomes from the same nucleus at once. The expression of Nanog is indispensable in order to maintain the regenerable pluripotency state in ES cell and early embryonic cell (Hatano, S. et al., Pluripotential competence of cells associated with Nanog activity. Mech Dev 122, 67-79 (2005); Mitsui, K. et al., The Homeoprotein Nanog Is Required for Maintenance of Pluripotency in Mouse Epiblast and ES Cells. Cell 113, 631-42. (2003)), and it is shown previously that the silence copy of somatic cell of Nanog is reactivated by nuclear transplantation and cell fusion (Hatano, S. et al., Pluripotential competence of cells associated with Nanog activity. Mech Dev 122, 67-79 (2005)). Gt(ROSA)26Sor locus is the upstream domain of Nanog, and it is known that homologous recombination occurs efficiently here. Electroporation of target vector having CEC including Pgk-neo / IRES.gfp gene subcloned by pROSA26-pA (Fig. 11a) was performed to the HMI ES cell. Two out of 18 examined G418 resistance clones are the correct target (CEC6^{tg/+}), found by Southern blot analysis producing the knock-in specific EcoRV fragment of 2.3kbs (Fig. 11b), and confirmed by FISH analysis (Fig. 11f). These clones were further divided and proliferated for ten days in the state which increased G418 concentration (8 mg/ml). All five clones examined from these conditions were found to be homozygosis (CEC6^{tg/tg}) about CEC as shown by disappearance of wild type specific band of 11kbs in Southern blot analysis (Fig. 11b). CEC6^{tg/tg} ES cell having a normal karyotype and adult thymus cells collected from female rF1 mouse were hybridized, and the GFP positive hybrid clones were selected by HAT medium. After 3 days from Cre treatment, chromosome painting analysis in all the hybrid cells showed that 2.2% (5 / 227) of cells had only two 6th chromosome. This shows elimination of both CEC tagged 6th chromosomes derived from ES cell (Fig. 11c). However, after 7 days from Cre treatment, the GFP negative cells were classified by FACS and amplified to the clone derived from single cell. Nineteen out of 20 clones analyzed by chromosome painting had three 6th chromosomes in each nucleus (Fig. 11d). Clearly, one clone has metacentric chromosome and showing formation of isochromosome Robertsonian translocation by duplication of 6th chromosome (Fig. 11e). Unlike after 3 days from Cre treatment, the hybrid clone having two 6th chromosomes was not actually detected in the stage. It suggests that the genetic disequilibrium of gene dosage of tetraploid cell having two 6th chromosomes would interfere the cell survival and the cell proliferation. Three 6th chromosomes are derived from somatic cell (JF1) as determined by PCR analysis of 129 JF1 nonspecific DNA sequence polymorphism in proximal D6Mit183, center D6Mit102, and distance D6Mit114 (Figs. 11f and g).

In order to confirm this, both 6th chromosomes derived from ES were eliminated and the Nanog transcript was amplified by Rt-PCR. While exon specific sequence polymorphism gives the prduct derived from ES having digestion resistance by Fok1, the product derived from JF1 somatic cell is susceptibility (Hatano, S. et al., Pluripotential competence of cells associated with Nanog activity. Mech Dev 122, 67-79 (2005)). Both of 570bps band derived from ES and 326bps and 244bps band derived from somatic cell may be detected before Cre treatment, on the other hand, only the band derived from somatic cell is observed after Cre treatment (Fig. 11h). The similar situation was observed in STELLA / PGC7 gene (which was expressed in ES cell and first germ cell, and also located in 6th chromosome). Only somatic cell derived STELLA / PGC7 gene RT-PCR product could be detected from Cre treatment hybrid cell RNA which produced the HinfI digestive fragment of 141bps, 136bps, and 33bps (Fig. 11h). Therefore, both 6th chromosomes derived from ES were selectively eliminated from the hybrid cell nucleus. In Cre treatment hybrid cell, it is not clear whether or not third 6th chromosome is obtained from duplication of JF1 somatic cell 6th chromosome or division recombination between 6th chromosome derived from somatic cell near the centromere region and 6th chromosome derived from ES cell.

The important thing is that GFP negative hybrid cell defected 6th chromosome derived from ES had capability to survive in the undifferentiated state and was confirmed by positive immunohistochemistry staining for anti-NANOG antibody and anti-OCT4 antibody (Fig. 11i). Western blot hybridization analysis actually showed that the relative NANOG expression level in three separate Cre treatment hybrid clones was about 1.5 times as much as the level in ES cell (Fig. 11j). Then, it was confirmed that the epigenetic reprogramming of somatic cell derived Nanog gene of ES fusion induction was sufficiently to maintain the undifferentiated state of hybrid cell without contribution of ES derived Nanog.

The CEC method is the approach developed newly, in order to induce a large scale change of hybrid cell genome component through elimination of chromosome which the target is narrowed down. In regeneration medicine, the stem cell which is MHC-suited and personalized is desired strongly as a source of supply for producing the transplant which the possibility of immune rejection is reduced. Most of MHC classes 1 and class II genes form the cluster on human 6th chromosome and mouse 17th chromosome. In first example, the selective elimination of chromosome including ES derived MHC gene from ESx somatic cell hybrid cell can provide the source supply of individualized MHC compatibility hybrid cell without needing remedial cloning. Since diploid MHC personalization pluripotent stem cell is produced, both copies of ES derived MHC chromosome can replace somatic cell derived MHC chromosome in ES nucleus combining the chromosome elimination art and the micronucleus mediated sex chromosome transfer which narrowed down the target of present inventors. Finally, the present inventors predict that the CEC method of present inventors enables production of same strain pluripotent stem cell as an individual from individual's own somatic cell through elimination of all the ES-derived chromosomes. Clearly, elimination of chromosome which the target is narrowed down can be applied to various biomedical study. Production of human embryonic stem cell having the heritable variation from patients understands the cause of human disease, and brings about the method of developing suitable medicines according to the pharmacological evaluation which is used mutated ES-derived cell.

### (Conclusion)

The human and mouse embryonic stem cells (ES cell) can provide pluripotency to adult somatic nucleus in ES somatic cell hybrid cell by the present invention. It is a powerful tool for creating reprogramming pluripotent stem cell from somatic cell without using the material of embryo. Thereby, the target chromosome is eliminated from the hybrid cell and great contribution is provided to the progress of medicine. Present inventors produced the universal chromosome elimination cassette (CEC) towards the purpose. In the present embodiment, use of CEC for target elimination of both copies of chromosomes 6 derived from ES is disclosed from the hybrid cell having an important pluripotency related gene including Nanog. The expression of Nanog from reprogrammed somatic nucleus was demonstrated by the hybrid cell from having maintained pluripotency after that. By eliminating ES derived MHC chromosome from hybrid cell or replacing ES derived MHC chromosome to somatic cell derived MHC chromosome in the present invention of present inventors, it became possible to produce the individual specific pluripotent stem cell for the stem cell treatment. Ultimately, all the ES derived chromosome may be eliminated from hybrid cell. Also it is important in the point that the pluripotent stem cell derived from patients having various mutation can be produced, and the cause and recovery of human disease can be examined by normal target elimination of ES chromosome.

ES cell has nuclear reprogramming capability. Reprogramming capability is the capability to provide pluripotency to somatic cell origin by cell fusion technique, in the treatment of mouse and human. This approach, which the chromosome derived from ES cell can be eliminated from hybrid cell, is very useful.

The chromosome in which tetrasomy was found can be restored to normal by using any methods of commonly known in the field. Cre-lox system is also included in this.

### (Embodiment 9: elimination of a plurality or all of chromosome)

According to the example described in the Embodiments 1-8, other chromosomesit can be eliminated. A plurality of chromosomes can be eliminated by repeating this.

Furthermore, all of chromosome to eliminate (for example, all chromosomes derived from ES cell) can be eliminated by repeating these.

These elimination can design concretely based on the description in view of well known art on the present application specifications for the person skilled in the art, by the information about the chromosome of ES cell which should be eliminated. Concretely, it performs as follows.
1. The CEC or basic principle and basic design introduces the altered CEC which is applied the concerned CEC into all the chromosomes of ES cell before fusion. Introduction is confirmed in Southern blot analysis or FISH analysis.
2. GFP or other marker genes (M) is introduced into somatic cells of patient by viral vector (M-somatic cell).
3. The cell fusion between the CECES cell and M-somatic cell are performed. The fused cell is selected from the marker gene derived from somatic cell.
4. The fused cell is treated by Cre (or other homologous recombination inducible enzyme).
5. The fused cell eliminated all CECs, which is introduced into ES cell is selected.
6. Elimination of all the chromosomes derived from ES cell is confirmed in genome PCR analysis or Southern blot analysis by using karyotype analysis and polymorphisms of the specific genome sequence for each chromosome in ES cell.
7. The produced stem cell (diploid) corresponding to an individual is induced differentiation to the required cell of patient and used for transplantation medical treatment.

As mentioned above, the cell from which all desired chromosomes were eliminated can be produced and applied medically.

### (Embodiment 10: chromosome elimination by human)

The similar experiment as Embodiment 8 can be performed in a human cell. The protocol is described below.

### (Reagent)

G418 (10 mg/ml) solution: 1 g of G418 (Geneticin, 860-1811IU, GIBCO) is dissolved in 100 ml of serum-free culture solution (DMEM) containing 100 µM HEPES, adjusted the pH to 7.1, then sterilized by filtration with 0.22 µm filter, and kept at 20 °C. When making selective culture solution, it dilutes and uses.
- PEG (1:1.4) solution (adjustment as required): 5 g of polyethylene glycol (PEG-1000, U218-07, BAKER) is dissolved in 6 ml of serum-free culture solutions (DMEM), finally added 1 ml of dimethyl sulfoxide (DMSO), and sterilized by filtration with 0.22 µm filter.
- PEG (1:3) solution (adjustment as required): 5 g of polyethylene glycol (PEG-1000) is dissolved in 15 ml of serum-free culture solutions (DMEM), and sterilized by filtration with 0.22 µm filter.
- Ouabain (1 x 10⁻³ M) solution: 36.4 mg hydrate ouabain is dissolve in 50 ml of serum-free culture solutions (DMEM) warmed preliminarily, dispensed after sterilizing by filtration with 0.22 µm filter, and kept at 20 °C. When making selective culture solution, it dilutes (final concentration 1 x 10⁻⁵ M) and uses.
- Colcemid (1mg/ml) solution: 1 mg of colcemids (DEMECOLCINE, D-7385, SIGMA) is dissolved in 1 ml of distilled water, and kept at 4 °C. The solution is diluted and the medium (20% FBS, DMEM) containing colcemid (0.05 µg/ml) is adjusted.
- Cytochalasin-B (10 µg/ml) solution: 10 mg of Cytochalasin-B (c-6762, SIGMA) is dissolved in 1 ml of DMSO, dissolved in 1000 ml of serum-free culture solutions (DMEM), and kept at 4 °C after sterilizing with 0.22 µm filter. In addition, the solution can be used repeatedly.
- PHA-P (1 mg/ml) solution: 10 mg of Phytohemagglutinin-P (311056, DIFCO) dissolved in 10 ml of distilled water, and kept at -20 °C after sterilizing with 0.22 µm filter. The solution is diluted and the serum-free culture solution (DMEM) containing PHA-P (50 µg/ml) is prepared.

### (Cell)

- Normal human fibroblast (NTI-4, JCRBO220 and MRC-5, ATCCCCL171): it is cultured in DMEM containing 10% FBS.
- Human cell derived from the diseased individual: it is cultured similarly in DMEM containing 10% FBS or cultured in other suitable media.

### (ES cell)

- Human ES cell: the cell established as following can be used.

Establishment of ES cell strain is performed by using feeder cells. Normally, ES cell is established by culturing the inner cell mass (ICM) separated from the blastocyst on feeder cells. Normally, as feeder cells, mouse fetus fibroblast or cell strain STO derived from it is used.

Preparation of human ES cell is performed with maintaining information as secrets completely after reaching donor's agreement. Concretely, it is performed carefully in the certified institution as described in the other position of the present specification. The experiment shown below is conducted in Institute for Frontier Medical Science, Kyoto University.

First, the general procedure is as follows. The embryo which become the source of ES cell is obtained and frozen. Then, frozen embryo is thawed and cultured to blastocyst stage. Next, inner cell mass is separated and cell strain is established. By the presence of stem cell marker expression and chromosome calibration, it is confirmed that it is stem cell, especially that it is ES cell. In order to confirm that it is ES cell, the detection of stem cell marker (alkaline phosphatase activity, specific antigen, Oct3/4, Nanog, or the like) is performed. Karyotype analysis is performed and is calibrated whether chromosome number and the form is normal. Then, the differentiation potency is calibrated. In order to calibrate the differentiation potency under cultivation, changes in culture condition, induction of cell differentiation by preparing the cell agglutination mass, and the differentiation potency to various functional cells are analyzed. Transplantation to the immune disorder animal or the like is performed and the tissue differentiation capacity by teratoma formation is analyzed.

Concretely, it is performed as follows.

### (Medium for ES cells)

Medium composition: (also called CMK medium in the present specification)

| | |
|---|---|
| DMEM / F12 (SigmaD-6421) | 80 ml |
| Non-essential amino acid (Gibco) | 0.8 ml |
| 200 mM L-glutamine | 1 ml |
| KSR(Gibco) | 20 ml |
| 2-mercaptoethanol | 0.8 µl |
| Human leukemia inhibitory factor (huLIF) (10 µg/ml) | 100 µl (10 ng/ml) |
| Basic fibroblast growth factors (bFGF) (1 mg/ml) | 0.4 µl (4 ng/ml) |

### (Cell dissociation solution)

0.25% trypsin (phosphate buffered saline (PBS))
1mM CaCl₂
20% KSR.

### (Dish)

The cultivation dish for producing feeder cells was coated by gelatin preliminarily. It was coated in 0.1% gelatin solution (swine skin, Type A: Sigma) and incubated at 37 °C for 1 hour or more. The suspension of cells is added after removing the gelatin solution on the cultivation dish. Several hours later, it can be used as feeder cells.

Establishment of ES cell by using feeder cells was performed as follows.

1. The medium for the feeder cells was changed to the medium for ES cells.

2. The inner cell mass was separated from the blastocyst by immunosurgery method or mechanical operation. Immunosurgery method is described briefly as follows.

After removing zona pellucida by enzyme treatment or the like, it is incubated in the antibody solution reacted to the surface antigen. Next, by incubating in complement solution, the trophectoderm which was the outermost layer cell was removed and the inner cell mass was separated.

In the case of human ES cell, the above mentioned procedure is conducted with careful attention to use the antibody solution reacted to the human cell surface antigen.

3. The separated inner cell mass was cultured on feeder cells.

4. It was confirmed that the inner cell mass had adhered to feeder cells on the next day, and medium was changed. Medium was changed every day thereafter.

5. The cell like ES cell which was proliferated from the inner cell mass after 5-10 days came to be recognized. Passage was performed when the cell mass became 100-200 µm.

6. The medium was removed and the cell dissociation solution was poured.

7. Since ES cell-like cell stripped from the cultivation dish when treated for 5-10 minutes, the cell mass was divided into several pieces by picking up with glass tube pulled thinly and by taking out and putting in several times.

8. The cell dissociation solution was washed and eliminated in the medium, and moved the cell mass on new feeder.

9. The culture medium was changed on the next day. Medium was changed every day thereafter.

10. After confirming that each cell mass was increased to about 100-200 µm on feeder, passage was performed by similar operation.

11. When the cell came to increase sufficiently (to the extent that it was increased over one 35 mm cultivation dish), passage can be performed like normal passage operation of monkey ES cell. After reaching the stage, it is confirmed that the stabilized cultivation is possible thereafter.

It is confirmed that the cell established in the embodiment is stem cell.

In the present embodiment, although DMEM / F12 is illustrated as basal medium, DMEM or other basal media can be shown to use without problems.

### (Staining by marker)

Next, the undifferentiated state of established cell was investigated by staining with a specific marker. ES cell is fixed by 4% PFA and immunostaining is performed in accordance with the immunostaining method of standard cultured cell (Willingham, M. C. et al., 1985. An Atlas of Immunoflorescence in Cultured Cell, Academic Press, Orlando, FL, pp. 1-13.). Blocking is performed by using 0.1% TritonX / PBS /2% skim milk at room temperature for 1 hour, and washing is performed by using TritonX / PBS 0.1 % at room temperature 4 times for 5 minutes each. The primary antibody is used 200 µg/ml monoclonal antibody of SSEA-4 and TRA-1-60 (CLONTECH) diluted to 1/100, and the second antibody is used what diluted FITC labeled goat anti-mouse IgG (H+L) (ZY MEDLABORATORIES, INC) diluted to 1/200. After the reaction of second antibody, staining in order of rhodaminephalloidin (MolecularProbe) and DAPI (SIGMA) is performed and the signal is detected.

Histochemical staining of ALP (alkaline phosphatase) was performed as follows. After washing the cultivation dish twice by Dulbecco's PBS(-), fixation is performed by using 95% cold ethanol for 30 minutes or more at 4 °C, subsequently dehydration was performed by using dehydrataed ethanol for 30 minutes or more at 4 °C. After removing the fixation solution and washing the dish with 0.1 M Tris-HCI (pH 9.0-9.5) 3 times for 5 minutes each at room temperature, 1.5-2ml staining solution (2.5 mg of naphthol AS-BI phosphate (Sigma, catalog number N-2125), 15 mg of first red TR salt (Sigma) [or 6 mg of first purple B salt (Sigma) and 12.5 mg of first blue BB salt (Sigma)] is added to 25 ml of 0.1 M Tris-HCI buffer solution, dissolved by stirring for 3-5 minutes under light shielding, and subsequently filtrated) is added to each dish and staining is performed by using ALP at room temperature for 15-30 minutes under light shielding. After washing by PBS(-) twice, glycerol is added and the speculum of ES cell colony stained red-brown color is performed by the phase contrast stereomicroscope.

Thus, it is confirmed that established stem cell maintains pluripotency as usual.
[Apparatus and instrument]
- High speed refrigerated centrifuge machine (Hitachi SCR20B)
- Rotor (Hitachi RPR9-2)
- Adapter made by rubber (Hitachi 300A500)
- Flask for centrifuge machine (Coaster 3025)
- Container for centrifuge machine made by acrylics (custom-made item, Ikemoto Rika)
- Filter for purification of micronucleus cell: holder made from Swinnex 25 mm MILLIPORE
and filter (8 µm, 5 µm, 3 µm; SN110614, SN-110613, and SN110612, NUCLEPORE) is set, respectively, and autoclave sterilization is performed.

### 3. Operation

1) The plasmid DNA transfection to normal human fibroblast, and separation of G418 resistance clone
   (1) The normal human fibroblast (2.5 x 10⁵ cells / flask) distributed by trypsinization two days before transfection is planted in four cultivation flasks (25 cm²).
   (2) It is changed to new culture medium (10% FBS, DMEM) 4 hours before transfection.
   (3) The calcium phosphate-DNA precipitation solution (2.5 ml) containing chromosome elimination cassette NA (25 µg DNA) which is made in Embodiment 1 is prepared, and 0.5 ml (5 µg DNA) of precipitation solutions made uniformly by pipetting are added to the cultivation flask, diffused gently and cultured in CO₂ incubator for 4 hours.
   (4) Cell surface is washed with serum-free culture solution (DMEM), and considered to treat with 1.5 ml of 15% glycerol/HBS solution for 1 minute.
   (5) Cell surface is washed with serum-free culture solution (DMEM), and cultured with the usual culture medium (10% FBS, DMEM) for two days.
   (6) Cells are dispersed by trypsinization and the cells (1x10⁵ cells/plate) suspended in selective culture solution (10% FBS, DMEM) containing G418 (400 µg-ml) is planted in 60 plastic petri dishes (diameter 100 mm), and selective culture is performed for about 3 weeks (culture medium is changed every 2-3 days).
   (7) The colonies which are consisted of G418 resistance cell appeared on each petri dish were cloned.
2) Cell fusion between G418 resistance human cell and mouse A9 cell, and separation of hybrid cells
   (1) Cells are dispersed by trypsinization, and each cell (1x10⁶ cells/each) suspended in culture medium (10% FBS, DMEM) is planted simultaneously in cultivation flask (25cm²) and cultured for one day.
   (2) After washing cell surface twice with PBS solution, 3 ml of PEG (1:1.4) solution is treated for 1 minute and is further changed to 3 ml of PEG (1:3) solution and treated for 1 minute.
   (3) After draining PEG solution and washing 3 times with serum-free culture solution (DMEM), it is cultured with the usual culture medium (10% FBS, DMEM) for one day.
   (4) Cells are diffused by trypsinization, the suspended cells (2 x 10⁵ cells/plate) in double selective culture solution (10% FBS, DMEM) containing ouabain (1 x 10⁻⁵ M) and G418 (800 µg/ml) is planted in plastic petri dish (diameter 100 mm), and selective culture is performed for about 3 weeks (culture medium is changed every 2-3 days).
   (5) At least one hybrid cell clone is separated from each combination (NTI-4 cell or MRC-5 cell).
3) Micronucleus cell separation from hybrid cell
   (1) Cells separated the micronucleus are cultured to the state of 80%-90% saturation in cultivation flask (75 cm²).
   (2) Cells are dispersed by trypsinization, suspended in 7 ml of selective culture solutions (10% FBS, DMEM) containing G418 (800 µg/ml), distributed 1 ml of cell suspension to each six flasks for centrifugation machine (25 cm²), added 4 ml of selective culture solution, and cultured for two days. In addition, the cell density before colcemid treatment is preferred 70-80% saturation degree and therefore it may not be always required the cultivation for two days.
   (3) The culture medium (20% FBS, DMEM) containing colcemid (0.05 µg/ml) is changed and cultured for more two days (micronucleus is formed).
   (4) Culture medium is removed and cytochalasin-B (10 µg/ml) solution which kept warm (37 °C) preliminary is filled almost to the limit of centrifugation flask.
   (5) The flask for centrifuge machine is inserted in the container for centrifuge machine made by acrylics, equiped the adapter made by rubber after warm water (34 °C) pouring (degree which does not exceed the water surface of the flask for centrifugation machine), and centrifugation is performed at 34 °C, 8000 rpm, and for 1 hour.
   (6) After removing the cytochalasin-B solution after centrifugal separation, the micronucleus cells located in the corner of flask for centrifugation machine are collected after suspending in 1 ml of serum free culture solution (DMEM), further, washed with 1 ml of serum free culture solution, and combined with the first micronucleus cell suspension (total about 12 ml).
   (7) The suspension is through the filter in order of 8 µm, 5 µm, and 3 µm and micronucleus cells are purified. Normally, one piece of 8 µm filters, 2 pieces of 5 µm, and 1 piece of 3 µm are used.

4) Micronucleus hybrid cell formation with micronucleus cell and the other kind cell
   (1) The other kind cell (for example, cells other than ES cell) is cultured to the state of 80% saturation with the cultivation flask (25cm²).
   (2) The purified micronucleus cell suspension is resuspended in 2 ml of serum free culture solutions (DMEM) containing PHA-P (50 µg/ml) after centrifugal separation at 1,500 rpm for 5 minutes.
   (3) During centrifugal separation, the resuspended minute cell scattered calmly after washing twice with serum free culture solution (DMEM) on cell surface, and settled into CO₂ incubator for 15 minutes (37 °C).
   (4) The micronucleus cell suspension is drained and 3 ml of PEG (1:1. 4) solution is added and treated for exactly 1 minute. Since toxicity of PEG is strong to cells, the treatment time is required to perform correctly here.
   (5) After removing PEG solution, it is washed at least 3 times with serum free culture solution (DMEM) and usually cultured for one day with culture medium (10% FBS, DMEM).
   (6) Cells are diffused by trypsinization, the suspended cells in selective culture solution (DMEM) containing G418 (800 µg/ml) is planted in 3 plastic petri dishes (diameter 100 mm), and selective culture is performed for about 3 weeks (culture medium is changed every 2-3 days).
   (7) The colonies which are consisted of G418 resistance cell appeared on each petri dish were cloned.
5) Human chromosome identification of micronucleus hybrid cell: chromosome analysis is performed with quinacrine-Hoechst combined staining method and it is confirmed that the normal human chromosome is included in the selected G418 resistance other kind cell clone. Chromosome in situ hybridization method is performed and the label site of chromosome elimination cassette on existing human chromosome is identified.

### (Chromosome elimination)

The chromosome elimination cassette is inseted on a desired chromosome by using introduced ES cell by homologous recombination. Subsequently, it is inserted on second locus by using chromosome elimination cassette. ES cell which CEC was introduced into both loci of the desired gene is obtained.

### 4. FISH analysis

ES cell having CEC in each chromosome is identified, and the library is produced.

ES cell introduced CEC homozygously at the desired locus of desired chromosome and somatic cell are fused. The fused cell is treated with pCMV-Cre and the clone from which the desired chromosome derived from ES cell was eliminated is obtained. By the genetic manipulation ES cell being induced differentiation to various tissues in in vivo or in vitro and conducted replanting experiment, the presence and the degree of rejection response is calibrated.

### (Selection of G418 high concentration drug resistance clone (protocol for homozygous cell selection))

The transgenic ES cell introduced Pgk-neo cassette is selected by 250 µg/ml G4.
2. G418 resistance ES cells (5x10⁶ pieces) are unleashed to culture dish of 3cm in diameter (6-well plate), and are cultured for three days under 5-10 mg/ml high concentration G418. Selection media are changed every day during this period. They are subcultured to 3 pieces of 3cm culture dish after three day from starting selective culture, and are cultured for 4-7 days under 5-10 mg/ml high concentration G418. Selection media are changed every day during this period.
4. The colony of high concentration G418 resistance ES cells appears about 40.
5. DNA from each colony is extracted and the clone which has Pgk-neo cassette in homozygous is selected by genome PCR method or Southern hybridization method.

### (4. Results and Discussion)

In case of human cell, although it is quite complicated procedure as described above, if it is performed with carefully observing the state of cell, the fused cell and the chromosomal introduction are comparatively easy. However, there are four points for the experimental method. (1) Since the toxicity of polyethylene glycol used for fusion is different between manufacturers or lots, a lot check is performed and the less toxic is used. (2) Since the susceptibility to phytohemagglutinin-P (PHA-P) changes with cells, adjustment is performed by decreasing PHA-P concentration or without using at all, or the like, if needed. (3) By the leak in the filter used for purification of micronucleus cells, parent cell may be mixed. Therefore, a part of purified micronucleus cells are scattered over the plate and the presence of mixing of parent cell is confirmed. (4) Since chromosome is not always introduced in the perfect state, chromosome analysis is essential.

Actually the experiment mentioned above is performed repeatedly, and finally a sufficient number of cells are separated. By searching the existence of human chromosome in chromosome analysis, it can be confirmed that each one normal human chromosome is included in the clone.

Thus, the fused cell between the ES cell eliminated one side copy or both copies of desired chromosome and the desired cell can be obtained.

### (Embodiment 11: example of chromosomal exchange)

The experimental procedure described in Embodiments 1-10 can be applied and chromosomal recombination cell can be produced. The detailed procedure is shown below.

The procedure is that the chromosome of somatic cell (two copies or one copy) is fused with ES cell, and the same ES cell derived chromosome as the inserted chromosome is eliminated by cassette.
The detailed procedure is shown below.
1. Each different drug resistance gene is introduced into two MHC chromosomes derived from somatic cell (paternity and maternity).
2. The cell fusion between the somatic cell and the A9 cell is performed. The micronucleus containing only MHC chromosome is produced by applying the above mentioned micronucleus making method to fused cell.
3. ES cell produces MHC-CECES cell in which CEC (including the marker of GFP or HSV-TK gene) is introduced into two MHC chromosomes.
4. The cell fusion is performed between MHC-CECES cell and MHC micronucleus derived from somatic cell. The minute nuclear fused MHC-CECES cell which MHC chromosome derived frmo somatic cell is introduced is selected by drug selection.
5. Cre treatment is performed in the minute nuclear fused MHC-CECES cell which MHC chromosome derived from somatic cell is introduced. MHC chromosome derived from ES cell is eliminated by FACS sorting of GFP negative cells or drug selection of ganciclovir resistance cell, and the diploid ES cell corresponding to the MHC individual having MHC chromosome derived from somatic cell is selected (MHC chromosomal recombinant stem cell).

6. The produced stem cell (diploid) corresponding to an individual is induced differentiation to the required cell of patient and used for transplantation medical treatment.

As mentioned above, the chromosomal recombinant cell can be produced and applied medically.

### (Embodiment 12: Example of regenerative medical treatment)

The fused cell after chromosome elimination obtained in the above mentioned embodiment can be differentiated to nerve cell and can be transplanted to living brain. The following conditions are used for the differentiation to nerve cell. The undifferentiated cell was washed in serum free medium 3 times and the serum was removed completely.
By using the medium added knockout serum substitution additive KSR (Knockout Serum Replacement; GIBCO/Invitrogen Cat. No. 10828-028) to ES cell medium instead of bovine serum, undifferentiated cell is cultured for8-11 days on PA6 feeder cell (refer to Kawasaki et al., 2000, Neuron 28; 31-40, Tada et al., 2003; Dev. Dyn., 227; 504-510). Thereby, it can be differentiated to nerve cell.

As mentioned above, although the present invention has been illustrated by using the preferable embodiment of the present invention, the present invention should not interpreted as limited to the embodiment. It is understood that the present invention should be interpreted as the scope of only the claims. It is understood that the person skilled in the art can implement the equivalent scope based on the description in the present invention and common general technical knowledge from the description of detailed preferable embodiment in the present invention. It is understood that the patent, patent application, and literature quoted in the present specification should be cited the contents for reference to the present specification as the contents themselves being concretely described in the present specification.

### Industrial Applicability

By using DNA kit for eliminating a desired chromosome, cassette (as a genetic engineering tool), the cell from which the desired chromosome was eliminated, the library, and eliminating a desired chromosome (especially MHC), tailor made medical treatment to individual patients becomes possible by introducing a required gene if needed.

## Claims

1. A gene cassette including a recombinase recognition sequence for one, or two in the opposite direction, wherein a sequence coding a marker gene is included therein.

2. The gene cassette according to claim 1, wherein said marker gene is a homologous recombination sequence including a sequence selected from the group consisting of the loxP sequence, the FRT site, the attB sequence, the attP sequence and the res site sequence.

3. The gene cassette according to claim 1, wherein said marker gene includes in the gene selected from the group consisting of the green fluorescence protein (GFP) gene, the yellow fluorescence protein (YFP) gene, the cyanogen fluorescence protein (CFP) gene, and the red fluorescence protein (dsRED) gene.

4. The gene cassette according to claim 1, wherein the gene cassette further includes a selection sequence.

5. The gene cassette according to claim 4, wherein said selection sequence includes in the sequence coded a selected gene from the group consisting of the thymidine kinase (TK) gene, the puromycin resistance gene, the neomycin resistance gene, the hygromycin resistance gene, the blasticidin resistance gene, the zeosin resistance gene, the HAT resistance gene, the diphtheria toxin resistance gene, and the fluorouracil resistance gene.

6. The gene cassette according to claim 1, wherein said recombinase recognition sequence includes the sequence of ATAACTTCGTATAATGTATGCTATACGAAGTTAT, which is described in sequence number 1.

7. The gene cassette according to claim 1, wherein the gene cassette further includes the IRES sequence, which is described in sequence number 2.

8. The gene cassette according to claim 1, wherein the gene cassette further includes the CAG sequence, which is described in sequence number 3.

9. The gene cassette according to claim 1, wherein said recombinase recognition sequence includes two in the opposite direction.

10. The gene cassette according to claim 1, wherein said recombinase recognition sequence is included two in the opposite direction and includes in the CAG sequence, the marker sequence, the IRES sequence, and the selection sequence in between the recombinase recognition sequences.

11. The gene cassette including a recombinase recognition sequence for one, or two in the opposite direction, wherein a composition for the elimination of the desired chromosome includes the sequence coding a marker gene and includes the gene cassette.

12. The composition according to claim 11, wherein said chromosome includes a pluripotency related gene.

13. The composition according to claim 11, wherein said elimination includes the removal of both copies in said chromosomes.

14. A kit for the elimination of a desired chromosome, which comprises
A) a gene cassette including a recombinase recognition sequence for one, or two in the opposite direction, wherein the gene cassette includes a sequence coding a marker gene; and
B) a nucleic acid molecule which is recombinase corresponding to the recombinase recognition sequence or nucleic acid molecules including the nucleic acid sequences coding the recombinase recognition sequence.

15. A kit for the elimination of a desired chromosome from a stem cell, which comprises
A) a gene cassette including the recombinase recognition sequence for one, or two in the opposite direction, wherein the gene cassette includes a sequence coding a marker gene;
B) a nucleic acid molecule which is recombinase corresponding to the recombinase recognition sequence or nucleic acid molecules including the nucleic acid sequences coding the recombinase recognition sequence; and
C) a stem cell.

16. A kit for providing a cell with pluripotency by the elimination of a desired chromosome from a cell having the desired genome, the chromosome, or the group of chromosomes, which comprises
A) a gene cassette including the recombinase recognition sequence for one, or two in the opposite direction, wherein the gene cassette includes a sequence coding a marker gene;
B) a nucleic acid molecule which is recombinase corresponding to the recombinase recognition sequence or nucleic acid molecules including the nucleic acid sequences coding the recombinase recognition sequence; and
C) a stem cell; and
D) a cell having the desired genome, the chromosome, or the group of chromosomes.

17. A method for providing a cell with pluripotency by the elimination of a desired chromosome or a group of chromosomes from a cell having the desired genome, which comprises
A) a step providing a gene cassette including the recombinase recognition sequence for one, or two in the opposite direction including a sequence coding a marker gene;
B) a step of introducing the gene cassette to a stem cell;
C) a step of fusing between the stem cell and the desired genome, the chromosome, or the group of chromosomes;
D) a step of providing a recombinase recognizing the recombinase recognition sequence or nucleic acid molecules including nucleic acid sequences coding the recombinase recognition sequence;
E) a step of exposing the fused cell in the condition that recombination occurs; and
F) a step of observing a signal derived from the marker gene in the fused cell and selecting the cell having eliminated the desired chromosome.

18. The cell made by the method according to claim 17.

19. A cell in which is eliminated the desired chromosome.

20. A stem cell in which is eliminated the desired chromosome.

21. A stem cell according to claim 20, wherein said stem cell is the ES cell.

22. The cell according to claim 19, wherein said chromosome includes the sequence coding the MHC or the corresponding gene.

23. A chromosome recombinant cell, wherein the desired chromosome or the group of chromosomes is modified.

24. A chromosome recombinant stem cell, wherein the desired chromosome or the group of chromosomes is modified.

25. The stem cell according to claim 24, wherein said stem cell is the ES cell.

26. A chromosome recombinant cell of a fused cell between the ES cell and the micronucleus having a desired genome, a chromosome or a group of chromosomes, having a pluripotency and having eliminated the desired chromosome derived from the ES cell from the fused cell.

27. A fused cell between an ES cell and a cell having a desired genome, a chromosome or a group of chromosomes, wherein the fused cell provides a pluripotency and has eliminated a desired chromosome from the fused cell.

28. A fused cell between an ES cell and a cell having a desired genome, a chromosome or a group of chromosomes, wherein the fused cell provides a pluripotency and has eliminated the desired chromosome of the ES cell from the fused cell.

29. A fused cell, wherein said desired chromosome is a chromosome including a sequence coding a MHC of an ES cell, the rejection response related gene, or the corresponding gene.

30. A fused cell, wherein said desired chromosome is the chromosome being selected from a group of the 6^{th} chromosome, the 11^{th} chromosome, the 12^{th} chromosome, and the 17^{th} chromosome of mouse and the 6^{th} chromosome of human or the chromosome including one copy or both copies of the chromosome(s) corresponding to the chromosome(s).

31. A fused cell, wherein said desired chromosome is the chromosome including one or more than two chromosome(s) of an ES cell.

32. A fused cell, wherein said desired chromosomes are all the chromosomes of the ES cell.

33. A fused cell, wherein a cell having said desired genome, a chromosome, or a group of chromosomes is a cell having the genome, chromosome, or a group of chromosomes derived from a diseased individual.

34. A fused cell, wherein a cell having said desired genome, a chromosome, or a group of chromosomes is a cell having the genome, a chromosome, or a group of chromosome derived from a diseased individual and said desired chromosomes are all the chromosomes in the ES cell.

35. A cell library, wherein the cell library includes a plurality of cell types having eliminated a desired chromosome.

36. A use of a gene cassette including a recombinase recognition sequence for one, or two in the opposite direction, for elimination of a desired chromosome wherein the gene cassette includes a sequence coding a marker gene.

37. A use of a gene cassette including a recombinase recognition sequence for one, or two in the opposite direction, for producing elimination of a desired chromosome wherein the gene cassette includes a sequence coding a marker gene.

38. A method for the elimination of a desired chromosome from a cell including a desired genome, a chromosome, or a group of chromosomes, which comprises
A) a step of providing a gene cassette including a recombinase recognition sequence for one, or two in the opposite direction, wherein the gene cassette includes a sequence coding a marker gene;
B) a step of introducing the gene cassette to the stem cell;
C) a step of fusing between the cell and the cell having the desired genome, the chromosome, or the group of chromosomes;
D) a step of providing the fused cell with the recombinase recognizing the recombinase recognition sequence or nucleic acid molecules including the nucleic acid sequences coding the recombinase recognition sequence;
E) a step of exposing the fused cell in the condition that recombination occurs; and
F) a step of observing the signal derived from the marker gene of the fused cell and selecting the cell having eliminated the desired chromosome.

39. A method for producting a chromosome recombinant cell, comprising:
A) a step of providing a gene cassette including a recombinase recognition sequence for one, or two in the opposite direction, wherein the gene cassette includes a sequence coding a marker gene;
B) a step of introducing the gene cassette to a stem cell;
C) a step of fusing between the cytoplasm of the second cell having the desired chromosome and the first cell;
D) a step of providing a recombinase recognizing the recombinase recognition sequence or a nucleic acid molecules including the nucleic acid sequences coding the recombinase recognition sequence so that can be eliminated the chromosome in the chromosomes of the first cell corresponding to the desired chromosome of the second cell;
E) a step of exposing the fused cell in the condition that recombination occurs; and
F) a step of observing the signal derived from the marker gene of the fused cell and selecting the cell having eliminated the desired chromosome.

40. The cell which is produced by the method according to claim 38 or 39.


SEQUENCE LISTING
<110> KYOTO UNIVERSITY
ReproCELL, Inc
TADA, Takashi
NAKATSUJI, Norio
MATSUMURA, Hiroyuki
TADA, Masako

<120> A METHOD FOR REMOVING A DESIRED CHROMOSOME AND A TAILOR-MADE TREATMENT USING THE SAME

<130> RC006PCT

<150> JP 2005-006859
<151> 2005-01-13

<160> 25

<170> PatentIn version 3.3

<210> 1
<211> 34
<212> DNA
<213> Artificial

<220>
<223> loxP sequence

<400> 1
ataacttcgt ataatgtatg ctatacgaag ttat 34

<210> 2
<211> 581
<212> DNA
<213> Artificial

<220>
<223> IRES sequence

<400> 2
aattccgccc ctctccctcc ccccccccta acgttactgg ccgaagccgc ttggaataag 60
gccggtgtgc gtttgtctat atgtgatttt ccaccatatt gccgtctttt ggcaatgtga 120
gggcccggaa acctggccct gtcttcttga cgagcattcc taggggtctt tcccctctcg 180
ccaaaggaat gcaaggtctg ttgaatgtcg tgaaggaagc agttcctctg gaagcttctt 240
gaagacaaac aacgtctgta gcgacccttt gcaggcagcg gaacccccca cctggcgaca 300
ggtgcctctg cggccaaaag ccacgtgtat aagatacacc tgcaaaggcg gcacaacccc 360
agtgccacgt tgtgagttgg atagttgtgg aaagagtcaa atggctctcc tcaagcgtat 420
tcaacaaggg gctgaaggat gcccagaagg taccccattg tatgggatct gatctggggc 480
ctcggtgcac atgctttaca tgtgtttagt cgaggttaaa aaaacgtcta ggccccccga 540
accacgggga cgtggttttc ctttgaaaaa cacgatgata a 581

<210> 3
<211> 2280
<212> DNA
<213> Artificial

<220>
<223> CAG sequence

<400> 3
gtcgacattg attattgact agttattaat agtaatcaat tacggggtca ttagttcata 60
gcccatatat ggagttccgc gttacataac ttacggtaaa tggcccgcct ggctgaccgc 120
ccaacgaccc ccgcccattg acgtcaataa tgacgtatgt tcccatagta acgccaatag 180
ggactttcca ttgacgtcaa tgggtggact atttacggta aactgcccac ttggcagtac 240
atcaagtgta tcatatgcca agtacgcccc ctattgacgt caatgacggt aaatggcccg 300
cctggcatta tgcccagtac atgaccttat gggactttcc tacttggcag tacatctacg 360
tattagtcat cgctattacc atgggtcgag gtgagcccca cgttctgctt cactctcccc 420
atctcccccc cctccccacc cccaattttg tatttattta ttttttaatt attttgtgca 480
gcgatggggg cggggggggg gggggcgcgc gccaggcggg gcggggcggg gcgaggggcg 540
gggcggggcg aggcggagag gtgcggcggc agccaatcag agcggcgcgc tccgaaagtt 600
tccttttatg gcgaggcggc ggcggcggcg gccctataaa aagcgaagcg cgcggcgggc 660
gggagtcgct gcgttgcctt cgccccgtgc cccgctccgc gccgcctcgc gccgcccgcc 720

ccggctctga ctgaccgcgt tactcccaca ggtgagcggg cgggacggcc cttctcctcc 780
gggctgtaat tagcgcttgg tttaatgacg gctcgtttct tttctgtggc tgcgtgaaag 840
ccttaaaggg ctccgggagg gccctttgtg cgggggggag cggctcgggg ggtgcgtgcg 900
tgtgtgtgtg cgtggggagc gccgcgtgcg gcccgcgctg cccggcggct gtgagcgctg 960
cgggcgcggc gcggggcttt gtgcgctccg cgtgtgcgcg aggggagcgc ggccgggggc 1020
ggtgccccgc ggtgcggggg ggctgcgagg ggaacaaagg ctgcgtgcgg ggtgtgtgcg 1080
tgggggggtg agcagggggt gtgggcgcgg cggtcgggct gtaacccccc cctgcacccc 1140
cctccccgag ttgctgagca cggcccggct tcgggtgcgg ggctccgtgc ggggcgtggc 1200
gcggggctcg ccgtgccggg cggggggtgg cggcaggtgg gggtgccggg cggggcgggg 1260
ccgcctcggg ccggggaggg ctcgggggag gggcgcggcg gccccggagc gccggcggct 1320
gtcgaggcgc ggcgagccgc agccattgcc ttttatggta atcgtgcgag agggcgcagg 1380
gacttccttt gtcccaaatc tggcggagcc gaaatctggg aggcgccgcc gcaccccctc 1440
tagcgggcgc gggcgaagcg gtgcggcgcc ggcaggaagg aaatgggcgg ggagggcctt 1500
cgtgcgtcgc cgcgccgccg tccccttctc catctccagc ctcggggctg ccgcaggggg 1560
acggctgcct tcggggggga cggggcaggg cggggttcgg cttctggcgt gtgaccggcg 1620
gctctagagc ctctgctaac catgttcatg ccttcttctt tttcctacag ctcctgggca 1680
acgtgctggt tgttgtgctg tctcatcatt ttggcaaaga attcctcgag gaattcactc 1740
ctcaggtgca ggctgcctat cagaaggtgg tggctggtgt ggccaatgcc ctggctcaca 1800
aataccactg agatcttttt ccctctgcca aaaattatgg ggacatcatg aagccccttg 1860
agcatctgac ttctggctaa taaaggaaat ttattttcat tgcaatagtg tgttggaatt 1920
ttttgtgtct ctcactcgga aggacatatg ggagggcaaa tcatttaaaa catcagaatg 1980
agtatttggt ttagagtttg gcaacatatg ccatatgctg gctgccatga acaaaggtgg 2040
ctataaagag gtcatcagta tatgaaacag ccccctgctg tccattcctt attccataga 2100
aaagccttga cttgaggtta gatttttttt atattttgtt ttgtgttatt tttttcttta 2160
acatccctaa aattttcctt acatgtttta ctagccagat ttttcctcct ctcctgacta 2220
ctcccagtca tagctgtccc tcttctctta tgaagatccc tcgacctgca gcccaagctt 2280

<210> 4
<211> 34
<212> DNA
<213> Artificial

<220>
<223> FRT sequence

<400> 4

gaagttccta ttctctagaa agtataggaa cttc 34

<210> 5
<211> 56
<212> DNA
<213> Artificial

<220>
<223> attB sequence

<400> 5
tcgagtgagg tggagtacgc gcccggggag cccaagggca cgccctggca cccgca 56

<210> 6
<211> 55
<212> DNA
<213> Artificial

<220>
<223> attP sequence

<400> 6
ctagacccta cgcccccaac tgagagaact caaaggttac cccagttggg gcacg 55

<210> 7
<211> 720
<212> DNA
<213> Artificial

<220>
<223> Green Fluorescent Protein

<220>
<221> CDS
<222> (1)..(720)

<400> 7
atg gtg agc aag ggc gag gag ctg ttc acc ggg gtg gtg ccc atc ctg 48
Met Val Ser Lys Gly Glu Glu Leu Phe Thr Gly Val Val Pro Ile Leu
1               5                   10                  15

gtc gag ctg gac ggc gac gta aac ggc cac aag ttc agc gtg tcc ggc 96
Val Glu Leu Asp Gly Asp Val Asn Gly His Lys Phe Ser Val Ser Gly
            20                  25                  30

gag ggc gag ggc gat gcc acc tac ggc aag ctg acc ctg aag ttc atc 144
Glu Gly Glu Gly Asp Ala Thr Tyr Gly Lys Leu Thr Leu Lys Phe Ile
        35                  40                  45

tgc acc acc ggc aag ctg ccc gtg ccc tgg ccc acc ctc gtg acc acc 192
Cys Thr Thr Gly Lys Leu Pro Val Pro Trp Pro Thr Leu Val Thr Thr
    50                  55                  60

ctg acc tac ggc gtg cag tgc ttc agc cgc tac ccc gac cac atg aag 240
Leu Thr Tyr Gly Val Gln Cys Phe Ser Arg Tyr Pro Asp His Met Lys
65                  70                  75                  80

cag cac gac ttc ttc aag tcc gcc atg ccc gaa ggc tac gtc cag gag 288
Gln His Asp Phe Phe Lys Ser Ala Met Pro Glu Gly Tyr Val Gln Glu
                85                  90                  95

cgc acc atc ttc ttc aag gac gac ggc aac tac aag acc cgc gcc gag 336
Arg Thr Ile Phe Phe Lys Asp Asp Gly Asn Tyr Lys Thr Arg Ala Glu
            100                 105                 110

gtg aag ttc gag ggc gac acc ctg gtg aac cgc atc gag ctg aag ggc 384
Val Lys Phe Glu Gly Asp Thr Leu Val Asn Arg Ile Glu Leu Lys Gly
        115                 120                 125

atc gac ttc aag gag gac ggc aac atc ctg ggg cac aag ctg gag tac 432
Ile Asp Phe Lys Glu Asp Gly Asn Ile Leu Gly His Lys Leu Glu Tyr
    130                 135                 140

aac tac aac agc cac aac gtc tat atc atg gcc gac aag cag aag aac 480
Asn Tyr Asn Ser His Asn Val Tyr Ile Met Ala Asp Lys Gln Lys Asn
145                 150                 155                 160

ggc atc aag gtg aac ttc aag atc cgc cac aac atc gag gac ggc agc 528
Gly Ile Lys Val Asn Phe Lys Ile Arg His Asn Ile Glu Asp Gly Ser
                165                 170                 175

gtg cag ctc gcc gac cac tac cag cag aac acc ccc atc ggc gac ggc 576
Val Gln Leu Ala Asp His Tyr Gln Gln Asn Thr Pro Ile Gly Asp Gly
            180                 185                 190

ccc gtg ctg ctg ccc gac aac cac tac ctg agc acc cag tcc gcc ctg 624
Pro Val Leu Leu Pro Asp Asn His Tyr Leu Ser Thr Gln Ser Ala Leu
        195                 200                 205

agc aaa gac ccc aac gag aag cgc gat cac atg gtc ctg ctg gag ttc 672
Ser Lys Asp Pro Asn Glu Lys Arg Asp His Met Val Leu Leu Glu Phe
    210                 215                 220

gtg acc gcc gcc ggg atc act ctc ggc atg gac gag ctg tac aag taa 720
Val Thr Ala Ala Gly Ile Thr Leu Gly Met Asp Glu Leu Tyr Lys
225                 230                 235

<210> 8
<211> 239
<212> PRT
<213> Artificial

<220>
<223> Synthetic Construct

<400> 8
Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210> 9
<211> 600
<212> DNA
<213> Artificial

<220>
<223> puromycin resistant gene

<220>
<221> CDS
<222> (1)..(600)

<400> 9
atg acc gag tac aag ccc acg gtg cgc ctc gcc acc cgc gac gac gtc 48
Met Thr Glu Tyr Lys Pro Thr Val Arg Leu Ala Thr Arg Asp Asp Val
1               5                   10                  15

ccc cgg gcc gta cgc acc ctc gcc gcc gcg ttc gcc gac tac ccc gcc 96
Pro Arg Ala Val Arg Thr Leu Ala Ala Ala Phe Ala Asp Tyr Pro Ala
            20                  25                  30

acg cgc cac acc gtc gac ccg gac cgc cac atc gag cgg gtc acc gag 144
Thr Arg His Thr Val Asp Pro Asp Arg His Ile Glu Arg Val Thr Glu
        35                  40                  45

ctg caa gaa ctc ttc ctc acg cgc gtc ggg ctc gac atc ggc aag gtg 192
Leu Gln Glu Leu Phe Leu Thr Arg Val Gly Leu Asp Ile Gly Lys Val
    50                  55                  60

tgg gtc gcg gac gac ggc gcc gcg gtg gcg gtc tgg acc acg ccg gag 240
Trp Val Ala Asp Asp Gly Ala Ala Val Ala Val Trp Thr Thr Pro Glu
65                    70                    75                    80

agc gtc gaa gcg ggg gcg gtg ttc gcc gag atc ggc ccg cgc atg gcc 288
Ser Val Glu Ala Gly Ala Val Phe Ala Glu Ile Gly Pro Arg Met Ala
                85                    90                    95

gag ttg agc ggt tcc cgg ctg gcc gcg cag caa cag atg gaa ggc ctc 336
Glu Leu Ser Gly Ser Arg Leu Ala Ala Gln Gln Gln Met Glu Gly Leu
            100                    105                    110

ctg gcg ccg cac cgg ccc aag gag ccc gcg tgg ttc ctg gcc acc gtc 384
Leu Ala Pro His Arg Pro Lys Glu Pro Ala Trp Phe Leu Ala Thr Val
        115                    120                    125

ggc gtc tcg ccc gac cac cag ggc aag ggt ctg ggc agc gcc gtc gtg 432
Gly Val Ser Pro Asp His Gln Gly Lys Gly Leu Gly Ser Ala Val Val
    130                    135                    140

ctc ccc gga gtg gag gcg gcc gag cgc gcc ggg gtg ccc gcc ttc ctg 480
Leu Pro Gly Val Glu Ala Ala Glu Arg Ala Gly Val Pro Ala Phe Leu
145                    150                    155                    160

gag acc tcc gcg ccc cgc aac ctc ccc ttc tac gag cgg ctc ggc ttc 528
Glu Thr Ser Ala Pro Arg Asn Leu Pro Phe Tyr Glu Arg Leu Gly Phe
                165                    170                    175

acc gtc acc gcc gac gtc gag tgc ccg aag gac cgc gcg acc tgg tgc 576
Thr Val Thr Ala Asp Val Glu Cys Pro Lys Asp Arg Ala Thr Trp Cys
            180                    185                    190

atg acc cgc aag ccc ggt gcc tga 600
Met Thr Arg Lys Pro Gly Ala
        195

<210> 10
<211> 199
<212> PRT
<213> Artificial

<220>
<223> Synthetic Construct

<400> 10
   Met Thr Glu Tyr Lys Pro Thr Val Arg Leu Ala Thr Arg Asp Asp Val
   1               5                   10                  15
   Pro Arg Ala Val Arg Thr Leu Ala Ala Ala Phe Ala Asp Tyr Pro Ala
               20                  25                  30
   Thr Arg His Thr Val Asp Pro Asp Arg His Ile Glu Arg Val Thr Glu
           35                  40                  45
   Leu Gln Glu Leu Phe Leu Thr Arg Val Gly Leu Asp Ile Gly Lys Val
       50                  55                  60
   Trp Val Ala Asp Asp Gly Ala Ala Val Ala Val Trp Thr Thr Pro Glu
   65                  70                  75                  80
   Ser Val Glu Ala Gly Ala Val Phe Ala Glu Ile Gly Pro Arg Met Ala
                   85                  90                  95
   Glu Leu Ser Gly Ser Arg Leu Ala Ala Gln Gln Gln Met Glu Gly Leu
               100                 105                 110
   Leu Ala Pro His Arg Pro Lys Glu Pro Ala Trp Phe Leu Ala Thr Val
           115                 120                 125
   Gly Val Ser Pro Asp His Gln Gly Lys Gly Leu Gly Ser Ala Val Val
       130                 135                 140
   Leu Pro Gly Val Glu Ala Ala Glu Arg Ala Gly Val Pro Ala Phe Leu
   145 150 155 160
   Glu Thr Ser Ala Pro Arg Asn Leu Pro Phe Tyr Glu Arg Leu Gly Phe
   165 170 175
   Thr Val Thr Ala Asp Val Glu Cys Pro Lys Asp Arg Ala Thr Trp Cys
   180 185 190
   Met Thr Arg Lys Pro Gly Ala
   195

<210> 11
<211> 4371
<212> DNA
<213> Artificial

<220>
<223> CEC sequence

<400> 11
   ataacttcgt ataatgtatg ctatacgaag ttattaggtc cctcgaagag gttcactagc 60
   tagttattaa tagtaatcaa ttacggggtc attagttcat agcccatata tggagttccg 120
   cgttacataa cttacggtaa atggcccgcc tggctgaccg cccaacgacc cccgcccatt 180
   gacgtcaata atgacgtatg ttcccatagt aacgccaata gggactttcc attgacgtca 240
   atgggtggac tatttacggt aaactgccca cttggcagta catcaagtgt atcatatgcc 300
   aagtacgccc cctattgacg tcaatgacgg taaatggccc gcctggcatt atgcccagta 360
   catgacctta tgggactttc ctacttggca gtacatctac gtattagtca tcgctattac 420
   catgggtcga ggtgagcccc acgttctgct tcactctccc catctccccc ccctccccac 480
   ccccaatttt gtatttattt atttttaatt tattttgtgc agcgatgggg gcgggggggg 540
   ggggggcgcg cgccaggcgg ggcggggcgg ggcgaggggc ggggcggggc gaggcggaga 600
   ggtgcggcgg cagccaatca gagcggcgcg ctccgaaagt ttccttttat ggcgaggcgg 660
   cggcggcggc ggccctataa aaagcgaagc gcgcggcggg cgggagtcgc tgcgttgcct 720
   tcgccccgtg ccccgctccg cgccgcctcg cgccgcccgc cccggctctg actgaccgcg 780
   ttactcccac aggtgagcgg gcgggacggc ccttctcctc cgggctgtaa ttagcgcttg 840
   gtttaatgac ggctcgtttc ttttctgtgg ctgcgtgaaa gccttaaagg gctccgggag 900
   ggccctttgt gcggggggga gcggctcggg gggtgcgtgc gtgtgtgtgt gcgtggggag 960
   cgccgcgtgc ggcccgcgct gcccggcggc tgtgagcgct gcgggcgcgg cgcggggctt 1020
   tgtgcgctcc gcgtgtgcgc gaggggagcg cggccggggg cggtgccccg cggtgcgggg 1080
   gggctgcgag gggaacaaag gctgcgtgcg gggtgtgtgc gtgggggggt gagcaggggg 1140
   tgtgggcgcg gcggtcgggc tgtaaccccc ccctgcaccc ccctccccga gttgctgagc 1200
   acggcccggc ttcgggtgcg gggctccgtg cggggcgtgg cgcggggctc gccgtgccgg 1260
   gcggggggtg gcggcaggtg ggggtgccgg gcggggcggg gccgcctcgg gccggggagg 1320
   gctcggggga ggggcgcggc ggccccggag cgccggcggc tgtcgaggcg cggcgagccg 1380
   cagccattgc cttttatggt aatcgtgcga gagggcgcag ggacttcctt tgtcccaaat 1440
   ctggcggagc cgaaatctgg gaggcgccgc cgcaccccct ctagcgggcg cgggcgaagc 1500
   ggtgcggcgc cggcaggaag gaaatgggcg gggagggcct tcgtgcgtcg ccgcgccgcc 1560
   gtccccttct ccatctccag cctcggggct gccgcagggg gacggctgcc ttcggggggg 1620
   acggggcagg gcggggttcg gcttctggcg tgtgaccggc ggctctagag cctctgctaa 1680
   ccatgttcat gccttcttct ttttcctaca gctcctgggc aacgtgctgg ttgttgtgct 1740
   gtctcatcat tttggcaaag aattcgccac catggtgagc aagggcgagg agctgttcac 1800
   cggggtggtg cccatcctgg tcgagctgga cggcgacgta aacggccaca agttcagcgt 1860
   gtccggcgag ggcgagggcg atgccaccta cggcaagctg accctgaagt tcatctgcac 1920
   caccggcaag ctgcccgtgc cctggcccac cctcgtgacc accctgacct acggcgtgca 1980
   gtgcttcagc cgctaccccg accacatgaa gcagcacgac ttcttcaagt ccgccatgcc 2040
   cgaaggctac gtccaggagc gcaccatctt cttcaaggac gacggcaact acaagacccg 2100
   cgccgaggtg aagttcgagg gcgacaccct ggtgaaccgc atcgagctga agggcatcga 2160
   cttcaaggag gacggcaaca tcctggggca caagctggag tacaactaca acagccacaa 2220
   cgtctatatc atggccgaca agcagaagaa cggcatcaag gtgaacttca agatccgcca 2280
   caacatcgag gacggcagcg tgcagctcgc cgaccactac cagcagaaca cccccatcgg 2340
   cgacggcccc gtgctgctgc ccgacaacca ctacctgagc acccagtccg ccctgagcaa 2400
   agaccccaac gagaagcgcg atcacatggt cctgctggag ttcgtgaccg ccgccgggat 2460
   cactctcggc atggacgagc tgtacaagta agaattcact cctcaggtgc aggctgccta 2520
   tcagaaggtg gtggctggtg tggccaatgc cctggctcac aaataccact gagatctggc 2580
   catacacttg agtgacaatg acatccactt tgcctttctc tccacaggtg tccactccca 2640
   ggtccaactg caggtcgagc atgcatctag ggcggccaat tccgcccctc tccctccccc 2700
   ccccctaacg ttactggccg aagccgcttg gaataaggcc ggtgtgcgtt tgtctatatg 2760
   tgattttcca ccatattgcc gtcttttggc aatgtgaggg cccggaaacc tggccctgtc 2820
   ttcttgacga gcattcctag gggtctttcc cctctcgcca aaggaatgca aggtctgttg 2880
   aatgtcgtga aggaagcagt tcctctggaa gcttcttgaa gacaaacaac gtctgtagcg 2940
   accctttgca ggcagcggaa ccccccacct ggcgacaggt gcctctgcgg ccaaaagcca 3000
   cgtgtataag atacacctgc aaaggcggca caaccccagt gccacgttgt gagttggata 3060
   gttgtggaaa gagtcaaatg gctctcctca agcgtattca acaaggggct gaaggatgcc 3120
   cagaaggtac cccattgtat gggatctgat ctggggcctc ggtgcacatg ctttacatgt 3180
   gtttagtcga ggttaaaaaa acgtctaggc cccccgaacc acggggacgt ggttttcctt 3240
   tgaaaaacac gatgataagc ttgccacaac ccacaaggag acgaccttcc atgaccgagt 3300
   acaagcccac ggtgcgcctc gccacccgcg acgacgtccc ccgggccgta cgcaccctcg 3360
   ccgccgcgtt cgccgactac cccgccacgc gccacaccgt cgacccggac cgccacatcg 3420
   agcgggtcac cgagctgcaa gaactcttcc tcacgcgcgt cgggctcgac atcggcaagg 3480
   tgtgggtcgc ggacgacggc gccgcggtgg cggtctggac cacgccggag agcgtcgaag 3540
   cgggggcggt gttcgccgag atcggcccgc gcatggccga gttgagcggt tcccggctgg 3600
   ccgcgcagca acagatggaa ggcctcctgg cgccgcaccg gcccaaggag cccgcgtggt 3660
   tcctggccac cgtcggcgtc tcgcccgacc accagggcaa gggtctgggc agcgccgtcg 3720
   tgctccccgg agtggaggcg gccgagcgcg ccggggtgcc cgccttcctg gagacctccg 3780
   cgccccgcaa cctccccttc tacgagcggc tcggcttcac cgtcaccgcc gacgtcgagt 3840
   gcccgaagga ccgcgcgacc tggtgcatga cccgcaagcc cggtgcctga cgcccgcccc 3900
   acgacccgca gcgcccgacc gaaaggagcg cacgacccca tggctccgac cgaagccgac 3960
   ccgggcggcc ccgccgaccc cgcacccgcc cccgaggccc accgactcta gagctcgctg 4020
   atcagcctcg actgtgcctt ctagttgcca gccatctgtt gtttgcccct cccccgtgcc 4080
   ttccttgacc ctggaaggtg ccactcccac tgtcctttcc taataaaatg aggaaattgc 4140
   atcgcattgt ctgagtaggt gtcattctat tctggggggt ggggtggggc aggacagcaa 4200
   gggggaggat tgggaagaca atagcaggca tgctggggat gcggtgggct ctatggcttc 4260
   tgaggcggaa agaaccagct ggggctcgat tggagggatc ctaagcttgg ctggacgtaa 4320
   actcctcttc agacctaata acttcgtata gcatacatta tacgaagtta t 4371

<210> 12
<211> 25
<212> DNA
<213> Artificial

<220>
<223> D6Mit183 primer 1

<400> 12
ttctcaatga acactagaac attcg 25

<210> 13
<211> 27

<212> DNA
<213> Artificial

<220>
<223> D6Mit183 primer 2

<400> 13
aaaacacagg tagaaaacat acataca 27

<210> 14
<211> 21
<212> DNA
<213> Artificial

<220>
<223> D6Mit102 primer 1

<400> 14
ccatgtggat atcttccctt g 21

<210> 15
<211> 26
<212> DNA
<213> Artificial

<220>
<223> D6Mit102 primer 2

<400> 15
gtatacccag ttgtaaatct tgtgtg 26

<210> 16
<211> 20
<212> DNA
<213> Artificial

<220>
<223> D6Mit14 primer 1

<400> 16
atgcagaaac atgagtgggg 20

<210> 17
<211> 20
<212> DNA
<213> Artificial

<220>
<223> D6Mit14 primer 2

<400> 17
cacaaggcct gatgacctct 20

<210> 18
<211> 20
<212> DNA
<213> Artificial

<220>
<223> GFP F primer

<400> 18
cgtaaacggc cacaagttca 20

<210> 19
<211> 21
<212> DNA
<213> Artificial

<220>
<223> GFP R primer

<400> 19
cgctttactt gtacagctcg t 21

<210> 20
<211> 20
<212> DNA
<213> Artificial

<220>
<223> Nanog F1 primer

<400> 20
gcgcatttta gcaccccaca 20

<210> 21
<211> 20
<212> DNA
<213> Artificial

<220>
<223> Nanog R1 primer

<400> 21
gttctaagtc ctaggtttgc 20

<210> 22
<211> 20
<212> DNA
<213> Artificial

<220>
<223> Stella/PGC7 F primer

<400> 22
acagactgac tgctaattgg 20

<210> 23
<211> 23
<212> DNA
<213> Artificial

<220>
<223> Stella/PGC7 R primer

<400> 23
ggaaattaga acgtacatac tcc 23

<210> 24
<211> 26
<212> DNA
<213> Artificial

<220>
<223> G3pdh F primer

<400> 24
tgaaggtcgg tgtgaacgga tttggc 26

<210> 25
<211> 21
<212> DNA
<213> Artificial

<220>
<223> G3pdh R primer

<400> 25
catgtaggcc atgaggtcca c 21